# EUROPEAN PATENT APPLICATION

(11) **EP 2 418 283 A1**
(43) Date of publication of application: **15.02.2012**
(21) Application number: 10008393.0
(22) Date of filing: 11.08.2010
(51) Int. Cl.: C12N 15/82

(54) **Process of transfecting plants**

(30) Priority: 07.08.2010 EP 10008267
(71) Applicant: Nomad Bioscience GmbH, 80333 München (DE)
(72) Inventor: Giritch, Anatoli, 06108 Halle (DE); Symonenko, Yuri, 06110 Halle (DE); Hahn, Simone, 06114 Halle (DE); Tiede, Doreen, 06366 Köthen (DE); Shvarts, Anton, 06110 Halle (DE); Roemer, Patrick, 06780 Zoerbig (DE); Gleba, Yuri, 10117 Berlin (DE)
(74) Representative: Blodig, Wolfgang

(57) **Abstract**

A process of transfecting a plant, comprising spraying parts of said plant with an aqueous suspension containing cells of an *Agrobacterium* strain and at least one abrasive suspended in said suspension, said *Agrobacterium* strain comprising a DNA molecule comprising a nucleic acid construct containing a DNA sequence of interest to be transfected into the plant.

SEQUENCE LISTING

<110> Nomad Bioscience GmbH

<120> Process of Transfecting Plants

<130> EAD-15402

<160> 6

<170> PatentIn version 3.3

<210> 1
<211> 3047
<212> DNA
<213> Artificial

<220>
<223> T-DNA region of pNMD280

<400> 1
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag gcatggaatt 180

cccagggggg tgggcatgcc aattccaatc ccacaaaaat ctgagcttaa cagcacagtt 240

gctcctctca gagcagaatc gggtattcaa caccctcata tcaactacta cgttgtgtat 300

aacggtccac atgccggtat atacgatgac tggggttgta caaaggcggc aacaaacggc 360

gttcccggag ttgcacacaa gaaatttgcc actattacag aggcaagagc agcagctgac 420

gcgtacacaa caagtcagca aacagacagg ttgaacttca tccccaaagg agaagctcaa 480

ctcaagccca agagctttgc taaggcccta acaagcccac caaagcaaaa agcccactgg 540

ctcacgctag gaaccaaaag gcccagcagt gatccagccc caaaagagat ctcctttgcc 600

ccggagatta caatggacga tttcctctat ctttacgatc taggaaggaa gttcgaaggt 660

gaaggtgacg acactatgtt caccactgat aatgagaagg ttagcctctt caatttcaga 720

aagaatgctg acccacagat ggttagagag gcctacgcag caggtctcat caagacgatc 780

tacccgagta acaatctcca ggagatcaaa taccttccca agaaggttaa agatgcagtc 840

aaaagattca ggactaattg catcaagaac acagagaaag acatatttct caagatcaga 900

agtactattc cagtatggac gattcaaggc ttgcttcata aaccaaggca agtaatagag 960

attggagtct ctaaaaaggt agttcctact gaatctaagg ccatgcatgg agtctaagat 1020

tcaaatcgag gatctaacag aactcgccgt gaagactggc gaacagttca tacagagtct 1080

tttacgactc aatgacaaga agaaaatctt cgtcaacatg gtggagcacg acactctggt 1140

ctactccaaa aatgtcaaag atacagtctc agaagaccaa agggctattg agacttttca 1200

acaaaggata atttcgggaa acctcctcgg attccattgc ccagctatct gtcacttcat 1260

cgaaaggaca gtagaaaagg aaggtggctc ctacaaatgc catcattgcg ataaaggaaa 1320

ggctatcatt caagatctct ctgccgacag tggtcccaaa gatggacccc cacccacgag 1380

gagcatcgtg gaaaaagaag acgttccaac cacgtcttca aagcaagtgg attgatgtga 1440

catctccact gacgtaaggg atgacgcaca atcccactat ccttcgcaag acccttcctc 1500

tatataagga agttcatttc atttggagag gacacgctcg agtataagag ctctattttt 1560

acaacaatta ccaacaacaa caaacaacaa acaacattac aattacattt acaattacca 1620

tggaacgagc tatacaagga aacgatgcta gggaacaagc ttatggtgaa cgttggaatg 1680

gaggatcagg aagttccact tctcccttca aacttcctga cgaaagtccg agttggactg 1740

agtggcggct acataacgat gagacgattt cgaatcaaga taatcccctt ggtttcaagg 1800

aaagctgggg tttcgggaaa gttgtattta agagatatct cagatacgac gggacggaaa 1860

cttcactgca cagagtcctt ggatcttgga cgggagattc ggttaactat gcagcatctc 1920

gatttctcgg tttcgaccag atcggatgta cctatagtat tcggtttcga ggagttagtg 1980

tcaccatttc tggagggtcg cgaactcttc agcatctcag tgaaatggca attcggtcta 2040

agcaagaact gctacagctt accccagtca aagtggaaag tgatgtatca agaggatgcc 2100

ctgaaggtgt tgaaaccttc gaagaagaaa gcgagtaagg atcctctaga gtcctgcttt 2160

aatgagatat gcgagacgcc tatgatcgca tgatatttgc tttcaattct gttgtgcacg 2220

ttgtaaaaaa cctgagcatg tgtagctcag atccttaccg ccggtttcgg ttcattctaa 2280

tgaatatatc acccgttact atcgtatttt tatgaataat attctccgtt caatttactg 2340

attgtaccct actacttata tgtacaatat taaaatgaaa acaatatatt gtgctgaata 2400

ggtttatagc gacatctatg atagagcgcc acaataacaa acaattgcgt tttattatta 2460

caaatccaat tttaaaaaaa gcggcagaac cggtcaaacc taaaagactg attacataaa 2520

tcttattcaa atttcaaaag tgccccaggg gctagtatct acgacacacc gagcggcgaa 2580

ctaataacgc tcactgaagg gaactccggt tccccgccgg cgcgcatggg tgagattcct 2640

tgaagttgag tattggccgt ccgctctacc gaaagttacg ggcaccattc aacccggtcc 2700

agcacggcgg ccgggtaacc gacttgctgc cccgagaatt atgcagcatt tttttggtgt 2760

atgtgggccc caaatgaagt gcaggtcaaa ccttgacagt gacgacaaat cgttgggcgg 2820

gtccagggcg aattttgcga caacatgtcg aggctcagca ggacctgcat aagctcttct 2880

gtcagcgggc ccactgcatc caccccagta cattaaaaac gtccgcaatg tgttattaag 2940

ttgtctaagc gtcaatttgt ttacaccaca atatatcctg ccaccagcca gccaacagct 3000

ccccgaccgg cagctcggca caaaatcacc actcgataca ggcagcc 3047

<210> 2
<211> 4424
<212> DNA
<213> Artificial

<220>
<223> T-DNA region of pNMD033

<220>
<221>  misc_feature
<222>  (160)..(165)
<223>  n is a, c, g, or t

<400>  2
tgatgggctg cctgtatcga gtggtgattt tgtgccgagc tgccggtcgg ggagctgttg      60

gctggctggt ggcaggatat attgtggtgt aaacaaattg acgcttagac aacttaataa     120

cacattgcgg acgtttttaa tgtactgggg tggatgcagn nnnnnctgct gagcctcgac     180

atgttgtcgc aaaattcgcc ctggacccgc ccaacgattt gtcgtcactg tcaaggtttg     240

acctgcactt catttggggc ccacatacac caaaaaaatg ctgcataatt ctcggggcag     300

caagtcggtt acccggccgc cgtgctggac cgggttgaat ggtgcccgta actttcggta     360

gagcggacgg ccaatactca acttcaagga atctcaccca tgcgcgccgg cggggaaccg     420

gagttccctt cagtgaacgt tattagttcg ccgctcggtg tgtcgtagat actagcccct     480

ggggcctttt gaaatttgaa taagatttat gtaatcagtc ttttaggttt gaccggttct     540

gccgcttttt ttaaaattgg atttgtaata ataaaacgca attgtttgtt attgtggcgc     600

tctatcatag atgtcgctat aaacctattc agcacaatat attgttttca ttttaatatt     660

gtacatataa gtagtagggt acaatcagta aattgaacgg agaatattat tcataaaaat     720

acgatagtaa cgggtgatat attcattaga atgaaccgaa accggcggta aggatctgag     780

ctacacatgc tcaggttttt tacaacgtgc acaacagaat tgaaagcaaa tatcatgcga     840

tcataggcgt ctcgcatatc tcattaaagc aggactctag gatcgatccc ccgggtcatc     900

aaatctcggt gacgggcagg accggacggg gcggtaccgg caggctgaag tccagctgcc     960

agaaacccac gtcatgccag ttcccgtgct tgaagccggc cgcccgcagc atgccgcggg    1020

gggcatatcc gagcgcctcg tgcatgcgca cgctcgggtc gttgggcagc ccgatgacag    1080

cgaccacgct cttgaagccc tgtgcctcca gggacttcag caggtgggtg tagagcgtgg    1140

agcccagtcc cgtccgctgg tggcgggggg agacgtacac ggttgactcg gccgtccagt    1200

cgtaggcgtt gcgtgccttc caggggcccg cgtaggcgat gccggcgacc tcgccgtcca    1260

cctcggcgac gagccaggga tagcgctccc gcagacggac gaggtcgtcc gtccactcct    1320

gcggttcctg cggctcggta cggaagttga ccgtgcttgt ctcgatgtag tggttgacga    1380

tggtgcagac cgccggcatg tccgcctcgg tggcacggcg gatgtcggcc gggcgtcgtt    1440

ctgggctcat ggtaattgta aatagtaatt gtaatgttgt ttgttgtttg ttgttgttgg    1500

taattgttgt aaaaatagtc gagttgagag tgaatatgag actctaattg gataccgagg    1560

ggaatttatg gaacgtcagt ggagcatttt tgacaagaaa tatttgctag ctgatagtga    1620

ccttaggcga cttttgaacg cgcaataatg gtttctgacg tatgtgctta gctcattaaa    1680

ctccagaaac ccgcggctca gtggctcctt caacgttgcg gttctgtcag ttccaaacgt    1740

aaaacggctt gtcccgcgtc atcggcgggg gtcataacgt gactccctta attctccgct    1800

catgatccga attccaatcc cacaaaaatc tgagcttaac agcacagttg ctcctctcag 1860
agcagaatcg ggtattcaac accctcatat caactactac gttgtgtata acggtccaca 1920
tgccggtata tacgatgact ggggttgtac aaaggcggca acaaacggcg ttcccggagt 1980
tgcacacaag aaatttgcca ctattacaga ggcaagagca gcagctgacg cgtacacaac 2040
aagtcagcaa acagacaggt tgaacttcat ccccaaagga gaagctcaac tcaagcccaa 2100
gagctttgct aaggccctaa caagcccacc aaagcaaaaa gcccactggc tcacgctagg 2160
aaccaaaagg cccagcagtg atccagcccc aaaagagatc tcctttgccc cggagattac 2220
aatggacgat ttcctctatc tttacgatct aggaaggaag ttcgaaggtg aaggtgacga 2280
cactatgttc accactgata atgagaaggt tagcctcttc aatttcagaa agaatgctga 2340
cccacagatg gttagagagg cctacgcagc aggtctcatc aagacgatct acccgagtaa 2400
caatctccag gagatcaaat accttcccaa gaaggttaaa gatgcagtca aaagattcag 2460
gactaattgc atcaagaaca cagagaaaga catatttctc aagatcagaa gtactattcc 2520
agtatggacg attcaaggct tgcttcataa accaaggcaa gtaatagaga ttggagtctc 2580
taaaaaggta gttcctactg aatctaaggc catgcatgga gtctaagatt caaatcgagg 2640
atctaacaga actcgccgtg aagactggcg aacagttcat acagagtctt ttacgactca 2700
atgacaagaa gaaaatcttc gtcaacatgg tggagcacga cactctggtc tactccaaaa 2760
atgtcaaaga tacagtctca gaagaccaaa gggctattga gacttttcaa caaaggataa 2820
tttcgggaaa cctcctcgga ttccattgcc cagctatctg tcacttcatc gaaaggacag 2880
tagaaaagga aggtggctcc tacaaatgcc atcattgcga taaaggaaag gctatcattc 2940
aagatctctc tgccgacagt ggtcccaaag atggaccccc acccacgagg agcatcgtgg 3000
aaaaagaaga cgttccaacc acgtcttcaa agcaagtgga ttgatgtgac atctccactg 3060
acgtaaggga tgacgcacaa tcccactatc cttcgcaaga cccttcctct atataaggaa 3120
gttcatttca tttggagagg acacgctcga gtataagagc tcatttttac aacaattacc 3180
aacaacaaca aacaacaaac aacattacaa ttacatttac aattatcgat accatggtga 3240
gcaagggcga ggagctgttc accggggtgg tgcccatcct ggtcgagctg gacggcgacg 3300
tgaacggcca caagttcagc gtgtccggcg agggcgaggg cgatgccacc tacggcaagc 3360
tgaccctgaa gttcatctgc accaccggca agctgcccgt gccctggccc accctcgtga 3420
ccaccttcag ctacggcgtg cagtgcttca gccgctaccc cgaccacatg aagcagcacg 3480
acttcttcaa gtccgccatg cccgaaggct acgtccagga gcgcaccatc ttcttcaagg 3540
acgacggcaa ctacaagacc cgcgccgagg tgaagttcga gggcgacacc ctggtgaacc 3600
gcatcgagct gaagggcatc gacttcaagg aggacggcaa catcctgggg cacaagctgg 3660
agtacaacta caacagccac aacgtctata tcatggccga caagcagaag aacggcatca 3720
aggtgaactt caagatccgc cacaacatcg aggacggcag cgtgcagctc gccgaccact 3780

accagcagaa cacccccatc ggcgacggcc ccgtgctgct gcccgacaac cactacctga 3840

gcacccagtc cgccctgagc aaagacccca acgagaagcg cgatcacatg gtcctgctgg 3900

agttcgtgac cgccgccggg atcactcacg gcatggacga gctgtacaag taaagcggat 3960

cctctagagt caagcagatc gttcaaacat ttggcaataa agtttcttaa gattgaatcc 4020

tgttgccggt cttgcgatga ttatcatata atttctgttg aattacgtta agcatgtaat 4080

aattaacatg taatgcatga cgttatttat gagatgggtt tttatgatta gagtcccgca 4140

attatacatt taatacgcga tagaaaacaa aatatagcgc gcaaactagg ataaattatc 4200

gcgcgcggtg tcatctatgt tactagatcg acctgcaggc atgcaagctt agatcagatt 4260

gtcgtttccc gccttcagtt taaactatca gtgtttgaca ggatatattg gcgggtaaac 4320

ctaagagaaa agagcgttta ttagaataat cggatattta aaagggcgtg aaaaggttta 4380

tccgttcgtc catttgtatg tgcatgccaa ccacagggtt cccc 4424

<210> 3
<211> 9687
<212> DNA
<213> Artificial

<220>
<223> T-DNA region of pNMD035 (Fig. 2)

<400> 3
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag cttggaattg 180

gtaccacgcg tttcgacaaa atttagaacg aacttaatta tgatctcaaa tacattgata 240

catatctcat ctagatctag gttatcatta tgtaagaaag ttttgacgaa tatggcacga 300

caaaatggct agactcgatg taattggtat ctcaactcaa cattatactt ataccaaaca 360

ttagttagac aaaatttaaa caactatttt ttatgtatgc aagagtcagc atatgtataa 420

ttgattcaga atcgttttga cgagttcgga tgtagtagta gccattattt aatgtacata 480

ctaatcgtga atagtgaata tgatgaaaca ttgtatctta ttgtataaat atccataaac 540

acatcatgaa agacactttc tttcacggtc tgaattaatt atgatacaat tctaatagaa 600

aacgaattaa attacgttga attgtatgaa atctaattga acaagccaac cacgacgacg 660

actaacgttg cctggattga ctcggtttaa gttaaccact aaaaaaacgg agctgtcatg 720

taacacgcgg atcgagcagg tcacagtcat gaagccatca aagcaaaaga actaatccaa 780

gggctgagat gattaattag tttaaaaatt agttaacacg agggaaaagg ctgtctgaca 840

gccaggtcac gttatcttta cctgtggtcg aaatgattcg tgtctgtcga ttttaattat 900

ttttttgaaa ggccgaaaat aaagttgtaa gagataaacc cgcctatata aattcatata 960

ttttcctctc cgctttgaag ttttagtttt attgcaacaa caacaacaaa ttacaataac 1020

aacaaacaaa atacaaacaa caacaacatg gcacaatttc aacaaacaat tgacatgcaa 1080
actctccaag ccgctgcggg acgcaacagc ttggtgaatg atttggcatc tcgtcgcgtt 1140
tacgataatg cagtcgagga gctgaatgct cgttccagac gtcccaaggt aataggaact 1200
ttctggatct actttatttg ctggatctcg atcttgtttt ctcaatttcc ttgagatctg 1260
gaattcgttt aatttggatc tgtgaacctc cactaaatct tttggtttta ctagaatcga 1320
tctaagttga ccgatcagtt agctcgatta tagctaccag aatttggctt gaccttgatg 1380
gagagatcca tgttcatgtt acctgggaaa tgatttgtat atgtgaattg aaatctgaac 1440
tgttgaagtt agattgaatc tgaacactgt caatgttaga ttgaatctga acactgttta 1500
aggttagatg aagtttgtgt atagattctt cgaaacttta ggatttgtag tgtcgtacgt 1560
tgaacagaaa gctatttctg attcaatcag ggtttatttg actgtattga actctttttg 1620
tgtgtttgca ggtccacttc tccaaggcag tgtctacgga acagaccctg attgcaacaa 1680
acgcatatcc ggagttcgag atttccttta ctcatacgca atccgctgtg cactccttgg 1740
ccggaggcct tcggtcactt gagttggagt atctcatgat gcaagttccg ttcggttctc 1800
tgacgtacga catcggcggt aacttttccg cgcacctttt caaagggcgc gattacgttc 1860
actgctgcat gcctaatctg gatgtacgtg acattgctcg ccatgaagga cacaaggaag 1920
ctatttacag ttatgtgaat cgtttgaaaa ggcagcagcg tcctgtgcct gaataccaga 1980
gggcagcttt caacaactac gctgagaacc cgcacttcgt ccattgcgac aaacctttcc 2040
aacagtgtga attgacgaca gcgtatggca ctgacaccta cgctgtagct ctccatagca 2100
tttatgatat ccctgttgag gagttcggtt ctgcgctact caggaagaat gtgaaaactt 2160
gtttcgcggc ctttcatttc catgagaata tgcttctaga ttgtgataca gtcacactcg 2220
atgagattgg agctacgttc cagaaatcag gtaacattcc ttagttacct ttcttttctt 2280
tttccatcat aagtttatag attgtacatg ctttgagatt tttctttgca aacaatctca 2340
ggtgataacc tgagcttctt cttccataat gagagcactc tcaattacac ccacagcttc 2400
agcaacatca tcaagtacgt gtgcaagacg ttcttccctg ctagtcaacg cttcgtgtac 2460
cacaaggagt tcctggtcac tagagtcaac acttggtact gcaagttcac gagagtggat 2520
acgttcactc tgttccgtgg tgtgtaccac aacaatgtgg attgcgaaga gttttacaag 2580
gctatggacg atgcgtggca ctacaaaaag acgttagcaa tgcttaatgc cgagaggacc 2640
atcttcaagg ataacgctgc gttaaacttc tggttcccga aggtgctctt gaaattggaa 2700
gtcttctttt gttgtctaaa cctatcaatt tctttgcgga aatttatttg aagctgtaga 2760
gttaaaattg agtcttttaa acttttgtag gtgagagaca tggttatcgt ccctctcttt 2820
gacgcttcta tcacaactgg taggatgtct aggagagagg ttatggtgaa caaggacttc 2880
gtctacacgg tcctaaatca catcaagacc tatcaagcta aggcactgac gtacgcaaac 2940
gtgctgagct tcgtggagtc tattaggtct agagtgataa ttaacggtgt cactgccagg 3000

taagttgtta cttatgattg ttttcctctc tgctacatgt attttgttgt tcatttctgt 3060
aagatataag aattgagttt tcctctgatg atattattag gtctgaatgg gacacagaca 3120
aggcaattct aggtccatta gcaatgacat tcttcctgat cacgaagctg ggtcatgtgc 3180
aagatgaaat aatcctgaaa aagttccaga agttcgacag aaccaccaat gagctgattt 3240
ggacaagtct ctgcgatgcc ctgatggggg ttattccctc ggtcaaggag acgcttgtgc 3300
gcggtggttt tgtgaaagta gcagaacaag ccttagagat caaggttagt atcatatgaa 3360
gaaataccta gtttcagttg atgaatgcta ttttctgacc tcagttgttc tcttttgaga 3420
attatttctt ttctaatttg cctgattttt ctattaattc attaggttcc cgagctatac 3480
tgtaccttcg ccgaccgatt ggtactacag tacaagaagg cggaggagtt ccaatcgtgt 3540
gatctttcca aacctctaga agagtcagag aagtactaca acgcattatc cgagctatca 3600
gtgcttgaga atctcgactc ttttgactta gaggcgttta agactttatg tcagcagaag 3660
aatgtggacc cggatatggc agcaaaggta aatcctggtc cacactttta cgataaaaac 3720
acaagatttt aaactatgaa ctgatcaata atcattccta aaagaccaca cttttgtttt 3780
gtttctaaag taatttttac tgttataaca ggtggtcgta gcaatcatga agtcagaatt 3840
gacgttgcct ttcaagaaac ctacagaaga ggaaatctcg gagtcgctaa aaccaggaga 3900
ggggtcgtgt gcagagcata aggaagtgtt gagcttacaa aatgatgctc cgttcccgtg 3960
tgtgaaaaat ctagttgaag gttccgtgcc ggcgtatgga atgtgtccta agggtggtgg 4020
tttcgacaaa ttggatgtgg acattgctga tttccatctc aagagtgtag atgcagttaa 4080
aaagggaact atgatgtctg cggtgtacac agggtctatc aaagttcaac aaatgaagaa 4140
ctacatagat tacttaagtg cgtcgctggc agctacagtc tcaaacctct gcaaggtaag 4200
aggtcaaaag gtttccgcaa tgatccctct tttttgtttt ctctagtttc aagaatttgg 4260
gtatatgact aacttctgag tgttccttga tgcatatttg tgatgagaca aatgtttgtt 4320
ctatgtttta ggtgcttaga gatgttcacg gcgttgaccc agagtcacag gagaaatctg 4380
gagtgtggga tgttaggaga ggacgttggt tacttaaacc taatgcgaaa agtcacgcgt 4440
ggggtgtggc agaagacgcc aaccacaagt tggttattgt gttactcaac tgggatgacg 4500
gaaagccggt ttgtgatgag acatggttca gggtggcggt gtcaagcgat tccttgatat 4560
attcggatat gggaaaactt aagacgctca cgtcttgcag tccaaatggt gagccaccgg 4620
agcctaacgc caaagtaatt ttggtcgatg gtgttcccgg ttgtggaaaa acgaaggaga 4680
ttatcgaaaa ggtaagttct gcatttggtt atgctccttg cattttaggt gttcgtcgct 4740
cttccatttc catgaatagc taagattttt tttctctgca ttcattcttc ttgcctcagt 4800
tctaactgtt tgtggtattt ttgttttaat tattgctaca ggtaaacttc tctgaagact 4860
tgattttagt ccctgggaag gaagcttcta agatgatcat ccggagggcc aaccaagctg 4920
gtgtgataag agcggataag gacaatgtta gaacggtgga ttccttcttg atgcatcctt 4980

ctagaagggt gtttaagagg ttgtttatcg atgaaggact aatgctgcat acaggttgtg 5040
taaatttcct actgctgcta tctcaatgtg acgtcgcata tgtgtatggg gacacaaagc 5100
aaattccgtt catttgcaga gtcgcgaact ttccgtatcc agcgcatttt gcaaaactcg 5160
tcgctgatga gaaggaagtc agaagagtta cgctcaggta aagcaactgt gttttaatca 5220
atttcttgtc aggatatatg gattataact taatttttga gaaatctgta gtatttggcg 5280
tgaaatgagt ttgctttttg gtttctcccg tgttataggt gcccggctga tgttacgtat 5340
ttccttaaca agaagtatga cggggcggtg atgtgtacca gcgcggtaga gagatccgtg 5400
aaggcagaag tggtgagagg aaagggtgca ttgaacccaa taaccttacc gttggagggt 5460
aaaattttga ccttcacaca agctgacaag ttcgagttac tggagaaggg ttacaaggta 5520
aagtttccaa ctttccttta ccatatcaaa ctaaagttcg aaacttttta tttgatcaac 5580
ttcaaggcca cccgatcttt ctattcctga ttaatttgtg atgaatccat attgactttt 5640
gatggttacg caggatgtga acactgtgca cgaggtgcaa ggggagacgt acgagaagac 5700
tgctattgtg cgcttgacat caactccgtt agagatcata tcgagtgcgt cacctcatgt 5760
tttggtggcg ctgacaagac acacaacgtg ttgtaaatat tacaccgttg tgttggaccc 5820
gatggtgaat gtgatttcag aaatggagaa gttgtccaat ttccttcttg acatgtatag 5880
agttgaagca ggtctgtctt tcctatttca tatgtttaat cctaggaatt tgatcaattg 5940
attgtatgta tgtcgatccc aagactttct tgttcactta tatcttaact ctctctttgc 6000
tgtttcttgc aggtgtccaa tagcaattac aaatcgatgc agtattcagg ggacagaact 6060
tgtttgttca gacgcccaag tcaggagatt ggcgagatat gcaattttac tatgacgctc 6120
ttcttcccgg aaacagtact attctcaatg aatttgatgc tgttacgatg aatttgaggg 6180
atatttcctt aaacgtcaaa gattgcagaa tcgacttctc caaatccgtg caacttccta 6240
aagaacaacc tattttcctc aagcctaaaa taagaactgc ggcagaaatg ccgagaactg 6300
caggtaaaat attggatgcc agacgatatt ctttcttttg atttgtaact ttttcctgtc 6360
aaggtcgata aattttattt tttttggtaa aaggtcgata attttttttt ggagccatta 6420
tgtaattttc ctaattaact gaaccaaaat tatacaaacc aggtttgctg gaaaatttgg 6480
ttgcaatgat caaaagaaac atgaatgcgc cggatttgac agggacaatt gacattgagg 6540
atactgcatc tctggtggtt gaaaagtttt gggattcgta tgttgacaag gaatttagtg 6600
gaacgaacga aatgaccatg acaagggaga gcttctccag gtaaggactt ctcatgaata 6660
ttagtggcag attagtgttg ttaaagtctt tggttagata atcgatgcct cctaattgtc 6720
catgttttac tggttttcta caattaaagg tggctttcga aacaagagtc atctacagtt 6780
ggtcagttag cggactttaa ctttgtggat ttgccggcag tagatgagta caagcatatg 6840
atcaagagtc aaccaaagca aaagttagac ttgagtattc aagacgaata tcctgcattg 6900
cagacgatag tctaccattc gaaaaagatc aatgcgattt tcggtccaat gttttcagaa 6960

cttacgagga tgttactcga aaggattgac tcttcgaagt ttctgttcta caccagaaag 7020
acacctgcac aaatagagga cttcttttct gacctagact caacccaggc gatggaaatt 7080
ctggaactcg acatttcgaa gtacgataag tcacaaaacg agttccattg tgctgtagag 7140
tacaagatct gggaaaagtt aggaattgat gagtggctag ctgaggtctg gaaacaaggt 7200
gagttcctaa gttccatttt tttgtaatcc ttcaatgtta ttttaacttt tcagatcaac 7260
atcaaaatta ggttcaattt tcatcaacca aataatattt ttcatgtata tataggtcac 7320
agaaaaacga ccttgaaaga ttatacggcc ggaatcaaaa catgtctttg gtatcaaagg 7380
aaaagtggtg atgtgacaac ctttattggt aataccatca tcattgccgc atgtttgagc 7440
tcaatgatcc ccatggacaa agtgataaag gcagcttttt gtggagacga tagcctgatt 7500
tacattccta aaggtttaga cttgcctgat attcaggcgg gcgcgaacct catgtggaac 7560
ttcgaggcca aactcttcag gaagaagtat ggttacttct gtggtcgtta tgttattcac 7620
catgatagag gagccattgt gtattacgat ccgcttaaac taatatctaa gttaggttgt 7680
aaacatatta gagatgttgt tcacttagaa gagttacgcg agtctttgtg tgatgtagct 7740
agtaacttaa ataattgtgc gtatttttca cagttagatg aggccgttgc cgaggttcat 7800
aagaccgcgg taggcggttc gtttgctttt tgtagtataa ttaagtattt gtcagataag 7860
agattgttta gagatttgtt ctttgtttga taatgtcgat agtctcgtac gaacctaagg 7920
tgagtgattt cctcaatctt tcgaagaagg aagagatctt gccgaaggct ctaacgaggt 7980
taaaaaccgt gtctattagt actaaagata ttatatctgt caaggagtcg gagactttgt 8040
gtgatataga tttgttaatc aatgtgccat tagataagta tagatatgtg ggtatcctag 8100
gagccgtttt taccggagag tggctagtgc cagacttcgt taaaggtgga gtgacgataa 8160
gtgtgataga taagcgtctg gtgaactcaa aggagtgcgt gattggtacg tacagagccg 8220
cagccaagag taagaggttc cagttcaaat tggttccaaa ttactttgtg tccaccgtgg 8280
acgcaaagag gaagccgtgg caggtaagga tttttatgat atagtatgct tatgtatttt 8340
gtactgaaag catatcctgc ttcattggga tattactgaa agcatttaac tacatgtaaa 8400
ctcacttgat gatcaataaa cttgattttg caggttcatg ttcgtataca agacttgaag 8460
attgaggcgg gttggcagcc gttagctctg gaagtagttt cagttgctat ggtcaccaat 8520
aacgttgtca tgaagggttt gagggaaaag gtcgtcgcaa taaatgatcc ggacgtcgaa 8580
ggtttcgaag gtaagccatc ttcctgctta tttttataat gaacatagaa ataggaagtt 8640
gtgcagagaa actaattaac ctgactcaaa atctaccctc ataattgttg tttgatattg 8700
gtcttgtatt ttgcaggtgt ggttgacgaa ttcgtcgatt cggttgcagc atttaaagcg 8760
gttgacaact ttaaaagaag gaaaaagaag gttgaagaaa agggtgtagt aagtaagtat 8820
aagtacagac cggagaagta cgccggtcct gattcgttta atttgaaaga agaaaacgtc 8880
ttacaacatt acaaacccga atcagtacca gtatttcgat aagaaacaag aaaccatgag 8940

agacctgata tccacaaccg tggtctcgag cttactagag cgtggtgcgc acgatagcgc 9000

atagtgtttt tctctccact tgaatcgaag agatagactt acggtgtaaa tccgtagggg 9060

tggcgtaaac caaattacgc aatgttttgg gttccattta aatcgaaacc ccttatttcc 9120

tggatcacct gttaacgcac gtttgacgtg tattacagtg ggaataagta aaagtgagag 9180

gttcgaatcc tccctaaccc cgggtagggg cccagcggcc gctctagcta gagtcaagca 9240

gatcgttcaa acatttggca ataaagtttc ttaagattga atcctgttgc cggtcttgcg 9300

atgattatca tataatttct gttgaattac gttaagcatg taataattaa catgtaatgc 9360

atgacgttat ttatgagatg ggtttttatg attagagtcc cgcaattata catttaatac 9420

gcgatagaaa acaaaatata gcgcgcaaac taggataaat tatcgcgcgc ggtgtcatct 9480

atgttactag atcgacctgc atccacccca gtacattaaa aacgtccgca atgtgttatt 9540

aagttgtcta agcgtcaatt tgtttacacc acaatatatc ctgccaccag ccagccaaca 9600

gctccccgac cggcagctcg gcacaaaatc accactcgat acaggcagcc catcagtcag 9660

atcaggatct cctttgcgac gctcacc 9687

<210> 4
<211> 9734
<212> DNA
<213> Artificial

<220>
<223> T-DNA region of pNMD661 (Fig. 3)

<400> 4
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag cttggaattg 180

gtaccacgcg tttcgacaaa atttagaacg aacttaatta tgatctcaaa tacattgata 240

catatctcat ctagatctag gttatcatta tgtaagaaag ttttgacgaa tatggcacga 300

caaaatggct agactcgatg taattggtat ctcaactcaa cattatactt ataccaaaca 360

ttagttagac aaaatttaaa caactatttt ttatgtatgc aagagtcagc atatgtataa 420

ttgattcaga atcgttttga cgagttcgga tgtagtagta gccattattt aatgtacata 480

ctaatcgtga atagtgaata tgatgaaaca ttgtatctta ttgtataaat atccataaac 540

acatcatgaa agacactttc tttcacggtc tgaattaatt atgatacaat tctaatagaa 600

aacgaattaa attacgttga attgtatgaa atctaattga acaagccaac cacgacgacg 660

actaacgttg cctggattga ctcggtttaa gttaaccact aaaaaaacgg agctgtcatg 720

taacacgcgg atcgagcagg tcacagtcat gaagccatca aagcaaaaga actaatccaa 780

gggctgagat gattaattag tttaaaaatt agttaacacg agggaaaagg ctgtctgaca 840

gccaggtcac gttatcttta cctgtggtcg aaatgattcg tgtctgtcga ttttaattat 900

ttttttgaaa ggccgaaaat aaagttgtaa gagataaacc cgcctatata aattcatata 960
ttttcctctc cgctttgaag ttttagtttt attgcaacaa caacaacaaa ttacaataac 1020
aacaaacaaa atacaaacaa caacaacatg gcacaatttc aacaaacaat tgacatgcaa 1080
actctccaag ccgctgcggg acgcaacagc ttggtgaatg atttggcatc tcgtcgcgtt 1140
tacgataatg cagtcgagga gctgaatgct cgttccagac gtcccaaggt aataggaact 1200
ttctggatct actttatttg ctggatctcg atcttgtttt ctcaatttcc ttgagatctg 1260
gaattcgttt aatttggatc tgtgaacctc cactaaatct tttggtttta ctagaatcga 1320
tctaagttga ccgatcagtt agctcgatta tagctaccag aatttggctt gaccttgatg 1380
gagagatcca tgttcatgtt acctgggaaa tgatttgtat atgtgaattg aaatctgaac 1440
tgttgaagtt agattgaatc tgaacactgt caatgttaga ttgaatctga acactgttta 1500
aggttagatg aagtttgtgt atagattctt cgaaacttta ggatttgtag tgtcgtacgt 1560
tgaacagaaa gctatttctg attcaatcag ggtttatttg actgtattga actctttttg 1620
tgtgtttgca ggtccacttc tccaaggcag tgtctacgga acagaccctg attgcaacaa 1680
acgcatatcc ggagttcgag atttccttta ctcatacgca atccgctgtg cactccttgg 1740
ccggaggcct tcggtcactt gagttggagt atctcatgat gcaagttccg ttcggttctc 1800
tgacgtacga catcggcggt aacttttccg cgcacctttt caaagggcgc gattacgttc 1860
actgctgcat gcctaatctg gatgtacgtg acattgctcg ccatgaagga cacaaggaag 1920
ctatttacag ttatgtgaat cgtttgaaaa ggcagcagcg tcctgtgcct gaataccaga 1980
gggcagcttt caacaactac gctgagaacc cgcacttcgt ccattgcgac aaacctttcc 2040
aacagtgtga attgacgaca gcgtatggca ctgacaccta cgctgtagct ctccatagca 2100
tttatgatat ccctgttgag gagttcggtt ctgcgctact caggaagaat gtgaaaactt 2160
gtttcgcggc ctttcatttc catgagaata tgcttctaga ttgtgataca gtcacactcg 2220
atgagattgg agctacgttc cagaaatcag gtaacattcc ttagttacct ttcttttctt 2280
tttccatcat aagtttatag attgtacatg ctttgagatt tttctttgca aacaatctca 2340
ggtgataacc tgagcttctt cttccataat gagagcactc tcaattacac ccacagcttc 2400
agcaacatca tcaagtacgt gtgcaagacg ttcttccctg ctagtcaacg cttcgtgtac 2460
cacaaggagt tcctggtcac tagagtcaac acttggtact gcaagttcac gagagtggat 2520
acgttcactc tgttccgtgg tgtgtaccac aacaatgtgg attgcgaaga gttttacaag 2580
gctatggacg atgcgtggca ctacaaaaag acgttagcaa tgcttaatgc cgagaggacc 2640
atcttcaagg ataacgctgc gttaaacttc tggttcccga aggtgctctt gaaattggaa 2700
gtcttctttt gttgtctaaa cctatcaatt tctttgcgga aatttatttg aagctgtaga 2760
gttaaaattg agtcttttaa acttttgtag gtgagagaca tggttatcgt ccctctcttt 2820
gacgcttcta tcacaactgg taggatgtct aggagagagg ttatggtgaa caaggacttc 2880

gtctacacgg tcctaaatca catcaagacc tatcaagcta aggcactgac gtacgcaaac 2940
gtgctgagct tcgtggagtc tattaggtct agagtgataa ttaacggtgt cactgccagg 3000
taagttgtta cttatgattg ttttcctctc tgctacatgt attttgttgt tcatttctgt 3060
aagatataag aattgagttt tcctctgatg atattattag gtctgaatgg gacacagaca 3120
aggcaattct aggtccatta gcaatgacat tcttcctgat cacgaagctg ggtcatgtgc 3180
aagatgaaat aatcctgaaa aagttccaga agttcgacag aaccaccaat gagctgattt 3240
ggacaagtct ctgcgatgcc ctgatggggg ttattccctc ggtcaaggag acgcttgtgc 3300
gcggtggttt tgtgaaagta gcagaacaag ccttagagat caaggttagt atcatatgaa 3360
gaaataccta gtttcagttg atgaatgcta ttttctgacc tcagttgttc tcttttgaga 3420
attatttctt ttctaatttg cctgattttt ctattaattc attaggttcc cgagctatac 3480
tgtaccttcg ccgaccgatt ggtactacag tacaagaagg cggaggagtt ccaatcgtgt 3540
gatctttcca aacctctaga agagtcagag aagtactaca acgcattatc cgagctatca 3600
gtgcttgaga atctcgactc ttttgactta gaggcgttta agactttatg tcagcagaag 3660
aatgtggacc cggatatggc agcaaaggta aatcctggtc cacactttta cgataaaaac 3720
acaagatttt aaactatgaa ctgatcaata atcattccta aaagaccaca cttttgtttt 3780
gtttctaaag taatttttac tgttataaca ggtggtcgta gcaatcatga agtcagaatt 3840
gacgttgcct ttcaagaaac ctacagaaga ggaaatctcg gagtcgctaa aaccaggaga 3900
ggggtcgtgt gcagagcata aggaagtgtt gagcttacaa aatgatgctc cgttcccgtg 3960
tgtgaaaaat ctagttgaag gttccgtgcc ggcgtatgga atgtgtccta agggtggtgg 4020
tttcgacaaa ttggatgtgg acattgctga tttccatctc aagagtgtag atgcagttaa 4080
aaagggaact atgatgtctg cggtgtacac agggtctatc aaagttcaac aaatgaagaa 4140
ctacatagat tacttaagtg cgtcgctggc agctacagtc tcaaacctct gcaaggtaag 4200
aggtcaaaag gtttccgcaa tgatccctct tttttgtttt ctctagtttc aagaatttgg 4260
gtatatgact aacttctgag tgttccttga tgcatatttg tgatgagaca aatgtttgtt 4320
ctatgtttta ggtgcttaga gatgttcacg gcgttgaccc agagtcacag gagaaatctg 4380
gagtgtggga tgttaggaga ggacgttggt tacttaaacc taatgcgaaa agtcacgcgt 4440
ggggtgtggc agaagacgcc aaccacaagt tggttattgt gttactcaac tgggatgacg 4500
gaaagccggt ttgtgatgag acatggttca gggtggcggt gtcaagcgat tccttgatat 4560
attcggatat gggaaaactt aagacgctca cgtcttgcag tccaaatggt gagccaccgg 4620
agcctaacgc caaagtaatt ttggtcgatg gtgttcccgg ttgtggaaaa acgaaggaga 4680
ttatcgaaaa ggtaagttct gcatttggtt atgctccttg cattttaggt gttcgtcgct 4740
cttccatttc catgaatagc taagattttt tttctctgca ttcattcttc ttgcctcagt 4800
tctaactgtt tgtggtattt ttgttttaat tattgctaca ggtaaacttc tctgaagact 4860

tgattttagt ccctgggaag gaagcttcta agatgatcat ccggagggcc aaccaagctg 4920
gtgtgataag agcggataag gacaatgtta gaacggtgga ttccttcttg atgcatcctt 4980
ctagaagggt gtttaagagg ttgtttatcg atgaaggact aatgctgcat acaggttgtg 5040
taaatttcct actgctgcta tctcaatgtg acgtcgcata tgtgtatggg gacacaaagc 5100
aaattccgtt catttgcaga gtcgcgaact ttccgtatcc agcgcatttt gcaaaactcg 5160
tcgctgatga gaaggaagtc agaagagtta cgctcaggta aagcaactgt gttttaatca 5220
atttcttgtc aggatatatg gattataact taatttttga gaaatctgta gtatttggcg 5280
tgaaatgagt ttgctttttg gtttctcccg tgttataggt gcccggctga tgttacgtat 5340
ttccttaaca agaagtatga cggggcggtg atgtgtacca gcgcggtaga gagatccgtg 5400
aaggcagaag tggtgagagg aaagggtgca ttgaacccaa taaccttacc gttggagggt 5460
aaaattttga ccttcacaca agctgacaag ttcgagttac tggagaaggg ttacaaggta 5520
aagtttccaa ctttccttta ccatatcaaa ctaaagttcg aaacttttta tttgatcaac 5580
ttcaaggcca cccgatcttt ctattcctga ttaatttgtg atgaatccat attgactttt 5640
gatggttacg caggatgtga acactgtgca cgaggtgcaa ggggagacgt acgagaagac 5700
tgctattgtg cgcttgacat caactccgtt agagatcata tcgagtgcgt cacctcatgt 5760
tttggtggcg ctgacaagac acacaacgtg ttgtaaatat tacaccgttg tgttggaccc 5820
gatggtgaat gtgatttcag aaatggagaa gttgtccaat ttccttcttg acatgtatag 5880
agttgaagca ggtctgtctt tcctatttca tatgtttaat cctaggaatt tgatcaattg 5940
attgtatgta tgtcgatccc aagactttct tgttcactta tatcttaact ctctctttgc 6000
tgtttcttgc aggtgtccaa tagcaattac aaatcgatgc agtattcagg ggacagaact 6060
tgtttgttca gacgcccaag tcaggagatt ggcgagatat gcaattttac tatgacgctc 6120
ttcttcccgg aaacagtact attctcaatg aatttgatgc tgttacgatg aatttgaggg 6180
atatttcctt aaacgtcaaa gattgcagaa tcgacttctc caaatccgtg caacttccta 6240
aagaacaacc tattttcctc aagcctaaaa taagaactgc ggcagaaatg ccgagaactg 6300
caggtaaaat attggatgcc agacgatatt ctttcttttg atttgtaact ttttcctgtc 6360
aaggtcgata aattttattt tttttggtaa aaggtcgata attttttttt ggagccatta 6420
tgtaattttc ctaattaact gaaccaaaat tatacaaacc aggtttgctg gaaaatttgg 6480
ttgcaatgat caaaagaaac atgaatgcgc cggatttgac agggacaatt gacattgagg 6540
atactgcatc tctggtggtt gaaaagtttt gggattcgta tgttgacaag gaatttagtg 6600
gaacgaacga aatgaccatg acaagggaga gcttctccag gtaaggactt ctcatgaata 6660
ttagtggcag attagtgttg ttaaagtctt tggttagata atcgatgcct cctaattgtc 6720
catgttttac tggttttcta caattaaagg tggctttcga aacaagagtc atctacagtt 6780
ggtcagttag cggactttaa ctttgtggat ttgccggcag tagatgagta caagcatatg 6840

atcaagagtc aaccaaagca aaagttagac ttgagtattc aagacgaata tcctgcattg 6900
cagacgatag tctaccattc gaaaaagatc aatgcgattt tcggtccaat gttttcagaa 6960
cttacgagga tgttactcga aaggattgac tcttcgaagt ttctgttcta caccagaaag 7020
acacctgcac aaatagagga cttcttttct gacctagact caacccaggc gatggaaatt 7080
ctggaactcg acatttcgaa gtacgataag tcacaaaacg agttccattg tgctgtagag 7140
tacaagatct gggaaaagtt aggaattgat gagtggctag ctgaggtctg gaaacaaggt 7200
gagttcctaa gttccatttt tttgtaatcc ttcaatgtta ttttaacttt tcagatcaac 7260
atcaaaatta ggttcaattt tcatcaacca aataatattt ttcatgtata tataggtcac 7320
agaaaaacga ccttgaaaga ttatacggcc ggaatcaaaa catgtctttg gtatcaaagg 7380
aaaagtggtg atgtgacaac ctttattggt aataccatca tcattgccgc atgtttgagc 7440
tcaatgatcc ccatggacaa agtgataaag gcagcttttt gtggagacga tagcctgatt 7500
tacattccta aaggtttaga cttgcctgat attcaggcgg gcgcgaacct catgtggaac 7560
ttcgaggcca aactcttcag gaagaagtat ggttacttct gtggtcgtta tgttattcac 7620
catgatagag gagccattgt gtattacgat ccgcttaaac taatatctaa gttaggttgt 7680
aaacatatta gagatgttgt tcacttagaa gagttacgcg agtctttgtg tgatgtagct 7740
agtaacttaa ataattgtgc gtatttttca cagttagatg aggccgttgc cgaggttcat 7800
aagaccgcgg taggcggttc gtttgctttt tgtagtataa ttaagtattt gtcagataag 7860
agattgttta gagatttgtt ctttgtttga taatgtcgat agtctcgtac gaacctaagg 7920
tgagtgattt cctcaatctt tcgaagaagg aagagatctt gccgaaggct ctaacgaggt 7980
taaaaaccgt gtctattagt actaaagata ttatatctgt caaggagtcg gagactttgt 8040
gtgatataga tttgttaatc aatgtgccat tagataagta tagatatgtg ggtatcctag 8100
ctaggagccg tttttaccgg agagtggcta gtgccagact tcgttaaagg tggagtgacg 8160
ataagtgtga tagataagcg tctggtgaac tcaaaggagt gcgtgattgg tacgtacaga 8220
gccgcagcca agagtaagag gttccagttc aaattggttc caaattactt tgtgtccacc 8280
gtggacgcaa agaggaagcc gtggcaggta aggattttta tgatatagta tgcttatgta 8340
ttttgtactg aaagcatatc ctgcttcatt gggatattac tgaaagcatt taactacatg 8400
taaactcact tgatgatcaa taaacttgat tttgcaggtt catgttcgta tacaagactt 8460
gaagattgag gcgggttggc agccgttagc tctggaagta gtttcagttg ctatggtcac 8520
caataacgtt gtcatgaagg gtttgaggga aaaggtcgtc gcaataaatg atccggacgt 8580
cgaaggtttc gaaggtaagc catcttcctg cttattttta taatgaacat agaaatagga 8640
agttgtgcag agaaactaat taacctgact caaaatctac cctcataatt gttgtttgat 8700
attggtcttg tattttgcag gtgtggttga cgaattcgtc gattcggttg cagcatttaa 8760
agcggttgac aactttaaaa gaaggaaaaa gaaggttgaa gaaaagggtg tagtaagtaa 8820

gtataagtac agaccggaga agtacgccgg tcctgattcg tttaatttga aagaagaaaa 8880

cgtcttacaa cattacaaac ccgaataatc gataactcga gtatttttac aacaattacc 8940

aacaacaaca aacaacaaac aacattacaa ttacatttac aattatccat gtgagacccc 9000

acaaccgtgg ggtctcagat cagcggcccc tagagcgtgg tgcgcacgat agcgcatagt 9060

gtttttctct ccacttgaat cgaagagata gacttacggt gtaaatccgt aggggtggcg 9120

taaaccaaat tacgcaatgt tttgggttcc atttaaatcg aaacccctta tttcctggat 9180

cacctgttaa cgcacgtttg acgtgtatta cagtgggaat aagtaaaagt gagaggttcg 9240

aatcctccct aaccccgggt aggggcccag cggccgctct agctagagtc aagcagatcg 9300

ttcaaacatt tggcaataaa gtttcttaag attgaatcct gttgccggtc ttgcgatgat 9360

tatcatataa tttctgttga attacgttaa gcatgtaata attaacatgt aatgcatgac 9420

gttatttatg agatgggttt ttatgattag agtcccgcaa ttatacattt aatacgcgat 9480

agaaaacaaa atatagcgcg caaactagga taaattatcg cgcgcggtgt catctatgtt 9540

actagatcga cctgcatcca ccccagtaca ttaaaaacgt ccgcaatgtg ttattaagtt 9600

gtctaagcgt caatttgttt acaccacaat atatcctgcc accagccagc caacagctcc 9660

ccgaccggca gctcggcaca aaatcaccac tcgatacagg cagcccatca gtcagatcag 9720

gatctccttt gcga 9734

<210> 5
<211> 7424
<212> DNA
<213> Artificial

<220>
<223> T-DNA region of pNMD670 (Fig. 4)

<400> 5
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag gcatgcctgc 180

aggtcaacat ggtggagcac gacacgcttg tctactccaa aaatatcaaa gatacagtct 240

cagaagacca aagggcaatt gagacttttc aacaaagggt aatatccgga aacctcctcg 300

gattccattg cccagctatc tgtcacttta ttgtgaagat agtggaaaag gaaggtggct 360

cctacaaatg ccatcattgc gataaaggaa aggccatcgt tgaagatgcc tctgccgaca 420

gtggtcccaa agatggaccc ccacccacga ggagcatcgt ggaaaaagaa gacgttccaa 480

ccacgtcttc aaagcaagtg gattgatgtg atatctccac tgacgtaagg gatgacgcac 540

aatcccacta tccttcgcaa gacccttcct ctatataagg aagttcattt catttggaga 600

ggagaaaact aaaccataca ccaccaacac aaccaaaccc accacgccca attgttacac 660

acccgcttga aaaagaaagt ttaacaaatg gccaaggtgc gcgaggttta ccaatctttt 720

acagactcca ccacaaaaac tctcatccaa gatgaggctt atagaaacat tcgccccatc 780
atggaaaaac acaaactagc taacccttac gctcaaacgg ttgaagcggc taatgatcta 840
gaggggttcg gcatagccac caatccctat agcattgaat tgcatacaca tgcagccgct 900
aagaccatag agaataaact tctagaggtg cttggttcca tcctaccaca agaacctgtt 960
acatttatgt ttcttaaacc cagaaagcta aactacatga gaagaaaccc gcggatcaag 1020
gacattttcc aaaatgttgc cattgaacca agagacgtag ccaggtaccc caaggaaaca 1080
ataattgaca aactcacaga gatcacaacg gaaacagcat acattagtga cactctgcac 1140
ttcttggatc cgagctacat agtggagaca ttccaaaact gcccaaaatt gcaaacattg 1200
tatgcgacct tagttctccc cgttgaggca gcctttaaaa tggaaagcac tcacccgaac 1260
atatacagcc tcaaatactt cggagatggt ttccagtata taccaggcaa ccatggtggc 1320
ggggcatacc atcatgaatt cgctcatcta caatggctca aagtgggaaa gatcaagtgg 1380
agggacccca aggatagctt tctcggacat ctcaattaca cgactgagca ggttgagatg 1440
cacacagtga cagtacagtt gcaggaatcg ttcgcggcaa accacttgta ctgcatcagg 1500
agaggagact tgctcacacc ggaggtgcgc actttcggcc aacctgacag gtacgtgatt 1560
ccaccacaga tcttcctccc aaaagttcac aactgcaaga agccgattct caagaaaact 1620
atgatgcagc tcttcttgta tgttaggaca gtcaaggtcg caaaaaattg tgacattttt 1680
gccaaagtca gacaattaat taaatcatct gacttggaca aatactctgc tgtggaactg 1740
gtttacttag taagctacat ggagttcctt gccgatttac aagctaccac ctgcttctca 1800
gacacacttt ctggtggctt gctaacaaag acccttgcac cggtgagggc ttggatacaa 1860
gagaaaaaga tgcagctgtt tggtcttgag gactacgcga agttagtcaa agcagttgat 1920
ttccacccgg tggatttttc tttcaaagtg gaaacttggg acttcagatt ccaccccttg 1980
caagcgtgga aagccttccg accaagggaa gtgtcggatg tagaggaaat ggaaagtttg 2040
ttctcagatg gggacctgct tgattgcttc acaagaatgc cagcttatgc ggtaaacgca 2100
gaggaagatt tagctgcaat caggaaaacg cccgagatgg atgtcggtca agaagttaaa 2160
gagcctgcag gagacagaaa tcaatactca aaccctgcag aaactttcct caacaagctc 2220
cacaggaaac acagtaggga ggtgaaacac caggccgcaa agaaagctaa acgcctagct 2280
gaaatccagg agtcaatgag agctgaaggt gatgccgaac caaatgaaat aagcgggacg 2340
atgggggcaa tacccagcaa cgccgaactt cctggcacga atgatgccag acaagaactc 2400
acactcccaa ccactaaacc tgtccctgca aggtgggaag atgcttcatt cacagattct 2460
agtgtggaag aggagcaggt taaactcctt ggaaaagaaa ccgttgaaac agcgacgcaa 2520
caagtcatcg aaggacttcc ttggaaacac tggattcctc aattaaatgc tgttggattc 2580
aaggcgctgg aaattcagag ggataggagt ggaacaatga tcatgcccat cacagaaatg 2640
gtgtccgggc tggaaaaaga ggacttccct gaaggaactc caaaagagtt ggcacgagaa 2700

ttgttcgcta tgaacagaag ccctgccacc atccctttgg acctgcttag agccagagac 2760

tacggcagtg atgtaaagaa caagagaatt ggtgccatca caaagacaca ggcaacgagt 2820

tggggcgaat acttgacagg aaagatagaa agcttaactg agaggaaagt tgcgacttgt 2880

gtcattcatg gagctggagg ttctggaaaa agtcatgcca tccagaaggc attgagagaa 2940

attggcaagg gctcggacat cactgtagtc ctgccgacca atgaactgcg gctagattgg 3000

agtaagaaag tgcctaacac tgagccctat atgttcaaga cctctgaaaa ggcgttaatt 3060

gggggaacag gcagcatagt catctttgac gattactcaa aacttcctcc cggttacata 3120

gaagccttag tctgtttcta ctctaaaatc aagctaatca ttctaacagg agatagcaga 3180

caaagcgtct accatgaaac tgctgaggac gcctccatca ggcatttggg accagcaaca 3240

gagtacttct caaaatactg ccgatactat ctcaatgcca cacaccgcaa caagaaagat 3300

cttgcgaaca tgcttggtgt ctacagtgag agaacgggag tcaccgaaat cagcatgagc 3360

gccgagttct tagaaggaat cccaactttg gtaccctcgg atgagaagag aaagctgtac 3420

atgggcaccg ggaggaatga cacgttcaca tacgctggat gccaggggct aactaagccg 3480

aaggtacaaa tagtgttgga ccacaacacc caagtgtgta gcgcgaatgt gatgtacacg 3540

gcactttcta gagccaccga taggattcac ttcgtgaaca caagtgcaaa ttcctctgcc 3600

ttctgggaaa agttggacag caccccttac ctcaagactt tcctatcagt ggtgagagaa 3660

caagcactca gggagtacga gccggcagag gcagagccaa ttcaagagcc tgagccccag 3720

acacacatgt gtgtcgagaa tgaggagtcc gtgctagaag agtacaaaga ggaactcttg 3780

gaaaagtttg acagagagat ccactctgaa tcccatggtc attcaaactg tgtccaaact 3840

gaagacacaa ccattcagtt gttttcgcat caacaagcaa aagatgagac tctcctctgg 3900

gcgactatag atgcgcggct caagaccagc aatcaagaaa caaacttccg agaattcctg 3960

agcaagaagg acattgggga cgttctgttt ttaaactacc aaaaagctat gggtttaccc 4020

aaagagcgta ttcctttttc ccaagaggtc tgggaagctt gtgcccacga agtacaaagc 4080

aagtacctca gcaagtcaaa gtgcaacttg atcaatggga ctgtgagaca gagcccagac 4140

ttcgatgaaa ataagattat ggtattcctc aagtcgcagt gggtcacaaa ggtggaaaaa 4200

ctaggtctac ccaagattaa gccaggtcaa accatagcag ccttttacca gcagactgtg 4260

atgctttttg gaactatggc taggtacatg cgatggttca gacaggcttt ccagccaaaa 4320

gaagtcttca taaactgtga gacgacgcca gatgacatgt ctgcatgggc cttgaacaac 4380

tggaatttca gcagacctag cttggctaat gactacacag ctttcgacca gtctcaggat 4440

ggagccatgt tgcaatttga ggtgctcaaa gccaaacacc actgcatacc agaggaaatc 4500

attcaggcat acatagatat taagactaat gcacagattt tcctaggcac gttatcaatt 4560

atgcgcctga ctggtgaagg tcccactttt gatgcaaaca ctgagtgcaa catagcttac 4620

acccatacaa agtttgacat cccagccgga actgctcaag tttatgcagg agacgactcc 4680

gcactggact gtgttccaga agtgaagcat agtttccaca ggcttgagga caaattactc 4740
ctaaagtcaa agcctgtaat cacgcagcaa aagaagggca gttggcctga gttttgtggt 4800
tggctgatca caccaaaagg ggtgatgaaa gacccaatta agctccatgt tagcttaaaa 4860
ttggctgaag ctaagggtga actcaagaaa tgtcaagatt cctatgaaat tgatctgagt 4920
tatgcctatg accacaagga ctctctgcat gacttgttcg atgagaaaca gtgtcaggca 4980
cacacactca cttgcagaac actaatcaag tcagggagag gcactgtctc actttcccgc 5040
ctcagaaact ttctttaacc gttaagttac cttagagatt tgaataagat gtcagcacca 5100
gctagtacaa cacagcccat agggtcaact acctcaacta ccacaaaaac tgcaggcgca 5160
actcctgcca cagcttcagg cctgttcact atcccggatg gggatttctt tagtacagcc 5220
cgtgccatag tagccagcaa tgctgtcgca acaaatgagg acctcagcaa gattgaggct 5280
atttggaagg acatgaaggt gcccacagac actatggcac aggctgcttg ggacttagtc 5340
agacactgtg ctgatgtagg atcatccgct caaacagaaa tgatagatac aggtccctat 5400
tccaacggca tcagcagagc tagactggca gcagcaatta aagaggtgtg cacacttagg 5460
caattttgca tgaagtatgc cccagtggta tggaactgga tgttaactaa caacagtcca 5520
cctgctaact ggcaagcaca aggtttcaag cctgagcaca aattcgctgc attcgacttc 5580
ttcaatggag tcaccaaccc agctgccatc atgcccaaag aggggctcat ccggccaccg 5640
tctgaagctg aaatgaatgc tgcccaaact gctgcctttg tgaagattac aaaggccagg 5700
gcacaatcca acgactttgc cagcctagat gcagctgtca ctcgaggaag gatcaccgga 5760
acgaccacag cagaggcagt cgttactctg cctcctccat aacagaaact ttctttaacc 5820
gttaagttac cttagagatt tgaataagat ggatattctc atcagtagtt tgaaaagttt 5880
aggttattct aggacttcca aatctttaga ttcaggacct ttggtagtac atgcagtagc 5940
cggagccggt aagtccacag ccctaaggaa gttgatcctc agacacccaa cattcaccgt 6000
gcatacactc ggtgtccctg acaaggtgag tatcagaact agaggcatac agaagccagg 6060
acctattcct gagggcaact tcgcaatcct cgatgagtat actttggaca acaccacaag 6120
gaactcatac caggcacttt ttgctgaccc ttatcaggca ccggagttta gcctagagcc 6180
ccacttctac ttggaaacat catttcgagt tccgaggaaa gtggcagatt tgatagctgg 6240
ctgtggcttc gatttcgaga cgaactcacc ggaagaaggg cacttagaga tcactggcat 6300
attcaaaggg cccctactcg gaaaggtgat agccattgat gaggagtctg agacaacact 6360
gtccaggcat ggtgttgagt ttgttaagcc ctgccaagtg acgggacttg agttcaaagt 6420
agtcactatt gtgtctgccg caccaataga ggaaattggc cagtccacag ctttctacaa 6480
cgctatcacc aggtcaaagg gattgacata tgtccgcgca gggccatagg ctgaccgctc 6540
cggtcaattc tgaaaaagtg tacatagtat taggtctatc atttgcttta gtttcaatta 6600
cctttctgct ttctagaaat agcttacccc acgtcggtga caacattcac agcttgccac 6660

acggaggagc ttacagagac ggcaccaaag caatcttgta caactcccca aatctagggt 6720

cacgagtgag tctacacaac ggaaagaacg cagcatttgc tgccgttttg ctactgactt 6780

tgctgatcta tggaagtaaa tacatatctc aacgcaatca tacttgtgct tgtggtaaca 6840

atcatagcag tcattagcac ttccttagtg aggactgaac cttgtgtcat caagattact 6900

ggggaatcaa tcacagtgtt ggcttgcaaa ctagatgcag aaaccataag ggccattgcc 6960

gatctcaagc cactctccgt tgaacggtta agtttccatt gatactcgaa agaggtcagc 7020

accagctagc aacaaacaag aacatgagag acctcgcgat ttaaatcgat ggtctcagat 7080

cggtcgtatc actggaacaa caaccgctga ggctgttgtc actctaccac caccataact 7140

acgtctacat aaccgacgcc taccccagtt tcatagtatt ttctggtttg attgtatgaa 7200

taatataaat aaaaaaaaaa aaaaaaaaaa aaaactagtg agctcttctg tcagcgggcc 7260

cactgcatcc accccagtac attaaaaacg tccgcaatgt gttattaagt tgtctaagcg 7320

tcaatttgtt tacaccacaa tatatcctgc caccagccag ccaacagctc cccgaccggc 7380

agctcggcac aaaatcacca ctcgatacag gcagcccatc agtc 7424

<210> 6
<211> 6660
<212> DNA
<213> Artificial

<220>
<223> T-DNA region of pNMD694 (Fig. 5)

<400> 6
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag gcatgcctgc 180

aggtcaacat ggtggagcac gacacgcttg tctactccaa aaatatcaaa gatacagtct 240

cagaagacca aagggcaatt gagacttttc aacaaagggt aatatccgga aacctcctcg 300

gattccattg cccagctatc tgtcacttta ttgtgaagat agtggaaaag gaaggtggct 360

cctacaaatg ccatcattgc gataaaggaa aggccatcgt tgaagatgcc tctgccgaca 420

gtggtcccaa agatggaccc ccacccacga ggagcatcgt ggaaaaagaa gacgttccaa 480

ccacgtcttc aaagcaagtg gattgatgtg atatctccac tgacgtaagg gatgacgcac 540

aatcccacta tccttcgcaa gacccttcct ctatataagg aagttcattt catttggaga 600

ggagaaaact aaaccataca ccaccaacac aaccaaaccc accacgccca attgttacac 660

acccgcttga aaaagaaagt ttaacaaatg gccaaggtgc gcgaggttta ccaatctttt 720

acagactcca ccacaaaaac tctcatccaa gatgaggctt atagaaacat tcgccccatc 780

atggaaaaac acaaactagc taacccttac gctcaaacgg ttgaagcggc taatgatcta 840

gaggggttcg gcatagccac caatccctat agcattgaat tgcatacaca tgcagccgct 900

aagaccatag agaataaact tctagaggtg cttggttcca tcctaccaca agaacctgtt 960
acatttatgt ttcttaaacc cagaaagcta aactacatga gaagaaaccc gcggatcaag 1020
gacattttcc aaaatgttgc cattgaacca agagacgtag ccaggtaccc caaggaaaca 1080
ataattgaca aactcacaga gatcacaacg gaaacagcat acattagtga cactctgcac 1140
ttcttggatc cgagctacat agtggagaca ttccaaaact gcccaaaatt gcaaacattg 1200
tatgcgacct tagttctccc cgttgaggca gcctttaaaa tggaaagcac tcacccgaac 1260
atatacagcc tcaaatactt cggagatggt ttccagtata taccaggcaa ccatggtggc 1320
ggggcatacc atcatgaatt cgctcatcta caatggctca aagtgggaaa gatcaagtgg 1380
agggacccca aggatagctt tctcggacat ctcaattaca cgactgagca ggttgagatg 1440
cacacagtga cagtacagtt gcaggaatcg ttcgcggcaa accacttgta ctgcatcagg 1500
agaggagact tgctcacacc ggaggtgcgc actttcggcc aacctgacag gtacgtgatt 1560
ccaccacaga tcttcctccc aaaagttcac aactgcaaga agccgattct caagaaaact 1620
atgatgcagc tcttcttgta tgttaggaca gtcaaggtcg caaaaaattg tgacattttt 1680
gccaaagtca gacaattaat taaatcatct gacttggaca aatactctgc tgtggaactg 1740
gtttacttag taagctacat ggagttcctt gccgatttac aagctaccac ctgcttctca 1800
gacacacttt ctggtggctt gctaacaaag acccttgcac cggtgagggc ttggatacaa 1860
gagaaaaaga tgcagctgtt tggtcttgag gactacgcga agttagtcaa agcagttgat 1920
ttccacccgg tggatttttc tttcaaagtg gaaacttggg acttcagatt ccaccccttg 1980
caagcgtgga aagccttccg accaagggaa gtgtcggatg tagaggaaat ggaaagtttg 2040
ttctcagatg gggacctgct tgattgcttc acaagaatgc cagcttatgc ggtaaacgca 2100
gaggaagatt tagctgcaat caggaaaacg cccgagatgg atgtcggtca agaagttaaa 2160
gagcctgcag gagacagaaa tcaatactca aaccctgcag aaactttcct caacaagctc 2220
cacaggaaac acagtaggga ggtgaaacac caggccgcaa agaaagctaa acgcctagct 2280
gaaatccagg agtcaatgag agctgaaggt gatgccgaac caaatgaaat aagcgggacg 2340
atgggggcaa tacccagcaa cgccgaactt cctggcacga atgatgccag acaagaactc 2400
acactcccaa ccactaaacc tgtccctgca aggtgggaag atgcttcatt cacagattct 2460
agtgtggaag aggagcaggt taaactcctt ggaaaagaaa ccgttgaaac agcgacgcaa 2520
caagtcatcg aaggacttcc ttggaaacac tggattcctc aattaaatgc tgttggattc 2580
aaggcgctgg aaattcagag ggataggagt ggaacaatga tcatgcccat cacagaaatg 2640
gtgtccgggc tggaaaaaga ggacttccct gaaggaactc caaaagagtt ggcacgagaa 2700
ttgttcgcta tgaacagaag ccctgccacc atccctttgg acctgcttag agccagagac 2760
tacggcagtg atgtaaagaa caagagaatt ggtgccatca caaagacaca ggcaacgagt 2820
tggggcgaat acttgacagg aaagatagaa agcttaactg agaggaaagt tgcgacttgt 2880

gtcattcatg gagctggagg ttctggaaaa agtcatgcca tccagaaggc attgagagaa 2940

attggcaagg gctcggacat cactgtagtc ctgccgacca atgaactgcg gctagattgg 3000

agtaagaaag tgcctaacac tgagccctat atgttcaaga cctctgaaaa ggcgttaatt 3060

gggggaacag gcagcatagt catctttgac gattactcaa aacttcctcc cggttacata 3120

gaagccttag tctgtttcta ctctaaaatc aagctaatca ttctaacagg agatagcaga 3180

caaagcgtct accatgaaac tgctgaggac gcctccatca ggcatttggg accagcaaca 3240

gagtacttct caaaatactg ccgatactat ctcaatgcca cacaccgcaa caagaaagat 3300

cttgcgaaca tgcttggtgt ctacagtgag agaacgggag tcaccgaaat cagcatgagc 3360

gccgagttct tagaaggaat cccaactttg gtaccctcgg atgagaagag aaagctgtac 3420

atgggcaccg ggaggaatga cacgttcaca tacgctggat gccaggggct aactaagccg 3480

aaggtacaaa tagtgttgga ccacaacacc caagtgtgta gcgcgaatgt gatgtacacg 3540

gcactttcta gagccaccga taggattcac ttcgtgaaca caagtgcaaa ttcctctgcc 3600

ttctgggaaa agttggacag caccccttac ctcaagactt tcctatcagt ggtgagagaa 3660

caagcactca gggagtacga gccggcagag gcagagccaa ttcaagagcc tgagccccag 3720

acacacatgt gtgtcgagaa tgaggagtcc gtgctagaag agtacaaaga ggaactcttg 3780

gaaaagtttg acagagagat ccactctgaa tcccatggtc attcaaactg tgtccaaact 3840

gaagacacaa ccattcagtt gttttcgcat caacaagcaa aagatgagac tctcctctgg 3900

gcgactatag atgcgcggct caagaccagc aatcaagaaa caaacttccg agaattcctg 3960

agcaagaagg acattgggga cgttctgttt ttaaactacc aaaaagctat gggtttaccc 4020

aaagagcgta ttcctttttc ccaagaggtc tgggaagctt gtgcccacga agtacaaagc 4080

aagtacctca gcaagtcaaa gtgcaacttg atcaatggga ctgtgagaca gagcccagac 4140

ttcgatgaaa ataagattat ggtattcctc aagtcgcagt gggtcacaaa ggtggaaaaa 4200

ctaggtctac ccaagattaa gccaggtcaa accatagcag ccttttacca gcagactgtg 4260

atgctttttg gaactatggc taggtacatg cgatggttca gacaggcttt ccagccaaaa 4320

gaagtcttca taaactgtga gacgacgcca gatgacatgt ctgcatgggc cttgaacaac 4380

tggaatttca gcagacctag cttggctaat gactacacag ctttcgacca gtctcaggat 4440

ggagccatgt tgcaatttga ggtgctcaaa gccaaacacc actgcatacc agaggaaatc 4500

attcaggcat acatagatat taagactaat gcacagattt tcctaggcac gttatcaatt 4560

atgcgcctga ctggtgaagg tcccactttt gatgcaaaca ctgagtgcaa catagcttac 4620

acccatacaa agtttgacat cccagccgga actgctcaag tttatgcagg agacgactcc 4680

gcactggact gtgttccaga agtgaagcat agtttccaca ggcttgagga caaattactc 4740

ctaaagtcaa agcctgtaat cacgcagcaa aagaagggca gttggcctga gttttgtggt 4800

tggctgatca caccaaaagg ggtgatgaaa gacccaatta agctccatgt tagcttaaaa 4860

ttggctgaag ctaagggtga actcaagaaa tgtcaagatt cctatgaaat tgatctgagt 4920

tatgcctatg accacaagga ctctctgcat gacttgttcg atgagaaaca gtgtcaggca 4980

cacacactca cttgcagaac actaatcaag tcagggagag gcactgtctc actttcccgc 5040

ctcagaaact ttctttaacc gttaagttac cttagagatt tgaataagat ggatattctc 5100

atcagtagtt tgaaaagttt aggttattct aggacttcca aatctttaga ttcaggacct 5160

ttggtagtac atgcagtagc cggagccggt aagtccacag ccctaaggaa gttgatcctc 5220

agacacccaa cattcaccgt gcatacactc ggtgtccctg acaaggtgag tatcagaact 5280

agaggcatac agaagccagg acctattcct gagggcaact tcgcaatcct cgatgagtat 5340

actttggaca acaccacaag gaactcatac caggcacttt ttgctgaccc ttatcaggca 5400

ccggagttta gcctagagcc ccacttctac ttggaaacat catttcgagt tccgaggaaa 5460

gtggcagatt tgatagctgg ctgtggcttc gatttcgaga cgaactcacc ggaagaaggg 5520

cacttagaga tcactggcat attcaaaggg cccctactcg gaaaggtgat agccattgat 5580

gaggagtctg agacaacact gtccaggcat ggtgttgagt ttgttaagcc ctgccaagtg 5640

acgggacttg agttcaaagt agtcactatt gtgtctgccg caccaataga ggaaattggc 5700

cagtccacag ctttctacaa cgctatcacc aggtcaaagg gattgacata tgtccgcgca 5760

gggccatagg ctgaccgctc cggtcaattc tgaaaaagtg tacatagtat taggtctatc 5820

atttgcttta gtttcaatta cctttctgct ttctagaaat agcttacccc acgtcggtga 5880

caacattcac agcttgccac acggaggagc ttacagagac ggcaccaaag caatcttgta 5940

caactcccca aatctagggt cacgagtgag tctacacaac ggaaagaacg cagcatttgc 6000

tgccgttttg ctactgactt tgctgatcta tggaagtaaa tacatatctc aacgcaatca 6060

tacttgtgct tgtggtaaca atcatagcag tcattagcac ttccttagtg aggactgaac 6120

cttgtgtcat caagattact ggggaatcaa tcacagtgtt ggcttgcaaa ctagatgcag 6180

aaaccataag ggccattgcc gatctcaagc cactctccgt tgaacggtta agtttccatt 6240

gatactcgaa agaggtcagc accagctagc aacaaacaag aacatgagag acctcgcgat 6300

ttaaatcgat ggtctcagat cggtcgtatc actggaacaa caaccgctga ggctgttgtc 6360

actctaccac caccataact acgtctacat aaccgacgcc taccccagtt tcatagtatt 6420

ttctggtttg attgtatgaa taatataaat aaaaaaaaaa aaaaaaaaaa aaaactagtg 6480

agctcttctg tcagcgggcc cactgcatcc accccagtac attaaaaacg tccgcaatgt 6540

gttattaagt tgtctaagcg tcaatttgtt tacaccacaa tatatcctgc caccagccag 6600

ccaacagctc cccgaccggc agctcggcac aaaatcacca ctcgatacag gcagcccatc 6660

## Description

### FIELD OF THE INVENTION

The present invention relates to a process for transient transfection of plants by spraying the plants with an aqueous suspension containing *Agrobacterium* cells. The invention also provides a process of generating or altering a trait in a plant growing on a field. The invention also relates to a process of producing a protein of interest in a plurality of plants on a field. The invention also relates to a process of protecting crop plants on a field from a pest. Moreover, the invention relates to an aqueous suspension containing cells of an *Agrobacterium* strain, suitable for large scale transient transfection of plants grown on a farm field for the processes of the invention. The invention also relates to the use of particulate inorganic material for transient transfection of plants by spraying with suspensions containing *Agrobacterium* cells and the particulate inorganic material.

### BACKGROUND OF THE INVENTION

Current genetic engineering processes for agriculture are all based on stable genetic modification of crop species, demonstrated first in 1983 (Fraley et al 1983; Barton et al 1983) and commercialized since 1996. Although the agriculture process based on plant stable genetic transformation is a reality today and is a basis of a very successful new practices, it has multiple limitations, the main ones being very long time and high cost required for development of transgenic crops. General consensus among the companies involved in plant biotechnology is that the R&D process requires, depending on the crop species, between 8 and 16 years, and the total average cost is estimated to be between $100 and $150 million. Because of these limitations, after more than 25 years since the discovery of plant genetic transformation process, only a handful traits and few GM crop species have been commercialized thus far.

It is known that plant cells and whole plants can also be re-programmed transiently (i.e. without stable integration of new genetic material on a plant chromosome), and the transient processes, such as viral infections, are all very fast. Such transient processes could in principle allow a very fast modification of plant metabolism in favor of certain traits or products that are of interest to the user. Obviously, such processes require a DNA or RNA vector (a virus or a bacterium), that has been engineered to effectively and safely transfect the plant, with the resultant effect being devoid of undesired side effects. Earlier attempts to use vectors based on plant viruses have been partially successful in that they allow transfection of plants for manufacturing of high-value recombinant proteins such as certain biopharmaceuticals (Gleba et al 2007, 2008; Lico et al 2008). Use of viruses for manipulation of other traits, such as input traits (for example, herbicide resistance, Shiboleth et al 2001; Zhang and Ghabiral 2006) have been described in the literature, but virus transfection introduces so many undesired changes in the infected host that this kind of transient process is not pursued anymore for input traits. Transient processes can also be built around the ability of *Agrobacterium* species to transfer part of its Ti plasmid to eukaryotic, in particular, plant cell. Use of Agrobacterium-based transfection is a basis for genetic manipulations such as genetic transformation protocols and of laboratory transient transfection assays. Industrial applications of *Agrobacterium*-based transfection have also been limited to recombinant protein manufacturing, because the optimal application conditions such as vacuum infiltration of plants with bacterial suspensions cannot be used on a large scale in the field, whereas spraying aerial parts or watering plants with bacterial solutions results in a supposedly very small proportion of plant cells to be transfected, and previous studies simply did not address that specific question. The combination of *Agrobacterium* delivery and use of virus as a secondary messenger in one process has been successful in manufacturing high-value recombinant proteins including complex biopharmaceuticals such as full IgG antibodies. However, when it comes to traits such as input traits or traits requiring subtle targeted reprogramming of plant cell metabolism, this magnifection process has the same limitations as viral vectors have.

There is considerable knowledge in the area of the use of microorganisms for controlling certain processes that require interaction of microbes with plants, including use of microorganisms such as *Lactobacillus* and Saccharomyces yeasts for biomass fermentation (preparation of fermented food, drinks), for biocontrol (*Agrobacterium, Myrotecium,* strains), and use of strains of *Rhizobium* for improved nitrogen fixation. In research papers and patents in which microorganisms have been explored as biocontrol agents, there is a considerable body of knowledge of how the living cells should be applied to plant surfaces; in particular, numerous studies have been performed that identified spraying conditions and adjuvants (wetters, stickers, etc.) to be used in the spray mixtures. Examples of such research are numerous, we refer in this case to just some papers that exemplify the state of the art in this area (Arguelles-Arias et al 2009; Nam et al 2009; reviewed by Johnson 2009).

There are registered *Agrobacterium rhizogenes*/*radiobacter* strains that have been used for decades for control of crown gall disease in vineyards and orchards. There are two commercially used strains, one being a natural strain carrying plasmid (K84), and the other, a genetically modified derivative that has been modified through deletion of the gene necessary for the conjugative plasmid transfer (K1026). (Kerr and Tate 1984; Vicedo et al 1993; Reader et al 2005; Kim et al 2006; reviewed in Moore 1988).

*Agrobacterium tumefaciens* and *A*. *rhizogenes* are broadly used in research laboratories worldwide for transient transfection and stable genetic transformation of plants. These applications are based on the ability of *Agrobacterium* to transfer genetic information to eukaryotic cells. Many of the genetically modified plants cultivated today, such as soybeans, canola and cotton, have been generated through Agrobacterium-mediated genetic transformation. The essential difference between the transient and stable transformation is that in the process of stable transformation, *Agrobacterium-delivered* DNA is eventually integrated into a plant chromosome, and is afterwards inherited by the plant progeny. Such integration events are rare even in laboratory experiments specifically designed to provide massive contacts between plant cells and bacteria; thus for the selection of stable transformants, specific selective screening methods have to be utilized. Subsequently, the knowledge accumulated in this science domain is of limited value to those interested in transient processes that have to be designed so as to have a massive character and affect multiple cells of the plant body.

Transient transfection, on the other hand, takes into account only earlier steps of *Agrobacterium*-driven DNA delivery into a nucleus of a plant cell, along with the fact that such delivered DNA molecules, if properly designed to constitute a transcription unit carrying plant-specific promoter and terminator and a coding part, will be transcribed in a nucleus even in the absence of said DNA integration into a plant chromosome, such expression resulting in a transient reprogramming of a plant cell. Such reprogramming has been first achieved early on and has been developed into a standard laboratory tool for rapid evaluation of different genetic experiments. Whereas there is considerable body of knowledge about *Agrobacterium-*mediated DNA transfer to plant cells, with exception of few cases, that information is limited to laboratory scale experiments, and thus far, there were very few attempts to develop industrial scale applications involving *Agrobacterium* as a DNA vector. One of the limitations of laboratory applications is the fact that *Agrobacterium*-based DNA delivery requires certain treatments that are difficult or impossible to apply in open field or on a large scale. In typical transient experiments, cultured plant cells or parts of plants are treated with an excess of bacteria to provide for maximum delivery. In typical research experiments, one is also interested in expression levels that are not economically viable if done on an industrial scale. In general, the research done in this domain has led the inventors to the conclusion that the parameters seriously affecting transient expression are those allowing for the best interaction access of agrobacteria to plant cells within a plant body. Most such studies utilize vacuum infiltration, injection into plant leaf or surfactant treatment, wounding of plant surface e.g. with razor blades, or combination thereof. In fact, the only group that is developing an *Agrobacterium*-based transfection process for commercial production of recombinant proteins that does not involve further (virus-based) amplification of the original DNA, is the group of Medicago (D'Aoust et al 2008, 2009; Vezina et al, 2009) that is entirely relying on vacuum infiltration as a delivery method. However, because of being based on great excess of bacteria to plant cell ratio, current laboratory protocols used for transient transfection of plants do not have serious translational value, i.e. they cannot be directly replicated on an industrial level. Except in few cases (e.g. Vaquero et al, 1999, D'Aoust et al, 2008, 2009) they also have not addressed quantitatively the issue of efficiency of the transient transfection process. (Examples of such research are multiple, we provide a citation for just a few representative ones: Li et al, 1992; Liu et al, 1992; Clough and Bent, 1998; De Buck et al, 1998, 2000; Chung et al, 2000; Yang et al, 2000; Zambre et al, 2003; Wroblewski et al, 2005; Lee and Yang, 2006; Zhao et al, 2006; Shang et al, 2007; Jones etal, 2009; Li et al, 2009; De Felippes and Weigel, 2010). Except in two cases described below, there were no attempts in the literature to quantify the efficiency of the transient process or to provide sufficient understanding that would lead to potential commercial large-scale exploitation of the phenomenon.

One of the industrial processes being under development today is magnifection, a process that is based on vacuum-infiltration of agrobacteria into leaves of plants. The magnifection process (trademarked by Icon Genetics GmbH as magnICON^{®} and covered by several patents/patent applications) is a simple and indefinitely scalable protocol for heterologous protein expression in plants, which is devoid of stable genetic transformation of a plant, but instead relies on transient amplification of viral vectors delivered to multiple areas of a plant body (systemic delivery) by *Agrobacterium* as DNA precursors. Such a process is in essence an infiltration of whole mature plants with a diluted suspension of agrobacteria carrying T-DNAs encoding viral RNA replicons. In this process, the bacteria assume the (formerly viral) functions of primary infection and systemic movement, whereas the viral vector provides for cell-to-cell (short distance) spread, amplification and high-level protein expression. Initial demonstration that viral infection can be initiated by agrobacteria delivering a viral genome copy into a plant cell comes from the pioneering work of Grimsley et al, 1986, in which a DNA virus has been delivered, and a first, although very inefficient, infection with tmv, a cytoplasmic RNA virus delivered as a DNA copy, came from the work of Turpen et al 1993. Current technology, however, is extremely efficient and a few adult tobacco plants are sufficient for early construct optimization and fast production of milligram to gram quantities of recombinant protein for pre-clinical or clinical evaluation, or, in case of individualized vaccines, for manufacturing. The scale-up (industrial) version is essentially the same, but is built around fully assembled viral vectors (rather than pro-vectors requiring *in planta* assembly) and requires simple apparatuses for high-throughput *Agrobacterium* delivery to whole plants by vacuum infiltration. The process can be scaled up but it requires submersion of aerial parts of plants into bacterial suspension under vacuum (the process involves inverting plants grown in pots or in trays), a procedure that imposes imitations on the volumes of biomass that can be treated in this way, on the throughput of the process, on the ways the plants can be cultivated prior to treatment, and it also carries certain costs that limit the use of the process to high-cost products, such as recombinant biopharmaceuticals only. The magnifection process is efficient as it allows transfection of almost all leaf cells in treated plants, or approximately 50% of the total aerial plant biomass (the rest being stems and petioles). The process has been optimized in many ways, in particular through improvement of viral replicon release through optimization of the posttranslational modification of the primary DNA transcripts (Marillonnet et al, 2005). However, the current process has been built entirely around bacterial delivery methods such as injection into plant leaf or vacuum-infiltration (e.g. Simmons et al, 2009), wounding of leaves (Andrews and Curtis, 2005), or pouring agrobacteria into soil ('agrodrenching', Ryu et al, 2004; Yang et al, 2008), whereas said methods can not be applied for the mass treatment of the plants in a field (reviewed in Gleba et al, 2004, 2007, 2008; Lico et al, 2008; original articles include Giritch et al. 2006; Marillonnet et al., 2004, 2005; Santi et al, 2006; and ideologically similar papers from other research groups - Voinnet et al, 2003; Sudarshana et al, 2006; Gao et al, 2006; Mett et al, 2007; Lindbo, 2007a,b; Plesha et al, 2007, 2009; Huang et al, 2006; Regnard et al 2009; Green et al, 2009; Shoji et al, 2009).

It should be mentioned that although *Agrobacterium tumefaciens* and *A*. *thizogenes* are the DNA vectors that are used in the majority of cases, there are other species of bacteria that can perform similar DNA transfer to plant cells (Broothaerts et al, 2005).

The attempts to quantify *Agrobacterium* treatment on whole plants after vacuum-infiltration have been performed by a few research groups only. In the papers of Joh et al, 2005, 2006, it has been concluded that the highest used bacteria density of 10⁹ cfu/ml was the best (as opposed to 10⁸ cfu/ml or 10⁷ cfu/ml), as measured by the total recombinant protein expression. In experiments of Lindbo, 2007a,b, essentially similar results have been as in our work, however, no counting of transfected cells has been performed and the conclusions were derived from recombinant protein expression levels.

The attempts to use *Agrobacterium* treatment on whole plants without vacuum-infiltration result in a very low number of initially transfected cells, thus greatly limiting the practical application of the process. One way of circumventing this initial limitation disclosed in literature is the use of an efficient secondary messenger such as a plant virus that would allow amplifying the initially inefficient process by complementing it with a virus-based cell-to-cell and systemic movement (Azhakanandam et al, 2007). The Agrobacterium-based delivery of DNA copies of plant viruses or plant viral vectors has been described a long time ago (Grimsley et al, 1986, and for TMV - Turpen et al, 1993), and it allows to spread initial replicon delivered to a few cells in plant to the rest of the body by using viral infection process such as cell-to-cell movement and systemic movement of virus. Such a process has limited practical utility for our purposes, because viral infection dramatically changes plant performance; all currently entertained applications are in the area of recombinant protein manufacturing in plants (reviewed in around Gleba et al, 2007, 2008). It should also be noted that the cited paper of Azhakanandam et al, 2007 does not even attempt to quantify the efficiency of the initial transfection and it is based on very high amount of agrobacteria in the transfection media.

Departing from the prior art, it is an object of the present invention to provide an efficient process of transiently transfecting plants so as to be applicable to many plants growing on a farm field. It is also an object of the invention to provide a process of altering a trait in plants growing on a farm field. Notably, it is an object of the invention to provide an efficient process allowing transient plant transfection using *Agrobacterium* on a large scale without the need for the application of pressure differences to introduce *Agrobacterium* into the intercellular space of plants. It is also an object to provide an *Agrobacterium* formulation suitable for this purpose.

### SUMMARY OF THE INVENTION

Accordingly, the present invention provides the following:
(1) A process of transfecting a plant, comprising spraying said plant such as aerial parts of said plant with an aqueous suspension containing cells of an *Agrobacterium* strain and at least one abrasive in said suspension, said *Agrobacterium* strain comprising a DNA molecule comprising a nucleic acid construct containing a DNA sequence of interest. Said DNA sequence of interest may encode a protein or RNA to be expressed in said plant.
(2) A process of generating or altering a trait in a plant, comprising
   providing said plant;
   expressing, in said plant, a protein or an RNA capable of generating or altering said trait, comprising spraying aerial parts of said plants with an aqueous suspension containing cells of an *Agrobacterium* strain and at least one abrasive suspended in said suspension,
   said *Agrobacterium* strain comprising a DNA molecule comprising a nucleic acid construct containing a DNA sequence of interest, said DNA sequence of interest encoding said protein or said RNA.
(3) A process of generating or altering a trait in a plant, comprising
   (i) growing said plant up to a desired growth state;
   (ii) expressing, in said plant, a protein or an RNA capable of generating or altering said trait, comprising spraying aerial parts of said plant with an aqueous suspension containing cells of an *Agrobacterium* strain and at least one abrasive suspended in said suspension,
      said *Agrobacterium* strain comprising a DNA molecule comprising a nucleic acid construct containing a DNA sequence of interest, said DNA sequence of interest encoding said protein or said RNA.
(4) A process of producing a protein of interest in a plant, comprising
   (i) growing said plant up to a desired growth state;
   (ii) expressing, in said plant, said protein of interest, comprising spraying aerial parts of said plant with an aqueous suspension containing cells of an *Agrobacterium* strain and at least one abrasive suspended in said suspension,
      said *Agrobacterium* strain comprising a DNA molecule comprising a nucleic acid construct containing a DNA sequence of interest, said DNA sequence of interest encoding said protein of interest.
(5) A process of protecting crop plants on a field from a pest, comprising
   (i) growing said plants in soil of said field;
   (ii) determining, in a desired growth state of said plants, infestation of at least one of said plants by a pest;
   (iii) expressing, in said plants, a protein or an RNA that is detrimental to the pest determined in the previous step, comprising spraying aerial parts of said plants with an aqueous suspension containing cells of an *Agrobacterium* strain and at least one abrasive suspended in said suspension,
      said *Agrobacterium* strain comprising a DNA molecule comprising a nucleic acid construct containing a DNA sequence of interest operably linked to a promoter, said DNA sequence of interest encoding said protein or said RNA.
(6) The process according to any one of (1) to (5), wherein said aqueous suspension contains said cells of said *Agrobacterium* strain in a concentration of at most 2.2·10⁷, preferably of at most 1.1·10⁷, more preferably of at most 4.4·10⁶, more preferably of at most 1.1·10⁶ cfu/ml of said suspension.
(7) The process according to any one of (1) to (6), wherein said abrasive is a particulate inorganic carrier for wettable powders, such as silica or carborundum.
(8) The process according to (7), wherein said aqueous suspension contains said abrasive in an amount of between 0.02 and 2, preferably between 0.05 and 1 and more preferably between 0.1 and 0.5 % by weight of said suspension.
(9) The process according to (7) or (8), wherein the median particle size of the abrasive added to the suspension is between 0.1 and 30, preferably between 0.1 and 10, more preferably between 0.5 and 10, and most preferably between 0.5 and 5 µm.
(10) The process according to any one of (7) to (9), wherein the abrasive has a D90 value of at most 40 µm, preferably of at most 30 µm; and wherein the abrasive does not contain particles having a size above 45 µm, preferably not above 40 µm.
(11) The process according to any one of (1) to (10), wherein said suspension further comprises an agricultural spray adjuvant, preferably a non-ionic surfactant or wetting agent.
(12) The process according to (11), wherein the spray adjuvant is an organo-silicone wetting agent, such as Silwet L-77.
(13) The process according to any one of (1) to (12), wherein said nucleic acid construct is flanked by a T-DNA border sequence on at least one side, which permits the transfer of said nucleic acid construct into cells of said plant.
(14) The process according to any one of (1) to (13), wherein said nucleic acid construct encodes a replicating viral vector encoding said protein of interest, said replicating viral vector being incapable of system movement in said plant.
(15) The process according to any one of (1) to (13), wherein said DNA sequence of interest is operably linked to a promoter active in plant cells.
(16) Aqueous suspension containing cells of an *Agrobacterium* strain and at least one abrasive suspended in said suspension, wherein said aqueous suspension contains said cells of said *Agrobacterium* strain in a concentration of at most 4.4·10⁷, preferably of at most 1.1·10⁷, preferably of at most 4.4·10⁶, more preferably of at most 1.1·10⁶ cfu/ml of said suspension; said *Agrobacterium* strain comprising a heterologous DNA molecule comprising a nucleic acid construct containing a heterologous DNA sequence of interest that may be operably linked to a promoter; said suspension optionally further comprising a preferably non-ionic wetting agent such as an organosilicone surfactant.
(17) The process according to any one of (1) to (15), wherein said plant is dicotyledonous plant.
(18) The process according to (17), wherein said plant is tobacco or other species of the *Nicotiana* genus, sugar beets or other species of the *Beta* genus, tomato, potato, pepper, soybean, alfalfa, pea, beans, rapeseed or other species of the *Brassica* genus, cotton.
(19) The process according to any one of (1) to (15), wherein said plant is monocotyledonous plant.
(20) The process according to (19), wherein said plant is rice, maize, wheat, barley, oats, millet, sorghum.
(21) The process according to (4), wherein said protein is a cellulase used in saccharification of cellulose or hemicellulose polymers.
(22) Use of particulate inorganic material for transient transfection of plants. In said use, plants may be sprayed with an aqueous suspension containing *Agrobacterium* cells and the particulate inorganic material.
(23) A process of transfecting a plant, comprising spraying aerial parts of said plant with an aqueous suspension containing at least one abrasive in said suspension, followed by spraying said aerial parts of said plant with an aqueous suspension containing cells of an *Agrobacterium* strain comprising a DNA molecule comprising a nucleic acid construct containing a DNA sequence of interest. Said DNA sequence of interest may encode a protein or RNA to be expressed in said plant.

The inventors of the present invention have found a way of strongly increasing the probability of achieving plant transfection by *Agrobacerium.* The inventors have found that addition of a particulate material insoluble in aqueous *Agrobacterium* suspensions strongly increases the transfection efficiency achievable by spraying of aerial parts of the plant with the suspension. The high efficiency achieved allows for the first time transfection of plants with *Agrobacterium* suspensions on a large scale such as on agricultural fields, whereby the cumbersome infiltration methods making use of pressure differences can be avoided. The invention also allows transfection of plants that have so far not been amenable to spray transformation with *Agrobacterium* suspensions.

### DESCRIPTION OF THE FIGURES

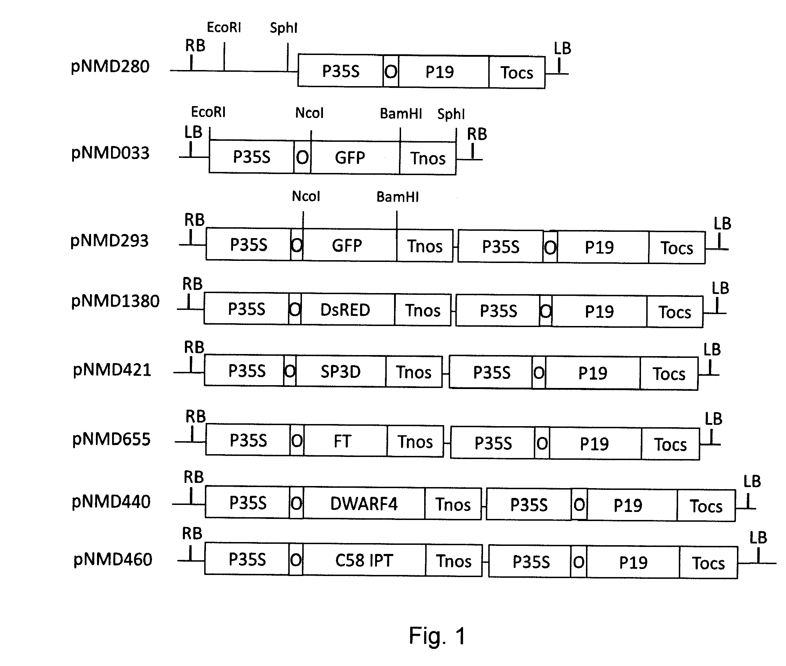

Fig. 1 shows schematically vectors used in the examples. RB and LB stand for the right and left borders of T-DNA. P35S: cauliflower mosaic virus 35S promoter; O: omega translational enhancer; Tnos: nopaline synthase terminator; Tocs: ocs terminator.

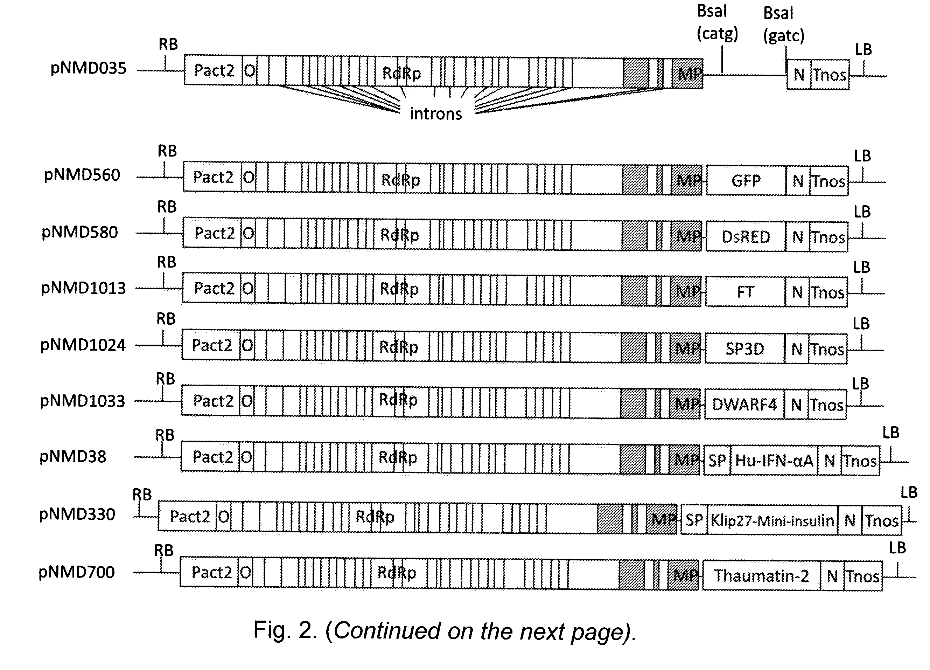

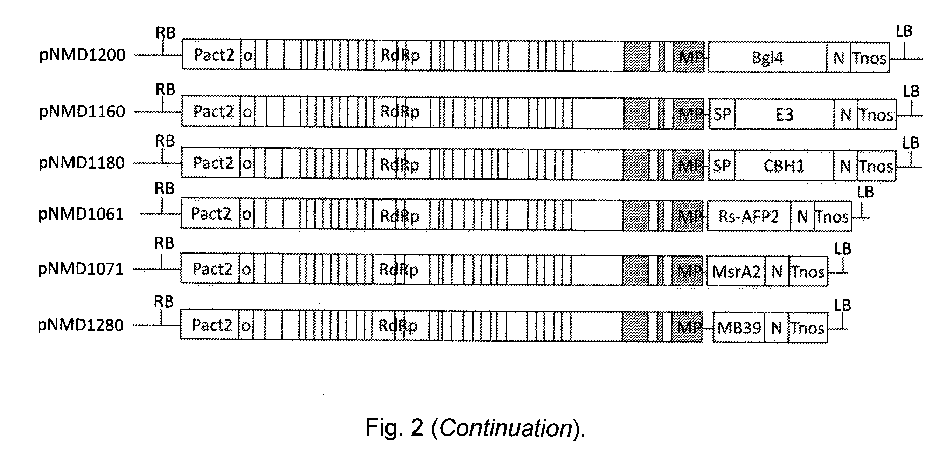

Fig. 2 depicts TMV-based viral vectors with cell-to cell movement ability. Pact2: promoter of *Arabidopsis* actin2 gene; o: 5' end from TVCV (turnip vein clearing virus); RdRp: RNA-dependent RNA polymerase open reading frame (ORF) from cr-TMV (crucifer-infecting tobamovirus); MP: movement protein ORF from cr-TMV; N: 3'-non-translated region from cr-TMV; Tnos or nos: nopaline synthase terminator; SP: signal peptide; white segments interrupting grey segments in the RdRp and MMP ORFs indicate introns inserted into these ORFs for increasing the likelihood of RNA replicon formation in the cytoplasm of plant cells, which is described in detail in WO2005049839.

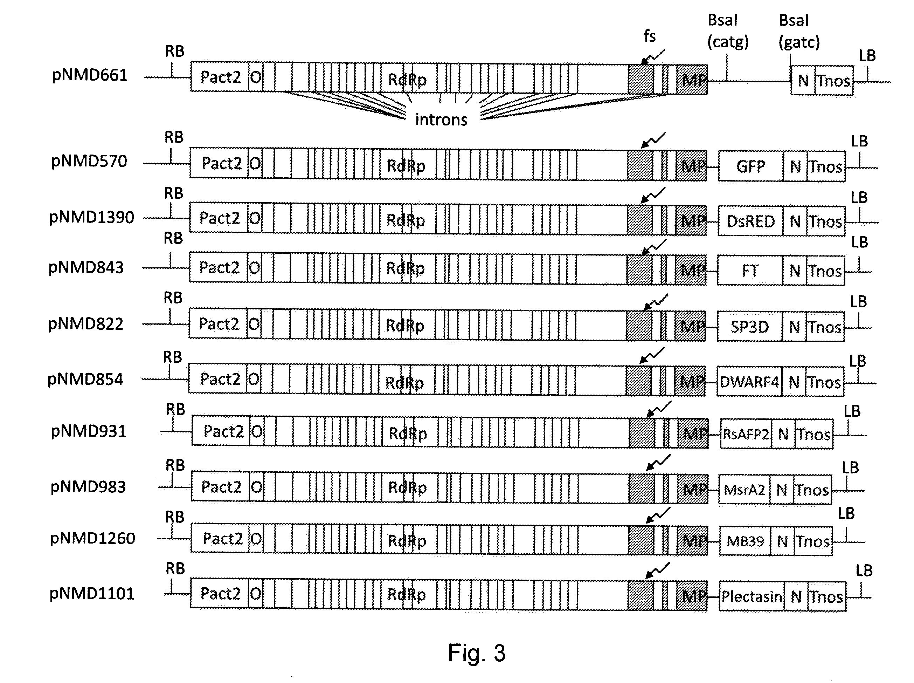

Fig. 3 depicts TMV-based vectors lacking cell-to cell movement ability. A point mutation in the MP ORF leads to a frame shift preventing correct MP translation.

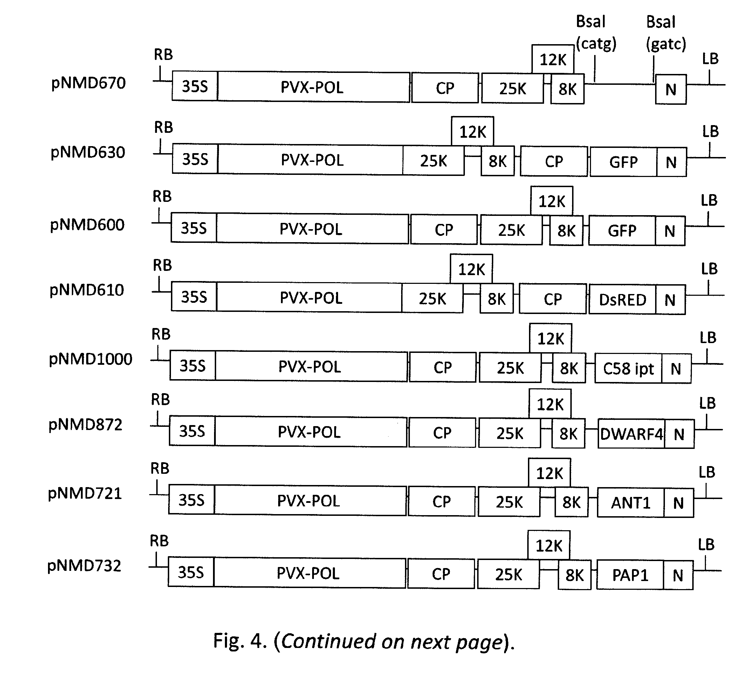

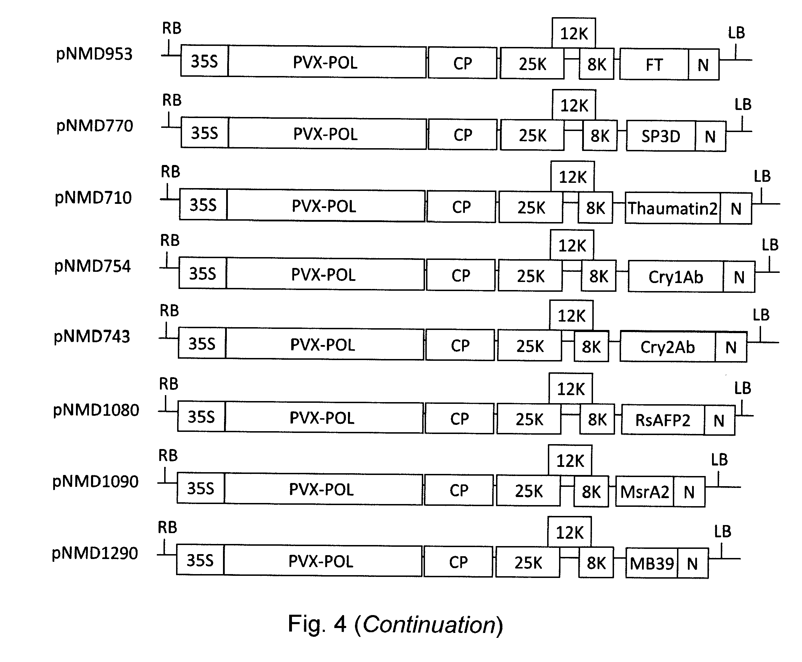

Fig. 4 depicts PVX (potato virus X)-based vectors with cell-to-cell movement ability. PVX-pol: RNA-dependent RNA polymerase from PVX; CP: coat protein ORF; 25K, 12K and 8 together indicate the 25KDA, 12 kDa and 8 kDa triple gene block modules from PVX; N: 3'-untranslated region from PVX.

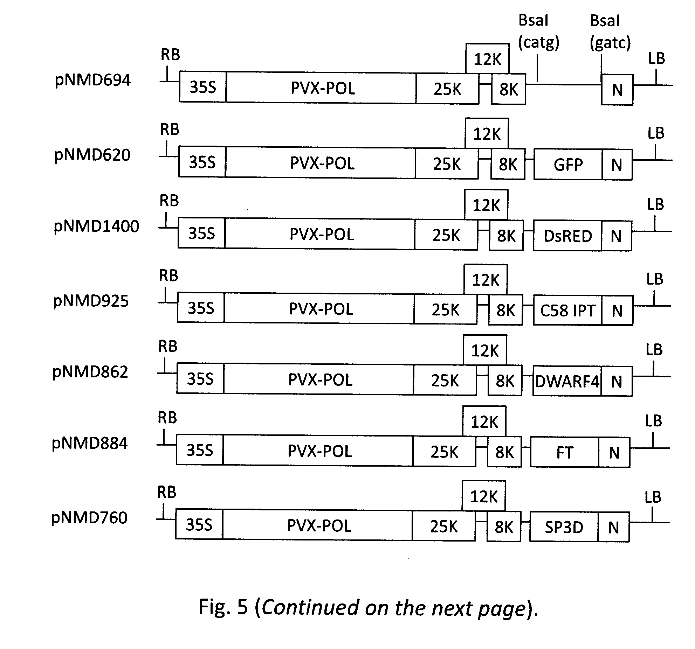

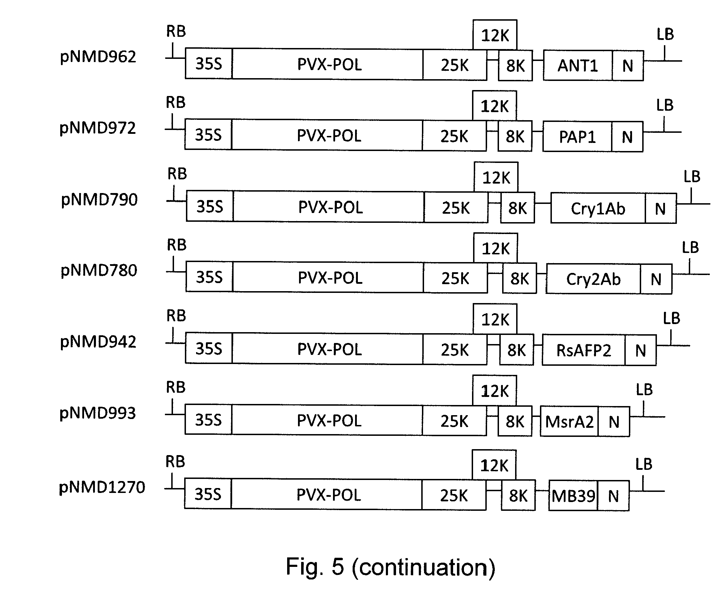

Fig. 5 depicts PVX-based vectors with deletion of the coat protein coding sequence were disabled for both systemic and cell-to cell movement.

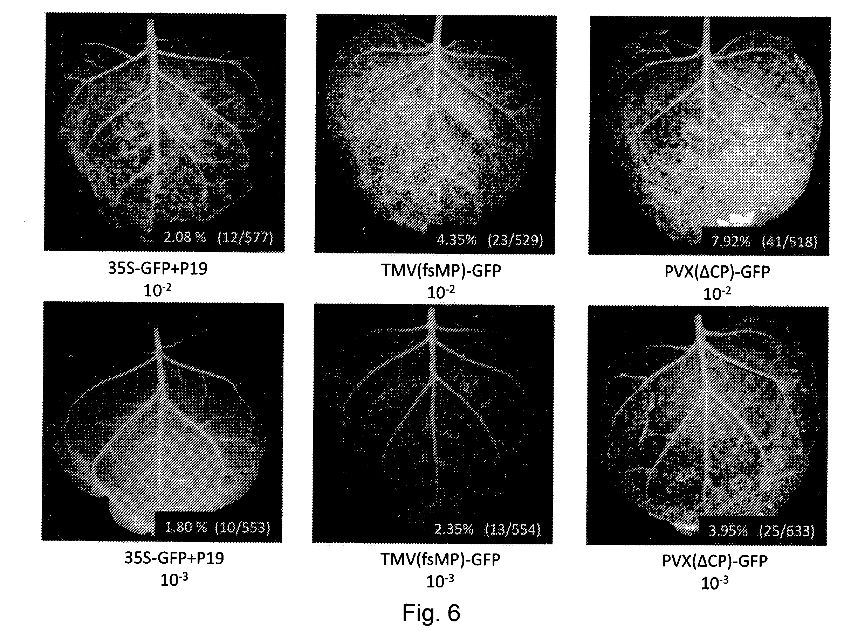

Fig. 6 photographs showing GFP fluorescence 4 dpi under uv light due to GFP expression after dipping leaves from *Nicotiana benthamiana* plants for 1 minute into diluted agrobacterial cultures containing 0.1 % by weight of the surfactant Silwet L-77 as described in Example 2. Numerals 10⁻² and 10⁻³ show the dilution factor of the overnight agrobacterial cultures of OD=1.5 at 600 nm. The vectors used in indicated and can be associated with the appropriate vector shown in Fig. 1 to 5. 35S-GFP+P19-transcriptional vector expressing GFP under the control of 35S promoter and co-expressed with P19 suppressor of silencing (pNMD293); TMV(fsMP)-GFP and PVX(ΔCP)-GFP-viral vectors lacking cell-to-cell movement ability (pNMD570 and pNMD620, respectively). The percentage of GFP-expressing cells (indicated) was counted after the isolation of protoplasts from the left half of the leaf blade.

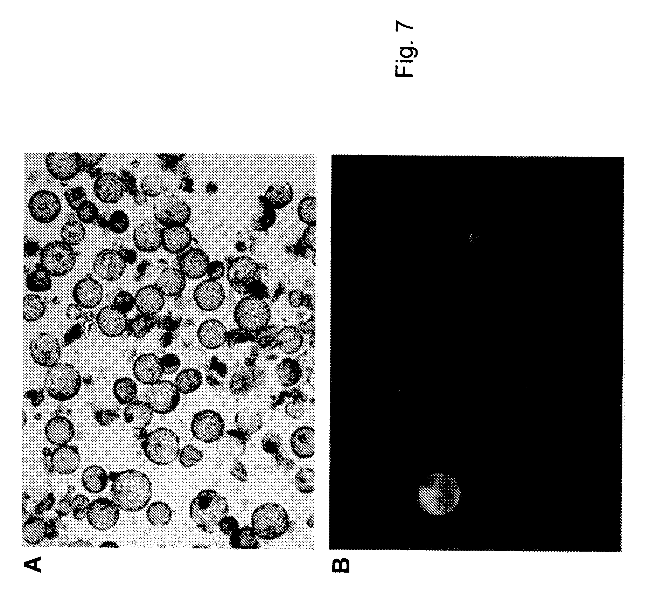

Fig. 7 shows photographs of isolated protoplasts for counting of GFP expressing cells. GFP-expressing protoplasts isolated after the dipping of *Nicotiana benthamiana* leaves in agrobacterial suspension (OD600=1.5), dilution factor 10⁻³. TMV(fsMP)-GFP-TMV-based viral vector lacking cell-to-cell movement ability (pNMD570). 0.1 % Silwet, 1 min dipping, protoplasts were isolated at 4dpi.

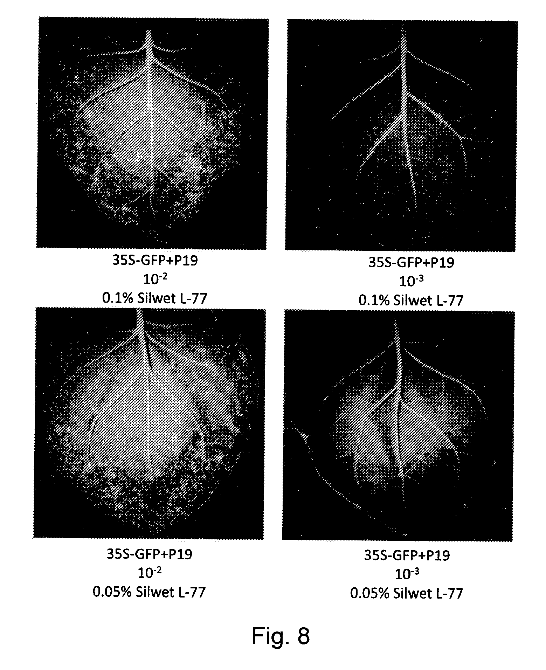

Fig. 8 Influence of Silwet L-77 concentration and agrobacterial culture density on the transfection efficiency after the dipping of *Nicotiana benthamiana* leaves in agrobacterial suspension (OD600=1.5), dilution factors 10⁻² and 10⁻³. 35S-GFP + P19 - transcriptional vector expressing GFP under the control of 35S promoter and co-expressed with P19 suppressor of silencing (pNMD293). Concentration of Silwet 0.1 % and 0.05%, 10 sec dipping, 8 dpi.

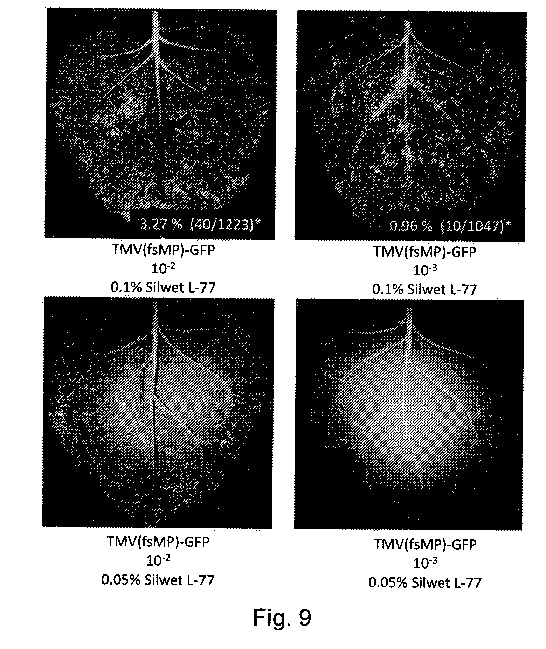

Fig. 9 Influence of Silwet L-77 concentration and agrobacterial culture density on the transfection efficiency after the dipping of *Nicotiana benthamiana* leaves in agrobacterial suspension (OD600=1.5), dilution factors 10⁻² and 10⁻³. TMV(fsMP)-GFP - TMV-based viral vector lacking cell-to-cell movement ability (pNMD570). Concentration of Silwet - 0.1 % and 0.05%, 10 sec dipping, 8 dpi. Percent of GFP-expressing cells was counted after the isolation of protoplasts from the left half of the leaf blade.

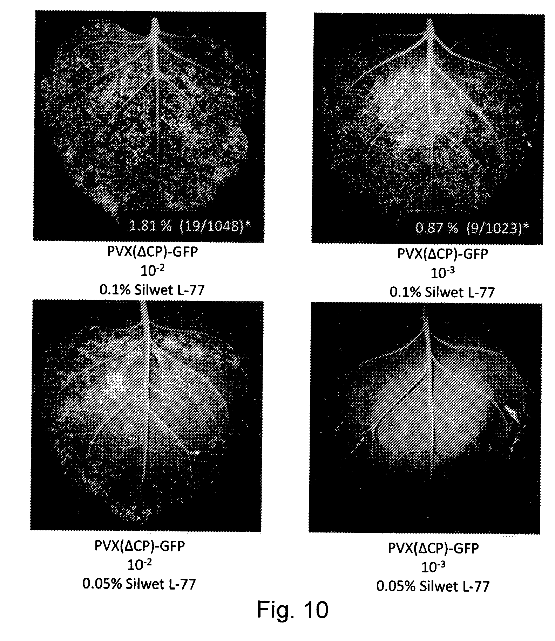

Fig. 10 Influence of Silwet L-77 concentration and agrobacterial culture density on the transfection efficiency after the dipping of Nicotiana benthamiana leaves in agrobacterial suspension (OD600=1.5), dilutions factors 10⁻² and 10⁻³. PVX(ΔCP)-GFP - PVX-based viral vector lacking cell-to-cell movement ability (pNMD620). Concentration of Silwet 0.1 % and 0.05%, 10 sec dipping, 8 dpi. Percent of GFP-expressing cells was counted after the isolation of protoplasts from the left half of the leaf blade.

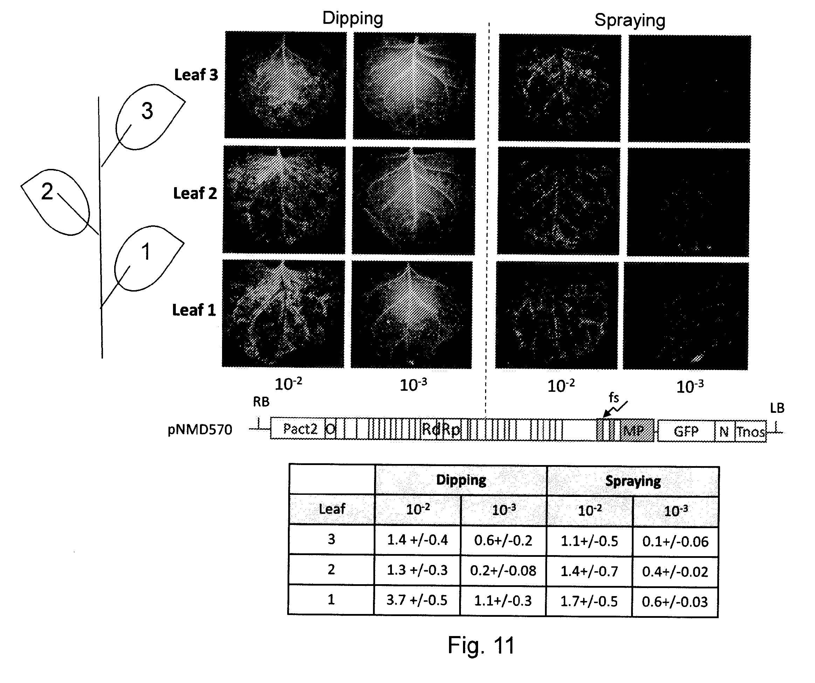

Fig. 11 Comparison of transfection rates achieved by dipping and spraying *Nicotiana benthamiana* into/with agrobacterial suspensions. Dilution factors and transfection rates are indicated. The Silwet L-77 concentration was 0.1 weight %. Agrobacterial culture (pNMD570, TMV-based viral vector lacking cell-to-cell movement ability) was grown to OD600=1.5 and diluted 10 -2 and 10 -3 in buffer for infiltration supplemented with 0.1% Silwet-77. Dipping duration 10 sec. Pictures are taken at 8 dpi. Percent of GFP-expressing cells was counted after the isolation of protoplasts from the left half of the leaf blade.

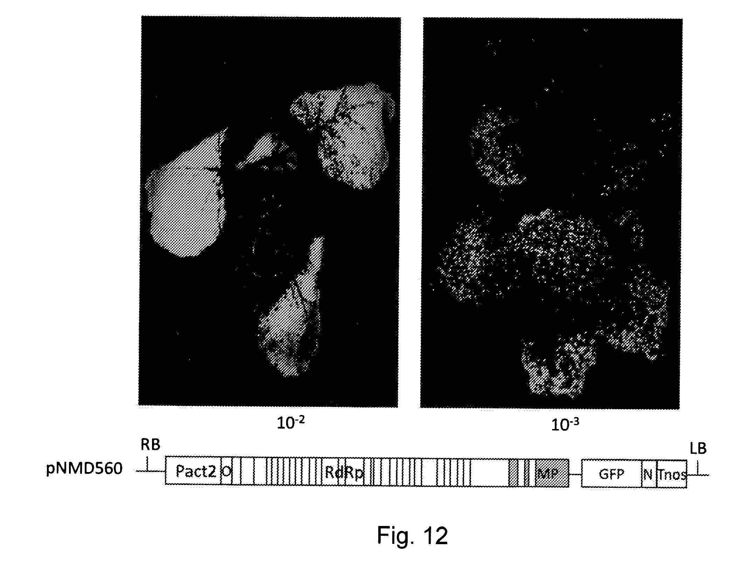

Fig. 12 shows photographs of GFP expression after delivery of diluted agrobacteria to *Nicotiana benthamiana* by spraying with surfactant: TMV-based viral vector with cell-to-cell movement ability (TMV-GFP, pNMD560); agrobacterial suspensions of OD600=1.5 were diluted by dilution factors of 10⁻² or 10⁻³ as indicated; 0.1 % SilwetL-77, photograph taken 8dpi.

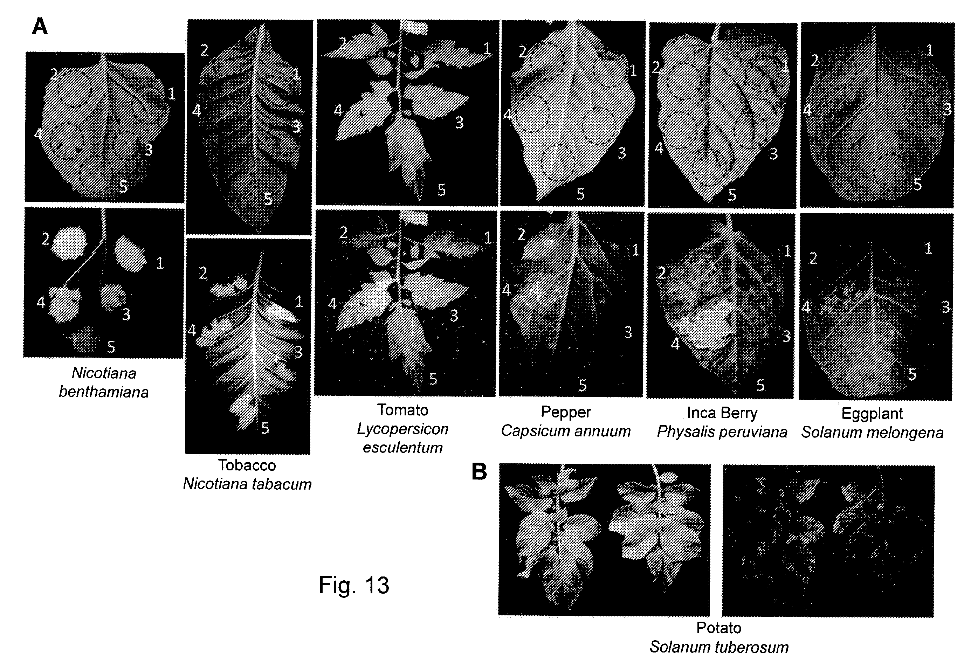

Fig. 13A shows the results of transfection experiments by infiltrating (using a needleless syringe) different plants with 100-fold (dilution factor 10⁻²) dilutions of OD600=1.5 agrobacterial cultures. The suspensions used for spraying contained 0.1 % by weight Silwet L-77 as described in example 3. For each case, the same leaf is shown under normal light and under uv light showing GFP expression. Dashed circles indicate the treated leaf area. Numerals next to the treated leaf area indicate the strain/vector used as follows:
   A) Syringe infiltration. Vectors: 1 - TMV(fsMP)-GFP (pNMD570); 2 - TMV(MP)-GFP pNMD560); 3 - PVX(ΔCP)-GFP (pNMD620); 4 - PVX(CP)-GFP (pNMD630); 5 - 35S-GFP + P19 (pNMD293). Agrobacterial cultures were grown to OD600=1.5 and diluted 100 fold; photographs taken 8 dpi.
   B) Similar as in A, however transfection was performed by vacuum infiltration. Vector: PVX(CP)-GFP (pNMD630). Agrobacterial cultures were grown to OD600=1.5 and diluted 100-fold; photographs taken 43 dpi.

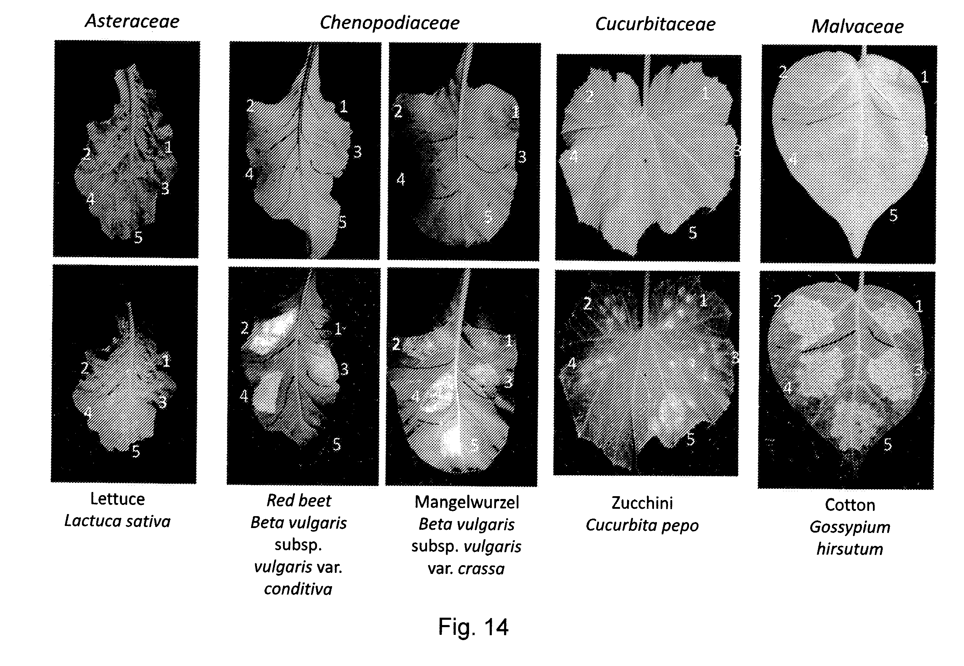

Fig. 14 Screening for optimal expression vector using syringe infiltration with family members of Asteraceae, Chenopodiaceae, Cucurbitaceae and Malvaceae. Numerals indicate the strains/vectors used as follows: 1 - TMV(fsMP)-GFP (pNMD570); 2 - TMV(MP)-GFP (pNMD560); 3 - PVX(ΔCP)-GFP pNMD620); 4 - PVX(CP)-GFP (pNMD630); 5 - 35S-GFP + P19 (pNMD293). Dilution factor of agrobacterial cultures (OD600=1.5-1.7): 10⁻²; photographs taken 8 dpi.

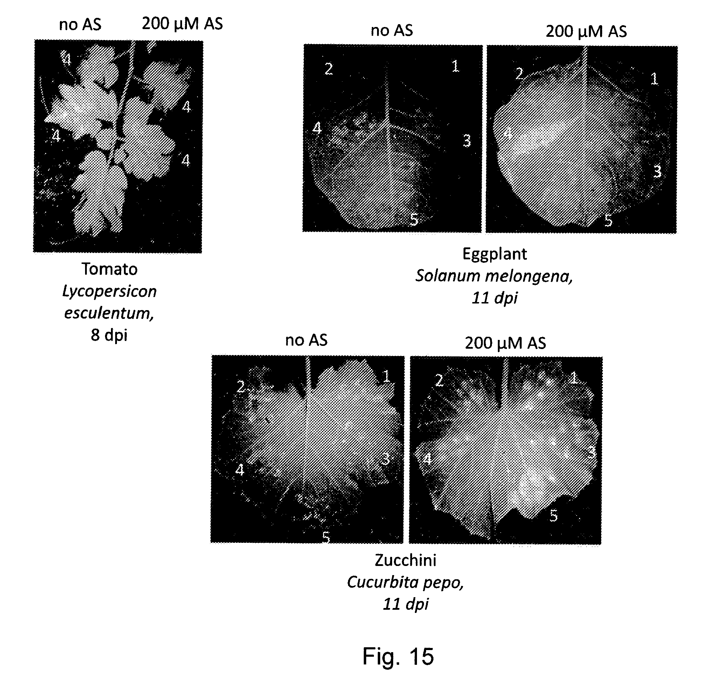

Fig. 15 shows factors enhancing agrobacterial transfection: acetosyringone (AS). Vectors: 1 - TMV(fsMP)-GFP (pNMD570); 2 - TMV(MP)-GFP (pNMD560); 3 - PVX(ΔCP)-GFP (pNMD620); 4 - PVX(CP)-GFP (pNMD630); 5 - 35S-GFP + P19 (pNMD293). Dilution of agrobacterial cultures (OD600=1.5-1.7): 10⁻². For AS treatment, 200 µM acetosyringone (AS) was added to agrobacterial suspensions 2 hours before transfection. For comparison, leaves transfected with suspensions not containing AS are also shown (no AS).

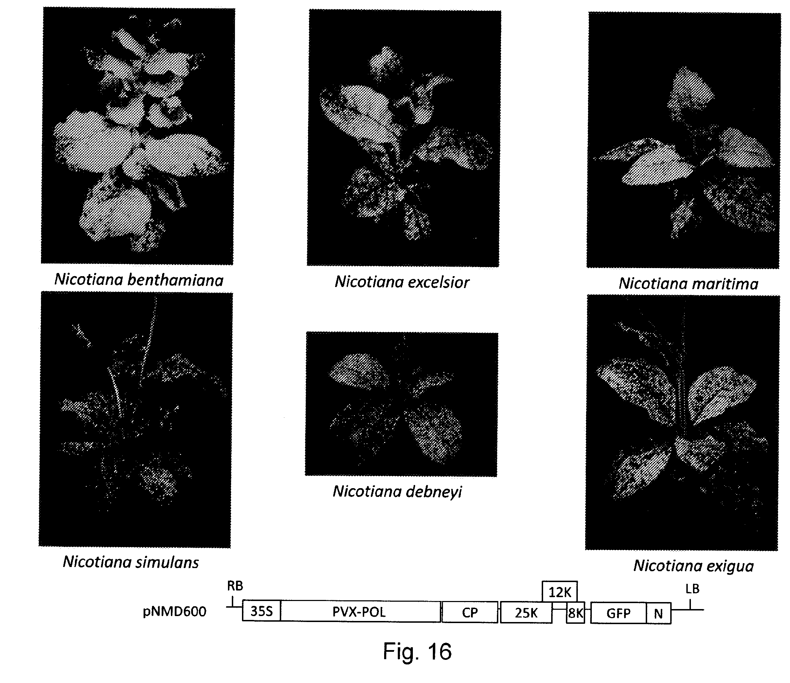

Fig. 16 Photographs showing GFP expression under uv light in various *Nicotiana* species after spraying entire plants with 1000-fold diluted agrobacterial suspensions of OD600=1.0. PVX-based viral vector with cell-to-cell and systemic movement ability were used (PVX(+CP)-GFP, pNMD600). Sprayed suspensions contained 0.1 weight % Silwet L-77. photographs taken 12 dpi.

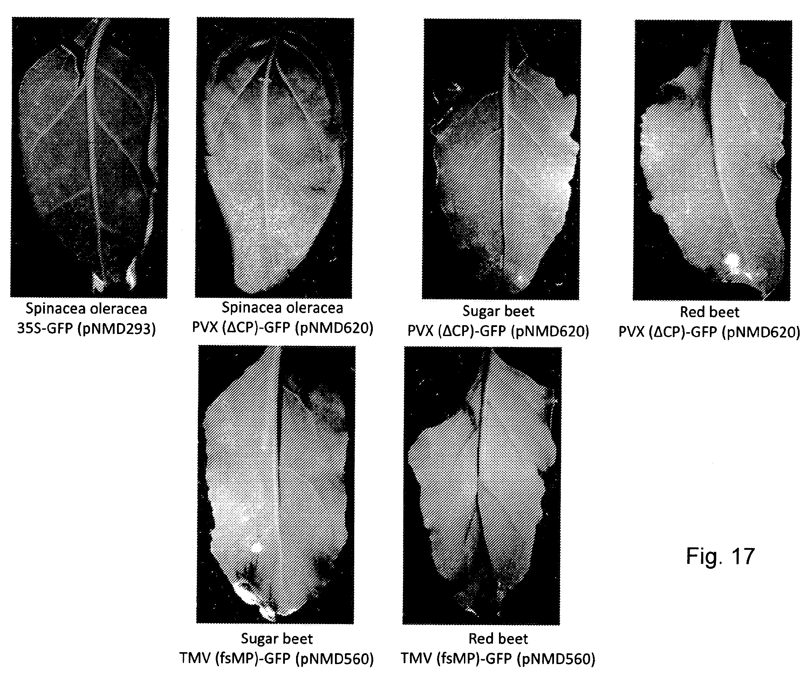

Fig. 17 shows GFP expression after delivery of diluted agrobacteria to spinach and beet plants by dipping with surfactant. A transcriptional vector as well as TMV- and PVX-based viral vectors without cell-to-cell movement ability were used. Agrobacterial cultures were grown to OD600=1.5 and diluted 1:100; 0.1 % Silwet L-77, dipping for 10 sec, photographs taken 12 dpi.

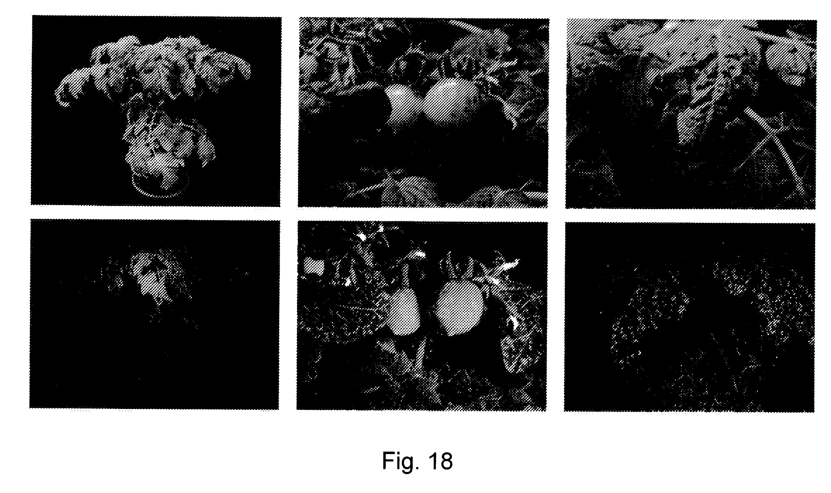

Fig. 18 shows GFP expression after delivery of diluted agrobacteria to tomato *Lycopersicon esculentum* plants by spraying in the presence of surfactant. The vector used was PVX(CP)-GFP (pNMD630). Dilution factor of agrobacterial culture (OD600=1.5): 10⁻², 0.1 % Silwet L-77, 200 µM acetosyringone; photographs taken 14 dpi.

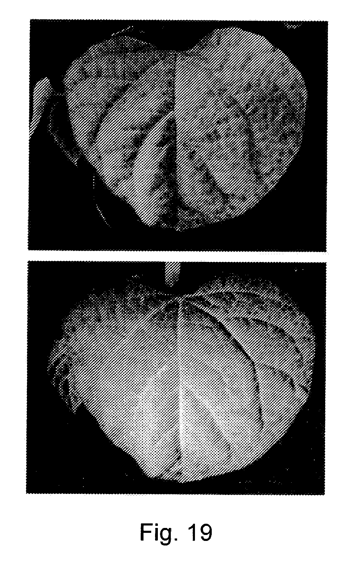

Fig. 19 shows GFP expression after delivery of diluted agrobacteria to Inca berry *Physalis peruviana* plants by spraying with surfactant. Vector PVX(CP)-GFP (pNMD630). Dilution of agrobacterial culture (OD600=1.5): 10⁻², 0.1 % Silwet L-77, 200 µM acetosyringone; photographs taken 14 dpi.

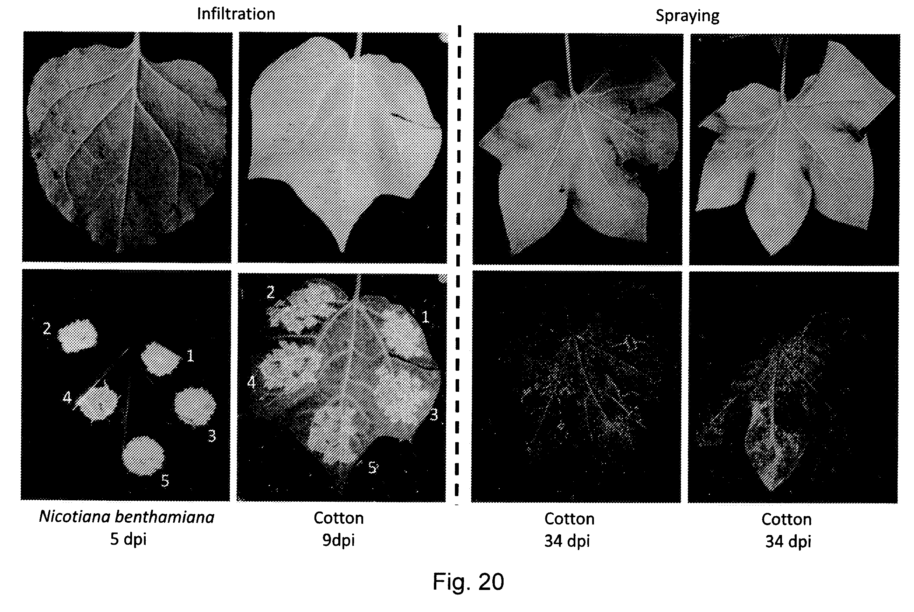

Fig. 20 shows a comparison between transfection by infiltration using a syringe and spraying. Numerals indicate the *Agrobacterium* strain/vector used. Delivery of diluted agrobacteria to cotton *Gossypium hirsutum* L. leaves by using syringe infiltration and spraying with agrobacterial suspensions. Overnight agrobacterial cultures (ICF320 strain) were grown to OD600=1.7-2.0, diluted by a factor of 10⁻² with buffer for infiltration, and incubated with 200µM acetosyringone for 2 hours before use. For spraying, agrobacterial suspensions were additionally supplemented with 0.1 % Silwet L-77.
   Infiltration: 1 - TMV(fsMP)-GFP (pNMD570); 2 - TMV(MP)-GFP (pNMD560); 3 - PVX(ΔCP)-GFP (pNMD620); 4 - PVX(CP)-GFP (pNMD630); 5 - 35S-GFP + P19 (pNMD293).
   Spraying: TMV(MP)-GFP (pNMD560).
   *Nicotiana benthamiana* plant was used as a positive control.

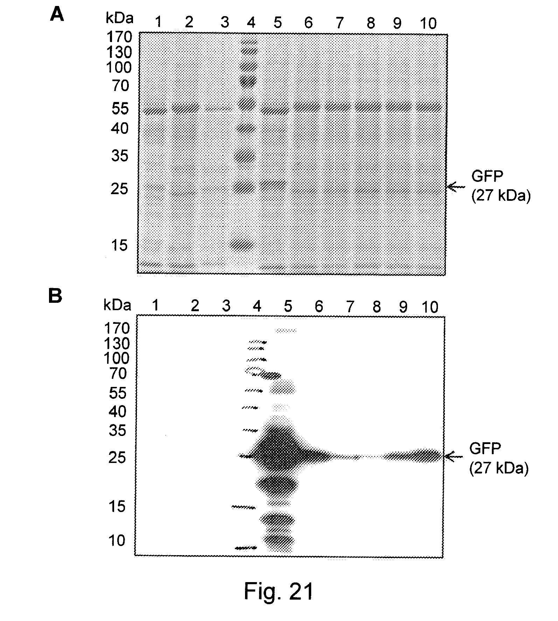

Fig. 21 Shows GFP expression in leaves of cotton *Gossipium* hirsutum L. infiltrated with suspension of agrobacteria carrying transcriptional and viral vectors.
   A) SDS-PAGE with Coomassie staining; B) Western blot probed with anti-GFP antibody (1:3000), second Antibody: anti mouse-HRP (1:5000). The lanes are as follows:
      1- N.benthamiana uninfected leaf;
      2- cotton uninfected leaf;
      3- red cabbage uninfected leaf;
      4- Protein ladder (Fermentas, #SM0671);
      5- TMV(fsMP)-GFP (pNMD570) in Nicotiana benthamiana;
      6- TMV(fsMP)-GFP (pNMD570) in cotton;
      7- TMV(MP)-GFP (pNMD560) in cotton;
      8- PVX(ΔCP)-GFP (pNMD620) in cotton;
      9- PVX(CP)-GFP (pNMD630) in cotton;
      10- 3559-GFP+P19 (pNMD293) in cotton.
      100 mg of leaf material were boiled in 600 µl of 1 x Laemmli buffer containing beta-mercaptoethanol; 2.5 µl aliquots were loaded on the gel.

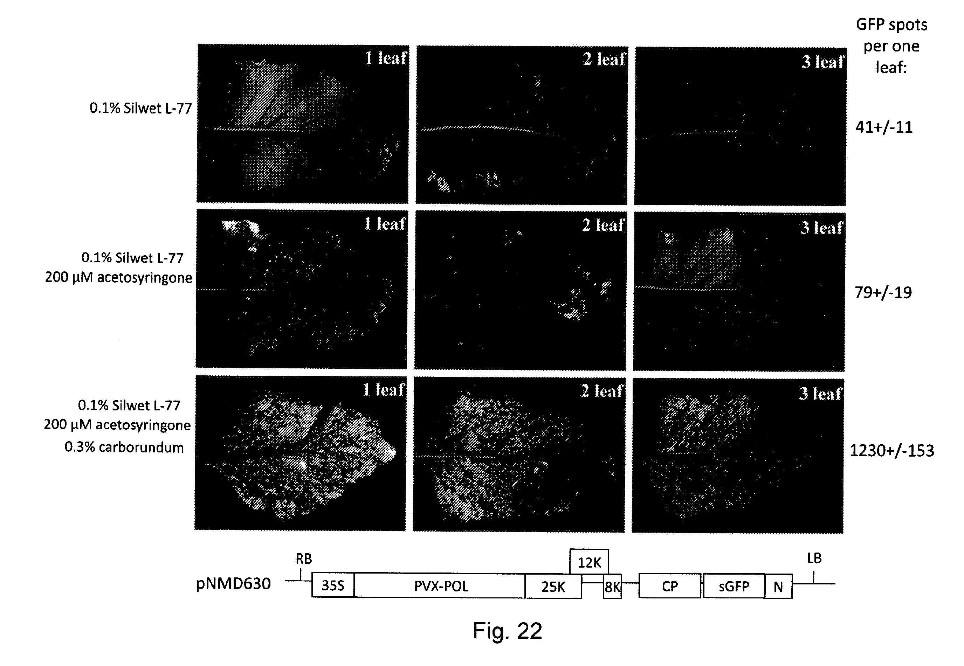

Fig. 22 shows GFP expression after delivery of agrobacteria to *Beta vulgaris vulgaris L.* leaves using spraying with surfactant; influence of acetosyringone and abrasive.
   Vector: PVX(CP)-GFP (pNMD630). Agrobacterial cells were incubated with 200 µM acetosyringone for 2 hours before spraying. For abrasive treatment, 0.3% carborundum (silicon carbide mixture of F800, F1000 and F1200 particles, Mineraliengrosshandel Hausen GmbH, Telfs, Austria) was added to agrobacterial suspension. Dilution factor of agrobacteria of OD600=1.4: 10⁻². GFP-expressing spots were leaves are indicated on the right.
Fig. 23 shows GFP expression after delivery of diluted agrobacteria to plants of different species by spraying with surfactant and abrasive. Vectors: TMV(MP)-GFP (pNM600) and PVX(CP)-GFP (pNMD630), dilution factor of agrobacterial culture (OD600=1.5): 10⁻², 0.1 % Silwet L-77, 0.3 % silicon carbide.
Fig. 24 shows expression after triple subsequent treatments of *Nicotiana benthamina* plants with agrobacteria harbouring viral vectors. Dipping of leaves was performed with 7-days interval in the order:
   A) PVX(ΔCP)-GFP, PVX (CP)-dsRED, TMV(MP)-GFP;
   B) TMV(fsMP)-GFP, PVX(CP)-dsRED, PVX(CP)-GFP;
   C) PVX(ΔCP)-GFP, TMV(MP)-dsRED, PVX(CP)-GFP;
   D) TMV(fsMP)-GFP, PVX(CP)-dsRED, TMV(MP)-GFP;
   E) TMV(fsMP)-GFP, TMV(MP)-dsRED, TMV(MP)-GFP;
   F) PVX(ΔCP)-GFP, TMV(MP)-dsRED, TMV(MP)-GFP;
   G) TMV(fsMP)-GFP, PVX(CP)-dsRED, TMV(MP)-GFP;
   H) PVX(ΔCP)-GFP, PVX(CP)-dsRED, TMV-GFP.
      Single treatments:
   I) TMV(fsMP)-GFP; J) PVX(ΔCP)-GFP; K) TMV(MP)-GFP; L) PVX(CP)-GFP; M) TMV(MP)-dsRED and N) PVX(CP)-dsRED.

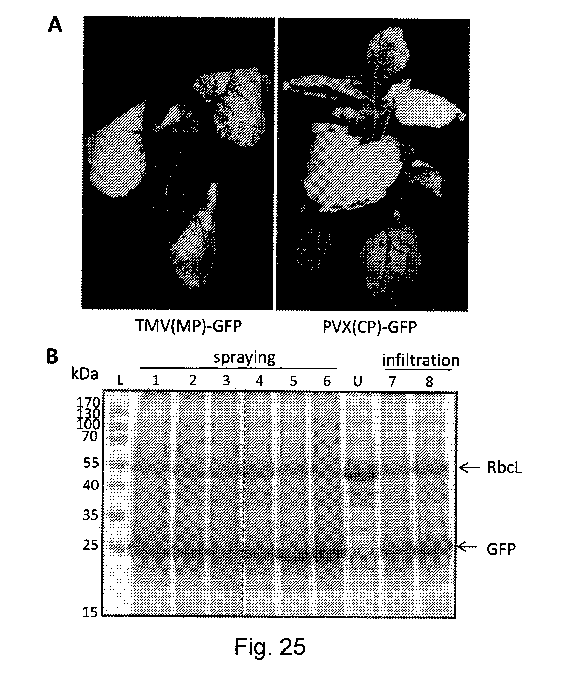

Fig. 25 Analysis of GFP expression in *Nicotiana benthamiana* plants sprayed with agrobacterial suspensions (10⁻² dilution factor) harbouring TMV(MP)-GFP (pNM600) and PVX(CP)-GFP (pNMD630) vectors.
   A) Spraying-transfected *Nicotiana benthamiana* plants, photograph taken 15 dpi.
   B) SDS-PAGE with Coomassie staining; 12% gel, reducing conditions. Leaves from spraying-transfected N.benthamiana plants expressing GFP) were harvested at 15 dpi. Plant material was extracted with 6 volumes of 1 x Laemmli buffer containing beta-mercaptoethanol. After heating at 95 C, 10 µl aliquots were loaded on the gel.
      L - Protein Ladder (Fermentas, #SM0671);
      1 - TMV(MP)-GFP, plant 1;
      2 - TMV(MP)-GFP, plant 2;
      3 - TMV(MP)-GFP, plant3;
      4 - PVX(CP)-GFP, plant 1;
      5 - PVX(CP)-GFP, plant 2;
      6 - PVX(CP)-GFP, plant3;
         U - uninfected *N*. *benthamiana* leaf tissue;
      7 - TMV(MP)-GFP, vacuum-infiltrated plant;
      8 - PVX(CP)-GFP, vacuum-infiltrated plant.
         RbcL-RUBISCO large subunit.

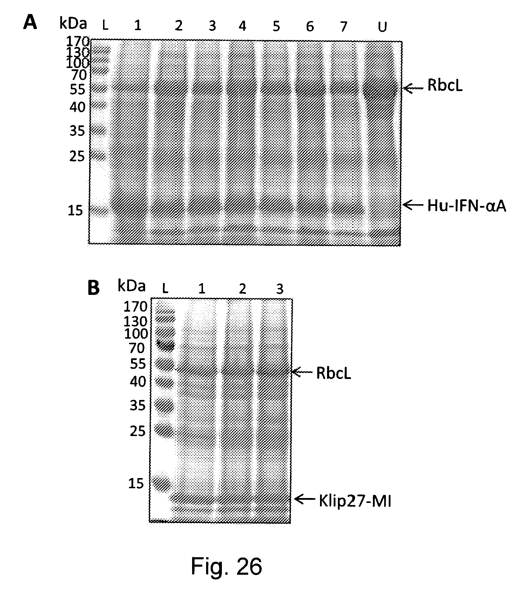

Fig. 26 SDS-PAGE with Coomassie staining for analysis of human alpha-a interferon (Hu-IFN-αA) and Klip27-Mini-Insulin expressed in *Nicotiana benthamiana* plants sprayed with agobacterial suspensions of TMV-based viral vectors capable for cell-to-cell movement.
   A) Hu-IFN-αA: pNMD38 and pNMD45 vectors, 10-2 dilution factor of agrobacterial culture, harvesting at 12 dpi.
      L - Protein Ladder (Fermentas, #SM0671);
      1 - pNMD38, syringe infiltration;
      2 - pNMD38, spraying, leaf1;
      3 - pNMD38, spraying, leaf2;
      4 - pNMD38, spraying, leaf3;
      5 - pNMD45, spraying, leaf1;
      6 - pNMD45, spraying, leaf2;
      7 - pNMD45, spraying, leaf3;
         U - uninfected N.benthamiana leaf tissue.
   B) Klip27-Mini-Insulin: pNMD331 vector, 10⁻² and 10⁻³ dilution factors of agrobacterial culture, harvesting at 12 dpi.
      L- Protein Ladder (Fermentas, #SM0671);
      1 - pNMD331, syringe infiltration, dilution 10⁻³;
      2 - pNMD331, spraying, dilution 10⁻³;
      3 - pNMD331, syringe infiltration, dilution 10⁻³.
   Plant material was extracted with 6 volumes of 1 x Laemmli buffer containing beta-mercaptoethanol. 10 µl aliquots were separated in 15% polyacrylamide gel under reducing conditions.

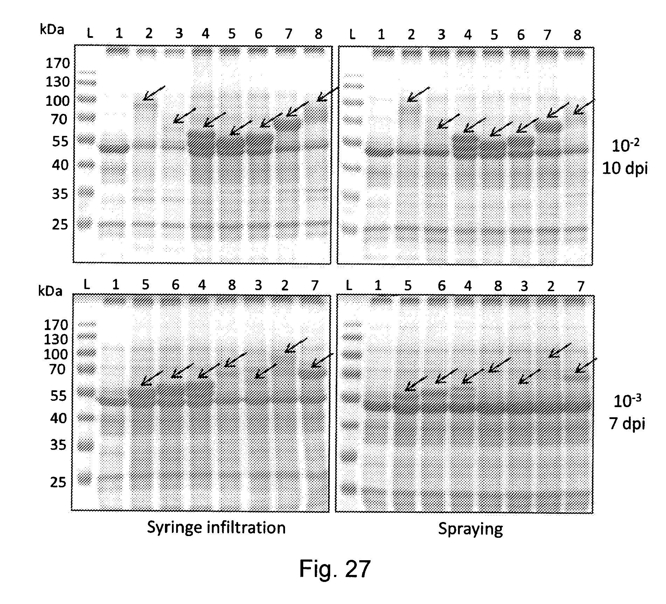

Fig. 27 Expression of cellulases in *N. benthamiana* plants achieved by spraying of diluted agrobacterial cultures harbouring TMV vectors capable for cell-to-cell movement (7 and 10 dpi). *N. benthamiana* plants were inoculated with 10⁻² (top panel) and 10⁻³ (bottom panel) dilutions of *Agrobacterium* cultures (OD600=1.3) either by needleless syringe or spraying with 0.1 % Silwet L-77. Protein levels of cellulase fusions were analyzed in crude extracts using SDS-PAGE with Coomassie staining.
   For crude extracts, 50 mg plant material (pooled samples of 3 independant leaves) harvested 10 dpi was ground in liquid nitrogen, extracted with 5 vol. 2xLaemmli buffer and denatured at 95°c for 5 min. 7.5 µl of each sample were analyzed by 10% SDS-PAGE and Coomassie staining.
   L - Protein Ladder (Fermentas, #SM0671);
   1 - uninfected *N. benthamiana* leaf tissue;
   2 - exocellulase E3 from *Thermobifida fusca* targeted to apoplast;
   3 - exoglucanase 1 (CBH I) from *Trichoderma reesei* targeted to apoplast;
   4 - b-glucosidase BGL4 from *Humicola grisea* targeted to chloroplasts;
   5 - b-glucosidase BGL4 from *Humicola grisea* expressed in cytosol;
   6 - His-tagged b-glucosidase BGL4 from *Humicola grisea;*
   7 - exocellulase E3 from *Thermobifida fusca* targeted to chloroplasts;
   8 - endoglucanase E1 from *Acidothermus cellulolyticus* targeted to apoplast.

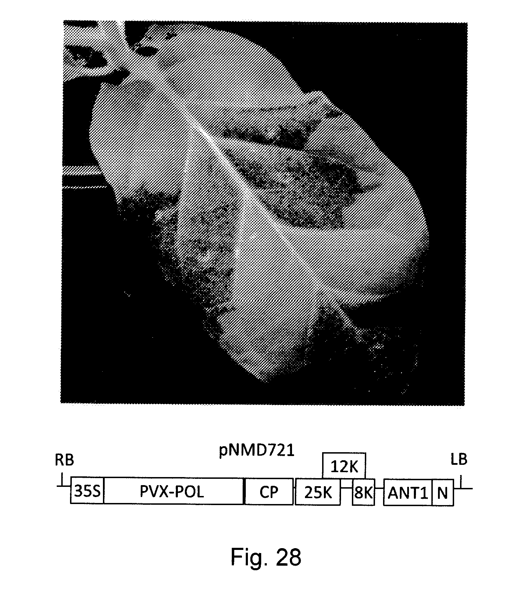

Fig. 28 Induction of anthocyanin biosynthesis in *Nicotiana tabacum* leaves via transient expression by infiltration of MYB transcription factor anthocyanin 1 (ANT1) from *Lycopersicon esculentum* (AAQ55181). 7 dpi, *Agrobacterium* culture (OD600=1.4) dilution factor 10⁻².

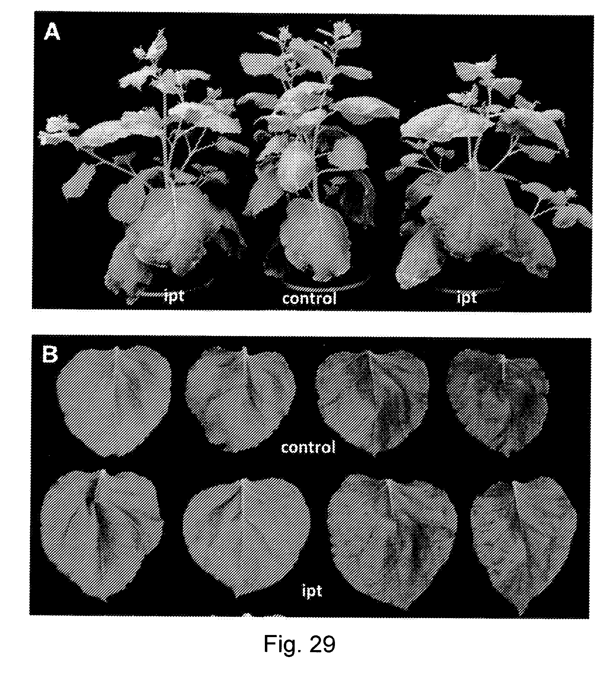

Fig. 29 Morphological changes in *Nicotiana benthamiana* plants caused by transient expression of isopentenyl transferase (ipt) gene delievered by spraying with diluted agrobacteria harbouring transcriptional vector containing ipt coding sequence under the control of 35S promoter. Agrobacterium culture (OD600=1.4) was diluted by a factor of 10⁻² and supplemented with 0.1 % Silwet L-77; photograph taken 45 dpi.
   A) Habitus of transfected plants. ipt - Plants sprayed with diluted agrobacterial culture (pNMD460 construct); control - plant sprayed with buffer for transfection containing no agrobacteria.
   B) Leaves of transfected plants. Top: leaves of plants sprayed with buffer for transfection containing no agrobacteria. Bottom: leaves of plants sprayed with diluted agrobacterial culture (pNMD460 construct).

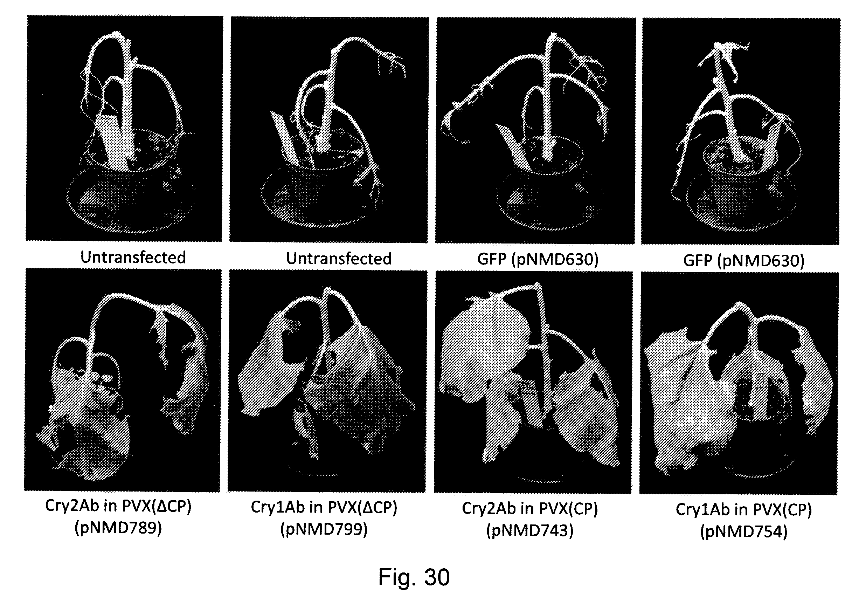

Fig. 30 Transient expression of *Bacillus thuringiensis* endotoxins after the spraying with diluted agrobacterial cultures harbouring corresponding PVX-based expression vectors protects *Nicotiana benthamiana* plants from feeding damage by larvae of the tobacco hornworm *Manduca sexta.* Plants were sprayed with agrobacterium cultures (OD600=1.4-1.7) diluted by factor 10⁻² and supplemented with 0.1 % Silwet L-77. Two weeks later, 3 larvae of the 3rd instar were fed on each plant. Pictures were taken in 2 weeks after the beginning of feeding.

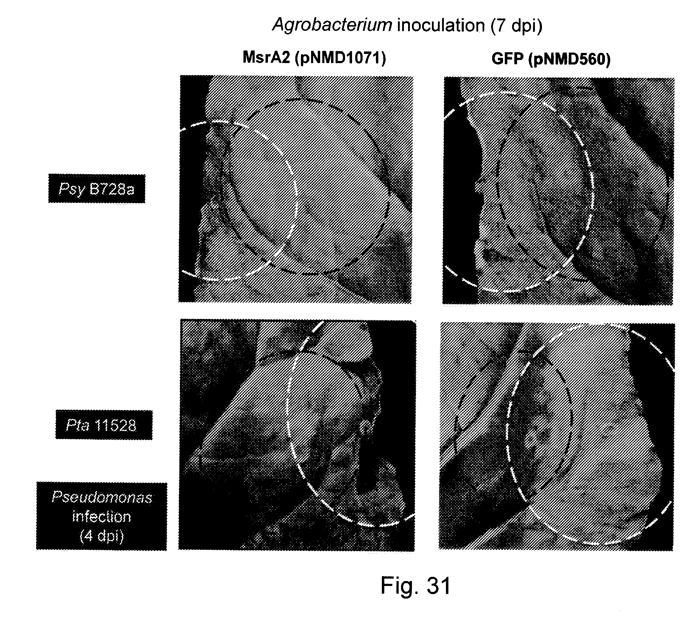

Fig. 31 Phenotypes of *N.benthamiana* transiently expressing defensin MsrA2 and GFP via TMV-based vectors with cell-to-cell movement ability (pNMD1071 and pNMD560, respectively). Inoculation with *Pseudomonas* was performed at 3 days post inoculation with Agrobacterium. Pictures were taken at 4 days post inoculation with *Pseudomonas.* Inoculation of leaves with both *Agrobacterium* and *Pseudomonas* was
   performed using needleless syringe.

### DETAILED DESCRIPTION OF THE INVENTION

In the invention, agrobacteria are used for transfecting plants by spraying with aqueous suspensions containing cells of an *Agrobacterium* strain. The *Agrobacterium* strain may belong to the species *Agrobacterium tumefaciens* or *Agrobacterium rhizogenes* that are commonly used for plant transformation and transfection and which is known to the skilled person from general knowledge. The *Agrobacterium* strain comprises a DNA molecule comprising a nucleic constructs containing a DNA sequence of interest. The DNA sequence of interest encodes a protein or an RNA to be expressed in plants. The nucleic construct is typically present in T-DNA of Ti-plasmids for introduction of the nucleic construct into plant cells by the secretory system of the *Agrobacterium* strain. On at least one side or on both sides, the nucleic acid construct is flanked by a T-DNA border sequence for allowing transfection of said plant(s) and introduction of cells of said plant with said DNA sequence of interest. In the nucleic acid construct, the DNA sequence of interest is present such as to be expressible in plant cells. For this purpose, the DNA sequence of interest is, in said nucleic acid construct, typically under the control of a promoter active in plant cells. Examples of the DNA sequence of interest are a DNA sequence encoding a DNA viral replicon or an RNA viral replicon or a gene to be expressed. The gene may encode an RNA of interest or a protein of interest to be expressed in cells of the plant(s). Also the viral replicons typically encode an RNA or a protein of interest to be expressed in plants. The DNA construct may comprise, in addition to the DNA sequence of interest, other sequences such as regulatory sequences for expression of the DNA sequence of interest. Agrobacterium-mediated gene transfer and vectors therefor are known to the skilled person, e.g. from the references cited in the introduction or from text books on plant biotechnology such as Slater, Scott and Fowler, Plant Biotechnology, second edition, Oxford University Press, 2008.

In embodiments wherein strong expression of a protein or RNA is desired or wherein accumulation of viral nucleic acids to high amounts in plant cells and possible negative effects on plant health is not a concern, the nucleic acid construct may encode a replicating viral vector that can replicate in plant cells. In order to be replicating, the viral vector contains an origin of replication that can be recognized by a nucleic acid polymerase present in plant cells, such as by the viral polymerase expressed from the replicon. In case of RNA viral vectors, the viral replicons may be formed by transcription, under the control of a plant promoter, from the DNA construct after the latter has been introduced into plant cell nucleic. In case of DNA viral replicons, the viral replicons may be formed by recombination between two recombination sites flanking the sequence encoding the viral relicon in the DNA construct, e.g. as described in WO00/17365 and WO 99/22003. If viral replicons are encoded by the DNA construct, RNA viral replicons are preferred. Use of DNA and RNA viral replicons has been extensively described in the literature at least over the last 15 years. Some examples are the following patent publications by Icon Genetics: W02008028661, W02007137788, WO 2006003018, W02005071090, WO2005049839, WO02097080, WO02088369, WO02068664. An example of DNA viral vectors are those based on geminiviruses. For the present invention, viral vectors or replicons based on plant RNA viruses, notably based on plus-sense single-stranded RNA viruses are preferred. Examples of such viral vectors are tobacco mosaic virus (TMV) and potex virus X (PVX) used in the examples. Potexvirus-based viral vectors and expression systems are described in EP2061890. Many other plant viral replicons are described in the patent publications mentioned above.

The aqueous suspension used for spraying in the processes of the invention may have a concentration of *Agrobacterium* cells of at most 1.1·10⁹ cfu/ml, which corresponds approximately to an *Agrobacterium* culture in LB-medium of an optical density at 600 nm of 1. Due to the high transfection efficiency achieved in the invention, much lower concentrations may, however, be used, which allows treatment of many plants such as entire farm fields without the need for huge fermenters for *Agrobacterium* production. Thus, the concentration is preferably at most 2.2·10⁷ cfu/ml, more preferably at most 1.1·10⁷ cfu/ml, more preferably at most 4.4·10⁶ cfu/ml. In one embodiment, the concentration is at most 1.1·10⁶ cfu/ml of the suspension. For avoiding determination of cell concentrations in terms of cfu/ml, concentrations of agrobacterial suspensions are frequently assessed by measuring the apparent optical density at 600 nm using a spectrophotometer. Herein, the concentration of 1.1·10⁷ cfu/ml corresponds to a calculated optical density at 600 nm of 0.01, whereby the calculated optical density is defined by a 100-fold dilution with water or buffer of a suspension having an optical density of 1.0 at 600 nm. Similarly, the concentrations of 4.4·10⁶ cfu/ml and 1.1·10⁶ cfu/ml correspond to a calculated optical density at 600 nm of 0.004 and 0.001, respectively, whereby the calculated optical densities are defined by a 250-fold or 1000-fold, respectively, dilution with water or buffer of a suspension having an optical density of 1.0 at 600 nm.

The abrasive used in the invention is a particulate material that is essentially insoluble in the aqueous suspension of *Agrobacterium* cells. The abrasive is believed to weaken, notably if used together with a wetting agent, the surface of plant tissue such as leaves, and thereby facilitates penetration of *Agrobacterium* cells into the intercellular space of plant tissue. As a result, the transfection efficiency increases.

The particulate material to be used as the abrasive of the invention may be carrier material as commonly used as carriers in wettable powder (WP) of pesticide formulations. In the context of wettable powders, these carriers are also referred to in the field of pesticide formulations as "fillers" or "inert fillers". Wettable powder formulations are part of the general knowledge in the field of plant protection. Reference is made to the handbook PESTICIDE SPECIFICATIONS, "Manual for Development and Use of FAO and WHO Specifications for Pesticides", edited by the World Health Organisation (WHO) and the FOOD and Agriculture Organization of the United States, Rome, 2002, ISBN 92-5-104857-6. Wettable powder formulations for plant protection are for example described in EP 1810569, EP1488697, EP1908348 and EP0789510. The abrasive may be a mineral material, typically an inorganic material. Examples of such carrier materials are diatomaceous earth, talc, clay, calcium carbonate, bentonite, acid clay, attapulgite, zeolite, sericite, sepiolite or calcium silicate. It is also possible to use quartz powder such as the highly pure quartz powder described in WO02/087324. Preferred examples are silica, such as precipitated and fumed hydrophilic silica, and carborundum. The abrasive properties of diluents or fillers such as silica used in wettable powders are known (see "Pesticide Application Methods" by G.A. Matthews, third edition, Blackwell Science, 2000, on page 52 thereof).

As commercial products of particulate inorganic materials for use as abrasives in the invention, the hydrophilic silica Sipernat™ 22S and Sipernat™ 50 S, manufactured by Evonic Degussa may be mentioned. Other products are "Hi-Sil™ 257", a synthetic, amorphous, hydrated silica produced by PPG Industries Taiwan Ltd. or "Hubersorb 600™", a synthetic calcium silicate, manufactured by Huber Corporation. A commercial sub-micron sized silica is Hi-Sil™ 233 (PPG Industries) having an average particle size of around 0.02 µm.

The abrasive may have a median particle size between 0.01 and 40, preferably between 0.015 and 30, more preferably between 0.05 and 30, even more preferably between 0.1 and 30, even more preferably between 0.1 and 20, even more preferably between 0.5 and 20, and most preferably between 1.0 and 16 µm. In one embodiment, the median particle size is between 0.015 and 1 or between 0.02 and 0.5 µm. The median particle size is the volume median particle size that can be measured by laser diffraction using a Mastersizer™ from Malvern Instruments, Ltd. In order to avoid clogging of spraying nozzles, the maximum particle size of the largest particles contained in the abrasive should be at most 45 µm, preferably at most 40 µm, which may be determined by sieving. This condition is considered fulfilled, if the sieve residue is below 1.5 % by weight (following ISO 3262-19). The abrasive may have a D90 value of at most 40 µm, preferably of at most 30 µm, measured by laser diffraction as described above. Typically, the particle sizes above relate to primary particle sizes.

The content of the abrasive in the aqueous suspension of the invention may be between 0.01 and 3, preferably between 0.02 and 2, more preferably between 0.05 and 1 and even more preferably between 0.1 and 0.5 % by weight of said suspension.

The aqueous suspension of the invention preferably contains an agricultural spray adjuvant. The spray adjuvant may be a surfactant or wetting agent. The surfactant and wetting agent has multiple advantages in the present invention. It reduces the surface tension of the water of the aqueous suspension and makes the waxy surface of plant leaves more permeable for agrobacteria. It further improves the stability of the suspension and reduces settling of the abrasive in the suspension. Surfactants used in the present invention are not particularly limited, and examples of the surfactants include the following (A), (B), and (C). These may be used singly or in combination.
(A) Nonionic surfactants: A measurement frequently used to describe surfactants is the HLB (hydrophilic/lipophilic balance). The HLB describes the ability of the surfactant to associate with hydrophilic and lipophilic compounds. Surfactants with a high HLB balance associate better with water soluble compounds than with oil soluble compounds. Herein, the HLB value should be 12 or greater, preferably at least 13. As noninionic surfactants, organo-silicone surfactants such as polyalkyleneoxide-modified heptamethyltrisiloxane are most preferred in the present invention. A commercial product is Silwet L77™ spray adjuvant from GE Advanced Materials.
   (A-1) Polyethylene glycol type surfactants: examples of polyethylene glycol type surfactants include polyoxyethylene alkyl (C12-18) ether, ethylene oxide adduct of alkylnaphthol, polyoxyethylene (mono or di) alkyl (C8-12) phenyl ether, formaldehyde condensation product of polyoxyethylene (mono or di) alkyl (C8-12) phenyl ether, polyoxyethylene (mono, di, or tri) phenyl phenyl ether, polyoxyethylene (mono, di, or tri) benzyl phenyl ether, polyoxypropylene (mono, di, or tri) benzyl phenyl ether, polyoxyethylene (mono, di, or tri) styryl phenyl ether, polyoxypropylene (mono, di or tri) styryl phenyl ether, a polymer of polyoxyethylene (mono, di, or tri) styryl phenyl ether, a polyoxyethylene polyoxypropylene block polymer, an alkyl (C12-18) polyoxyethylene polyoxypropylene block polymer ether, an alkyl (C8-12) phenyl polyoxyethylene polyoxypropylene block polymer ether, polyoxyethylene bisphenyl ether, polyoxyethylene resin acid ester, polyoxyethylene fatty acid (C12-18) monoester, polyoxyethylene fatty acid (C12-18) diester, polyoxyethylene sorbitan fatty acid (C12-18) ester, ethylene oxide adduct of glycerol fatty acid ester, ethylene oxide adduct of castor oil, ethylene oxide adduct of hardened caster oil, ethylene oxide adduct of alkyl (C12-8) amine and ethylene oxide adduct of fatty acid (C12-18) amide;
   (A-2) Polyvalent alcohol type surfactants: examples of polyvalent alcohol type surfactants include glycerol fatty acid ester, polyglycerin fatty acid ester, pentaerythritol fatty acid ester, sorbitol fatty acid (C12-18) ester, sorbitan fatty acid (C12-8) ester, sucrose fatty acid ester, polyvalent alcohol alkyl ether, and fatty acid alkanol amide;
   (A-3) Acetylene-type surfactants: examples of acetylene type surfactants include acetylene glycol, acetylene alcohol, ethylene oxide adduct of acetylene glycol and ethylene oxide adduct of acetylene alcohol.
(B) Anionic surfactants:
   (B-1) Carboxylic acid type surfactants: examples of carboxylic acid type surfactants include polyacrylic acid, polymethacrylic acid, polymaleic acid, a copolymer of maleic acid and olefin (for example, isobutylene and diisobutylene), a copolymer of acrylic acid and itaconic acid, a copolymer of methacrylic acid and itaconic acid, a copolymer of maleic acid and styrene, a copolymer of acrylic acid and methacrylic acid, a copolymer of acrylic acid and methyl acrylate, a copolymer of acrylic acid and vinyl acetate, a copolymer of acrylic acid and maleic acid, N-methyl-fatty acid (C12-18) sarcosinate, carboxylic acids such as resin acid and fatty acid (C12-18) and the like, and salts of these carboxylic acids.
   (B-2) Sulfate ester type surfactants: examples sulfate ester type surfactants include alkyl (C12-18) sulfate ester, polyoxyethylene alkyl (C12-18) ether sulfate ester, polyoxyethylene (mono or di) alkyl (C8-12) phenyl ether sulfate ester, sulfate ester of a polyoxyethylene (mono or di) alkyl (C8-12) phenyl ether polymer, polyoxyethylene (mono, di, or tri) phenyl phenyl ether sulfate ester, polyoxyethylene (mono, di, or tri) benzyl phenyl ether sulfate ester, polyoxyethylene (mono, di, or tri) styryl phenyl ether sulfate ester, sulfate ester of a polyoxyethylene (mono, di, or tri) styryl phenyl ether polymer, sulfate ester of a polyoxyethylene polyoxypropylene block polymer, sulfated oil, sulfated fatty acid ester, sulfated fatty acid, sulfate ester of sulfated olefin and the like, and salts of these sulfate esters.
   (B-3) Sulfonic acid type surfactants: examples of sulfonic acid type surfactants include paraffin (C12-22) sulfonic acid, alkyl (C8-12) benzene sulfonic acid, formaldehyde condensation product of alkyl (C8 - 12) benzene sulfonic acid, formaldehyde condensation product of cresol sulfonic acid, -olefin (C14-16) sulfonic acid, dialkyl (C8-12) sulfosuccinic acid, lignin sulfonic acid, polyoxyethylene (mono or di) alkyl (C8-12) phenyl ether sulfonic acid, polyoxyethylene alkyl (C12-18) ether sulfosuccinate half ester, naphthalene sulfonic acid, (mono, or di) alkyl (C1-6) naphthalene sulfonic acid, formaldehyde condensation product of naphthalene sulfonic acid, formaldehyde condensation product of (mono, or di) alkyl (C1-6) naphthalene sulfonic acid, formaldehyde condensation product of creosote oil sulfonic acid, alkyl (C8-12) diphenyl ether disulfonic acid, Igepon T (tradename), polystyrene sulfonic acid, sulfonic acids of a styrene sulfonic acid - methacrylic acid copolymer and the like, and salts of these sulfonic acids.
   (B-4) Phosphate ester type surfactants: examples of phosphate ester type surfactants include alkyl (C8-12) phosphate ester, polyoxyethylene alkyl (C12-18) ether phosphate ester, polyoxyethylene (mono or di) alkyl (C8-12) phenyl ether phosphate ester, phosphate ester of a polyoxyethylene (mono, di, or tri) alkyl (C8-12) phenyl ether polymer, polyoxyethylene (mono, di, or tri) phenyl phenyl ether phosphate ester, polyoxyethylene (mono, di, or tri) benzyl phenyl ether phosphate ester, polyoxyethylene (mono, di, or tri) styryl phenyl ether phosphate ester, phosphate ester of a polyoxyethylene (mono, di, or tri) styryl phenyl ether polymer, phosphate ester of a polyoxyethylene polyoxypropylene block polymer, phosphatidyl choline, phosphate ester of phosphatidyl ethanolimine and condensed phosphoric acid (for example, such as tripolyphosphoric acid) and the like, and salts of these phosphate esters.
      Salts of above-mentioned (B-1) to (B-4) include alkaline metals (such as lithium, sodium and potassium), alkaline earth metals (such as calcium and magnesium), ammonium and various types of amines (such as alkyl amines, cycloalkyl amines and alkanol amines).
(C) Amphoteric surfactants: Examples of amphoteric surfactants include betaine type surfactants and amino acid type surfactants.

The above surfactants may be used singly or in combination of two or more surfactants. Notably, the preferred organo-silicone surfactants may be combined with other surfactants. The total concentration of surfactants in the aqueous suspension of the invention may be easily tested by conducting comparative spraying experiments, similarly as done in the examples. However, in general, the total concentration of surfactants may be between 0.005 and 2 volume-%, preferably between 0.01 and 0.5 volume-%, more preferably between 0.025 and 0.2 volume-% of said suspension. Since the density of surfactants is generally close to 1.0 g/ml, the total concentration of surfactants may be defined as being between 0.05 and 20 g per liter of said suspension, preferably between 0.1 and 5.0 g, more preferably between 0.25 and 2.0 g per liter of said suspension (including abrasive). If the above organo-silicone surfactants such as polyalkyleneoxide-modified heptamethyltrisiloxane are used, the concentration of the organo-silicone surfactant in the agrobacterial suspension used for spraying may be between 0.01 and 0.5 volume-%, preferably between 0.05 and 0.2 volume-%. Alternatively, the concentration of the organo-silicone surfactant in the agrobacterial suspension used for spraying may be defined as being between 0.1 and 5.0 g, preferably between 0.5 and 2.0 g per liter of said suspension.

In order to improve the physical properties of the aqueous suspension, it is possible to add highly dispersed sub-micron size silicic acid (silica) or porous polymers such as urea/formaldehyde condensate (Pergopak™). Notably, where the median particle size of the abrasive is between 0.1 and 30 µm, or in one of the preferred sub-ranges of this range given above, it is possible to add a highly dispersed sub-micron size silica to the suspension. Herein, sub-micron size silica is silica having a median particle size between 0.01 and 0.5 µm, preferably between 0.02 and 0.5 µm, more preferably between 0.02 and 0.1 µm. Highly dispersed silicic acid such as Hi-Sil™ 233 (PPG Industries) can contribute to the abrasive properties of the aqueous suspension (see Jensen et al., Bull. Org. mond. Sante, Bull. Wld Hlth Org. 41 (1969) 937-940). These agents may be incorporated in an amount of from 1 to 10 g per liter of the suspension of the invention.

Further possible additives to the agrobacterial suspension are buffer substances to keep maintain the pH of the suspension used for spraying at a desired pH, typically between 7.0 and 7.5. Further, inorganic soluble salts such as sodium chloride by be added to adjust the ionic strength of the suspension. Nutrient broth such as LB medium may also be contained in the suspension.

The aqueous suspension may be produced as follows. In one method, the *Agrobacterium* stain to be used in the process of the invention is inoculated into culture medium and grown to a high cell concentration. Larger cultures may be inoculated with small volumes of a highly concentrated culture medium for obtaining large amounts of the culture medium. Agrobacteria are generally grown up to a cell concentration corresponding to an OD at 600 nm of at least 1, typically of about 1.5. Such highly concentrated agrobacterial suspensions are then diluted to achieve the desired cell concentration. For diluting the highly concentrated agrobacterial suspensions, water is used. The water may contain a buffer. The water may further contain the surfactant of the invention. Alternatively, the concentrated agrobacterial suspensions may be diluted with water, and any additives such as the surfactant and the optional buffer substances are added after or during the dilution process. The abrasive may be added before, during or after dilution. It is however preferred to agitate the suspension during addition of the abrasive to uniformly disperse the abrasive in the agrobacterial suspension. The step of diluting the concentrated agrobacterial suspension may be carried out in the spray tank of the sprayer used for spraying the diluted suspensions.

The sprayer to be used in the process of the invention mainly depends on the number of plants or the area to be sprayed. For one or a small number of plants to be sprayed, pump sprayers as widely used in household and gardening can be used. These may have volumes of the spray tank of between 0.5 and 2 liters. For applications on a medium scale, manually operated hydraulic sprayers such as lever-operated knapsack sprayers or manually operated compression sprayers may be used. However, the high transfection efficiency achieved in the invention has its full potential in the transfection of many plants such as plants growing on a farm field or in a greenhouse. For this purpose, power-operated hydraulic sprayers such as tractor-mounted hydraulic sprayers equipped with spray booms can be used. Aerial application techniques using helicopters or airplanes are also possible for large fields. All these types of sprayers are known in the art and are described for example in the book "Pesticide Application Methods" by G.A. Matthews, third edition, Blackwell Science, 2000. In order to ensure a homogeneous suspension in the spray tanks of the sprayers, small or medium size sprayers may be shaken at regular intervals or continuously during spraying. Large sprayers such as the tractor-mounted sprayers should be equipped with an agitator in the spray tank.

Considering the presence of agrobacterial cells and abrasive in the suspensions to be sprayed, sprayers used in the invention should produce spray of a droplet size at least of fine spray. Also, medium spray or coarse spray in the classification of sprays used in the above-mentioned book by G.A. Matthews, page 74, may be used. The main purpose of the spraying in the invention is wetting of plant tissue with the suspension. Thus, the exact droplet size is not critical. However, the transfection efficiency may be further improved by providing the spray to plant surfaces with increased pressure.

In the process of the invention, at least parts of plants are sprayed. In an important embodiment, plants growing in soil on a field are sprayed, i.e. plants not growing in movable pots or containers. Such plants cannot be turned upside down and dipped into agrobacterial suspension for vacuum infiltration. At least parts of plants are sprayed such as leaves. Preferably, most leaves are sprayed or entire plants.

The present invention is mainly used for transient transfection of plants with a DNA sequence of interest. The term "transient" means that the no selection methods are used for selecting cells or plants transfected with the DNA sequence of interest in the background of non-transfected cells or plants using, e.g. selectable agents and selectable marker genes capable of detoxifying the selectable agents. As a result, the transfected DNA is generally not stably introduced into plant chromosomal DNA. Instead, transient methods make use of the effect of transfection in the very plants transfected.

The invention is generally used for transfecting multi-cellular plants, notably, higher plants. Both monocot and dicot plants can be transfected, whereby dicot plants are preferred. Plants for the use in this invention include any plant species with preference given to agronomically and horticulturally important crop species. Common crop plants for the use in present invention include alfalfa, barley, beans, canola, cowpeas, cotton, corn, clover, lotus, lentils, lupine, millet, oats, peas, peanuts, rice, rye, sweet clover, sunflower, sweetpea, soybean, sorghum triticale, yam beans, velvet beans, vetch, wheat, wisteria, and nut plants. The plant species preferred for practicing this invention include, but not restricted to, representatives of Gramineae, Compositeae, Solanaceae and Rosaceae.

Further preferred species for the use in this invention are plants from the following genera: Arabidopsis, Agrostis, Allium, Antirrhinum, Apium, Arachis, Asparagus, Atropa, Avena, Bambusa, Brassica, Bromus, Browaalia, Camellia, Cannabis, Capsicum, Cicer, Chenopodium, Chichorium, Citrus, Coffea, Coix, Cucumis, Curcubita, Cynodon, Dactylis, Datura, Daucus, Digitalis, Dioscorea, Elaeis, Eleusine, Festuca, Fragaria, Geranium, Glycine, Helianthus, Heterocallis, Hevea, Hordeum, Hyoscyamus, Ipomoea, Lactuca, Lens, Lilium, Linum, Lolium, Lotus, Lycopersicon, Majorana, Malus, Mangifera, Manihot, Medicago, Nemesia, Nicotiana, Onobrychis, Oryza, Panicum, Pelargonium, Pennisetum, Petunia, Pisum, Phaseolus, Phleum, Poa, Prunus, Ranunculus, Raphanus, Ribes, Ricinus, Rubus, Saccharum, Salpiglossis, Secale, Senecio, Setaria, Sinapis, Solanum, Sorghum, Stenotaphrum, Theobroma, Trifolium, Trigonella, Triticum, Vicia, Vigna, Vitis, Zea, and the Olyreae, the Pharoideae and others.

In one embodiment, the process of the invention can be used for producing a protein of interest in a plant or in many plants growing on a field. For this purpose, the plants may be sprayed with the agrobacterial suspension at a desired growth state of the plants. If the main aim is to achieve the highest possible expression levels followed by harvesting plants for obtaining plant material containing high amounts of the protein, viral vectors may be used, since they generally give the highest expression levels.

In another embodiment, the process of the invention is used for generating or altering a trait in a plant such as an input trait. In this embodiment, excessive expression of a protein or RNA of interest may not be desired for avoiding deleterious effects on plant health. For such embodiments, non-replicating vectors (also referred to herein as "transcriptional vectors"), i.e. vectors lacking a functional origin of replication recognised by a nucleic acid polymerase present in the plant cells are preferred. An example of such embodiment is the expression of hormonal molecules as secondary messengers in plant cells. In the example of Fig. 29, we demonstrate the delivery of isopenthenyl transferase, key enzyme of cytokinin biosynthesis, into *Nicotiana benthamiana* cells by spraying with diluted agrobacteria carrying a transcriptional vector containing the ipt coding sequence under the control of a 35S promoter. Morphological changes of transfected plants caused by cytokinin overproduction were observed (Fig. 29). Another application of the invention is RNA expression, e.g. for RNA interference, wherein the interference signal can spread in the plant from cells having expressed the signal to other cells. As example is the targeting of undesired viral DNA in plants as described by Pooggin in Nat. Biotech. 21 (2003) 131. An example of oncogene silencing that can be adapted to a transient system is described by Escobar et al. Proc. Natl. Acad. Sci. USA 98 (2001) 13437-13442. A further example is the control of coleopteran insect pests through RNA interference similar as described by Baum et al., Nat. Biotech. 25 (2007) 1322-1326 that can be adapted to the transient process of the invention by transiently transfecting pest-infested plants with DNA of interest encoding and expressing the dsRNA. Further methods applicable to the transient process of the invention are those described by Huang et al., Proc. Natl. Acad. Sci. USA 103 (2006) 14302-14306; Chuang et al., Proc. Natl. Acad. Sci. USA 97 (2000) 4985-4990.

Further, the process of the invention allows altering at a desired point in time traits relating to the regulation of flowering time or fruit formation such as tuborisation in potato (Martinez-Garcia et al., Proc. Natl. Acad. Sci. USA 99 (2002) 15211-15216) or the regulation of the flavonoid pathway using a transcription factor (Deluc et al., Plant Physiol. 147 (2008) 2041-2053). Flowering may be induced by transiently expressing the movable florigen protein FT (Zeevaart, Current Opinion in Plant Biology 11 (2008) 541-547; Corbesier et al., Science 316 (2007) 1030-1033). Parthenocarpic fruits in tomatoes may by produced on a large scale using the invention and the method described by Pandolfini et al., BMC Biotechnology 2 (2002). Further applications of the invention are in the context of altering cotton fiber development by way of MYB transcription factors as described by Lee et al., Annals of Botany 100 (2007) 1391-1401 or activation of plant defensive genes (Bergey et al., Proc. Natl. Acad. Sci. USA 93 (1996) 12053-12058. We have demonstrated that transient expression of defensin MsrA2 in *Nicotiana benthamiana* leaves significantly decreases the Pseudomonas infection symptoms (Fig. 31).

The invention also provides a process of protecting crop plants on a field from a pest. In such process, infestation of at least one of the plants from a plurality of plants growing in a lot or farm field may be determined. Due to the rapidness of the process of the invention expression of a protein or RNA detrimental to the pest needs to be caused only if infestation by the pest is determined. Thus, strong and constitutive expression of pest toxins or dsRNA for RNAi even in the absence of a risk of infestation is not necessary. Transient expression of *Bacillus thuringiensis* endotoxins after the spraying with diluted agrobacterial cultures harbouring corresponding PVX-based expression vectors protected *Nicotiana benthamiana* plants from feeding damage by larvae of the tobacco hornworm Manduca sexta (Fig. 30).

### EXAMPLES

The invention is further described in the following by way of examples. The invention is however not limited to these examples.

### Reference Example 1: Determination of Agrobacterium cell concentration in liquid culture in terms of colony forming units (cfu)

The concentration of *Agrobacterium* cells in liquid suspension in terms of colony forming units per ml (cfu/ml) of liquid suspensions can be determined using the following protocol. Cells of *Agrobacterium tumefaciens* strain ICF 320 transformed with construct pNMD620 were grown in 7.5 ml of liquid LBS medium containing 25 mg/L kanamycin (AppliChem, A1493) and 50 mg/L rifampicin (Carl Roth, 4163.2). The bacterial culture was incubated at 28°C with continuous shaking. Absorbance or optical density of bacterial culture expressed in absorbance units (AU) was monitored in 1-ml aliquots of the culture using a spectrophotometer at 600 nm wavelength (OD600). The cell concentration estimated as a number of colony-forming units per milliliter of liquid culture (cfu/ml) can be analyzed at OD600 values 1; 1.3; 1.5; 1.7 and 1.8. For this purpose 250-µl aliquots of liquid culture were diluted with LBS-medium to achieve a final volume of 25 ml (dilution 1:100). 2.5 ml of such 1:100 dilution were mixed with 22.5 ml of LBS to achieve the dilution 1:1000. Liquid culture dilutions 1:100; 1:1,000; 1:10,000; 1:100,000; 1:1,000,000; 1:10,000,000 and 1:100,000,000 were prepared similarly. Aliquots of last three dilutions were spread on agar-solidified LBS medium supplemented with 25 mg/L kanamycin and 50 mg/L rifampicin (250 µl of bacterial culture per plate of 90 mm diameter). Plating of aliquots for each dilution was performed in triplicate. After 2 days incubation at 28°C, bacterial colonies were counted for each plate. Plating of 1: 1,000,000 and 1:10,000,000 dilutions resulted in few hundred and few dozen colonies per plate, respectively. So far as dilution 1:100,000,000 provided just few colonies per plate, this dilution was not used for calculation of cell concentration. The cell concentration was estimated according to the formula: cfu/ml = 4 x number of colonies per plate x dilution factor.

For transforming cell concentrations as measured by absorbance measurements at 600 nm (in LB medium) and in terms of cell-forming units, the following relation is used herein: an OD600 of 1.0 corresponds to 1.1 x10⁹ cfu/ml.

### LBS medium (liquid)

1% soya peptone (papaic hydrolysate of soybean meal; Duchefa, S1330)
0.5% yeast extract (Duchefa, Y1333)
1 % sodium chloride (Carl Roth, 9265.2)
dissolved in water, and the is adjusted to pH 7.5 with 1 M NaOH (Carl Roth, 6771.2)
To prepare the solid LBS medium, liquid LBS medium was supplemented with 1.5% agar (Carl Roth, 2266.2). Media were autoclaved at 121 °C for 20 min.

### Example 1: Vectors used in the following examples

In this study we used standard transcriptional vectors based on 35S CaMV promoter as well as TMV- and PVX-based viral replicons with or without cell-to-cell movement ability.

All transcriptional vectors were created on the basis of plCBV10, a pBIN19-derived binary vector (Marillonnet et al., 2004, 2006). They contained two expression cassettes inserted within right and left borders of same T-DNA region (Fig. 1). For cloning of pNMD293 expression vector, two intermediate constructs (pNMD280 and pNMD033) were created. pNMD280 contained the expression cassette comprising, in sequential order, the Cauliflower mosaic virus (CAMV) 35S promoter, omega translational enhancer from Tobacco Mosaic Virus, coding sequence of P19 suppressor of silencing from Tomato Bushy Stunt Virus (TBSV) (GenBank accession no. CAB56483.1) and terminator from octopine synthase gene of *Agrobacterium tumefaciens* inserted between T-DNA right and left borders. To enable the next cloning step, two restriction sites, EcoRI and SphI, were introduced between the T-DNA right border and the 35S promoter sequence. pNMD033 construct contained between left and right T-DNA borders the expression cassette flanked with EcoRI and SpHI restriction sites and comprized of 35S promoter, omega translational enhancer, coding sequence of jellyfish green fluorescent protein and terminator from octopin synthase gene of *Agrobacterium tumefaciens,* listed in sequential order. For cloning of pNMD293 construct, GFP expression cassette was excised from pNMD033 construct using EcoRI and SphI restriction enzymes and transferred into pNMD280 vector linearized with same restrictases. Resulting pNMD293 construct contained two expression cassettes inserted between T-DNA right and left borders. An expression cassette adjacent to the right border comprised CAMV 35S promoter, omega translational enhancer, coding sequences of green fluorescent protein and the nos terminator (listed in sequential order). Expression cassette adjacent to the left border contained 35S promoter followed by omega translational enhancer, coding sequence of P19 suppressor of silencing and ocs terminator. All other construct were created on the basis pNMD293 vector, by replacing the GFP coding sequence with PCR-amplified coding sequences of another genes of interest using for cloning Ncol and BamHI restriction sites. Genes of interest introduced in transcriptional vector constructs encoded sGFP, modified green fluorescent protein (GFP) from jelly fish *Aequorea victoria* (GeneBank accession no. EF030489) (pNMD293); DsRED, red fluorescent protein from a *Discosoma* sp. reef coral (GeneBank accession no. AF168419.2) (pNMD1380); SP3D flowering factor from tomato (GeneBank accession no. AY186735) (pNMD421); Flowering Locus T (FT) from Arabidopsis (GeneBank accession no. BAA77839) (pNMD655); brassinosteroid regulator DWARF4 from Arabidopsis (NM_114926) (pNMD440); isopentenyl transferase (IPT), key enzyme of cytokinin biosynthesis from *Agrobacterium tumefaciens* strain C58/ATCC33970 (GeneBank accession no. AE007871.2) (pNMD460).

TMV-based vectors with cell-to cell movement ability (Fig. 2) were created on the basis of vectors described in Marillonnet et al. (2006). pNMD035 construct was employed as a cloning vector for consequent insertion of coding sequences of genes of interest using Bsal cloning sites. Resulting constructs contained, in sequential order, a fragment from the *Arabidopsis* actin 2 (*ACT2*) promoter (GenBank accession no. AB026654); the 5' end of TVCV (GenBank accession no. BRU03387, base pairs 1-5455) and a fragment of cr-TMV [GenBank accession no. Z29370, base pairs 5457-5677, both together containing 16 intron insertions]; a gene of interest; cr-TMV 3' nontranslated region (3' NTR; GenBank accession no. Z29370), and the nopaline synthase (Nos) terminator. The entire fragment was cloned between the T-DNA left and right borders of binary vector. Genes of interest used in these constructs encoded GFP (pNMD 560), dsRED (pNMD580), human interferon alpha-a with rice amylase apoplast-targeting signal (pNMD38), klip27-mini-insulin with rice amylase apoplast-targeting signal (pNMD330), thaumatin 2 from Taumatococcus danielii (pNMD700), ß-glucosidase BGL4 from *Humicola grisea* (pNMD1200), exocellulase E3 from *Thermobifida fusca* (pNMD1160), exoglucanase 1 (CBH I) from *Trichoderma reesei* (pNMD1180), defensin Rs-AFP2 from *Rafanus sativus* (pNMD1061), defensin MsrA2 (a synthetic derivative of dermaseptin B1 from frog *Phyllomedusa bicolor)* (pNMD1071), defensin MB39 (modified cecropin from the Cecropia Moth *Hyalophora cecropia)* (pNMD1280), defensin plectasin from fungus *Pseudoplectania nigrella* (pNMDxxxx).

TMV-based vectors lacking cell-to cell movement ability were identical to corresponding TMV-based vectors capable of cell-to-cell movement with an exception of point mutation in MP-coding sequence leading to the open reading frame shift that distorted the MP translation (Fig. 3). Cloning of these constructs was performed using pNMD661 as a cloning vector.

For cloning of most of PVX-based vectors with cell- to-cell and systemic movement ability, pNMD670 cloning vector was used. Resulting constructs contained, in sequential order, 35S CaMV promoter, coding sequences of RNA-dependent RNA polymerase, coat protein, triple gene block modules comprising 25kDa, 12 kDa and 8 kDa proteins, gene of interest and 3'untranslated region. The entire fragment was cloned between the T-DNA left and right borders of binary vector (Fig. 4). Another group of PVX-based constructs had similar structure with difference in CP position, which was inserted between PVX polymerase and triple gene block (e. g., pNMD600).

PVX-based vectors with deletion of coat protein coding sequence were disabled for both systemic and cell-to cell movement. Cloning of these constructs was performed using pNMD694 as a cloning vector. This type of vectors contained, in sequential order, 35S CaMV promoter, coding sequences of RNA-dependent RNA polymerase, triple gene block module, gene of interest and 3'untranslated region inserted between the T-DNA left and right borders of binary vector (Fig.5).

### Example 2: Diluted agrobacteria can be delivered to Nicotiana benthamina using surfactant by spraying

We have shown that *Nicotiana benthamiana* plants can be transfected by spraying of plants with diluted agrobacterial cultures containing surfactant (Fig. 6). To evaluate the parameters influencing the transfection and optimize the transfection efficiency, we used dipping of *Nicotiana benthamiana* leaves in agrobacterial suspension. This approach allows exact measurements and easy testing of multiple experiment versions. Overnight agrobacterial cultures (OD600=1.5) were diluted 1:100 and 1:1000 (dilution factors 10⁻² and 10⁻², respectively) in 10 mM MES buffer (pH 5.5) containing 10 mM magnesium sulfate and supplemented with surfactant Silwet L-77. Three types of constructs providing GFP expression were tested: 1) transcriptional vectors, 2) TMV-based viral replicons and 3) PVX-based viral replicons (Fig. 6). Viral vectors used in these experiments were disabled for both systemic and cell-to-cell movement. They provided the expression of the reporter gene only in cells transfected with T-DNA. Percent of GFP-expressing cells was counted after the isolation of leaf protoplasts (Fig. 7). Depending of agrobacterial suspension concentration and regardless the type of vector, 2-8% of total leaf cells were transfected as a result of *Agrobacterium-*mediated T-DNA transfer when 0.1 % per volume Silwet-L77 and 1 min dipping time were used.

To find the optimal surfactant concentration, we tested 0.1 % and 0.05% Silwet L-77 in dipping experiments. For all three types of vectors, the transfection efficiency provided by using of 0.1 % Silwet was significantly higher if compared to 0.05 % concentration (Fig. 8-10).

10 sec dipping of *Nicotiana benthamiana* leaves in diluted agrobacterial suspension supplemented with 0.1 % Silwet L-77 provided transfection rates close to the efficiency of spraying with same suspension (Fig. 11). In both cases the transfection efficiency was higher for older developed leaves. The transfection rate varied from 1.4 to 3.7 % for dipping, and from 1.1 to 1.7 % for spraying at 1:100 dilution of agrobacterial culture. At 1:1000 dilution the variation was 0.2 -1.1 % for dipping and 0.1-0.6% for spraying.

The Silwet L-77 used in all examples herein was purchased from Kurt Obermeier GmbH & Co. KG (Bad Berleburg, Germany). The supplier is GE Silicones, Inc., USA. The Silwet L-77 used is an organosilicone product composed of 84.0 % of polyalkyleneoxide modified heptamethyltrisiloxane (CAS-No. 27306-78-1) and 16 % of allyloxypolyethylene-glycol methyl ether (CAS-No. 27252-80-8). All concentrations of Silwet L-77 content given in the examples or figures relate to this commercial product.

### Example 3: Diluted agrobacteria can be delivered to other species by spraying using surfactant and abrasive

We tested the number of plant species using agobacterial transfection with spraying and surfactant. First, we screened each species for optimal expression vector. For this purpose plant leaves were infiltrated using needleless syringe with 1:100 dilutions of OD=1.5 of five agrobacterial cultures harboring GFP expression transcriptional vectors: 1) transcriptional vector 35S-GFP + P19 (pNMD293), 2) TMV-based viral vector capable of cell-to-cell movement TMV(MP)-GFP (pNMD560), 3) TMV-based viral vector disabled for cell-to-cell movement TMV(fsMP)-GFP (pNMD570), 4) PXV-based viral vector capable of both systemic and cell-to-cell movement PVX(CP)-GFP (pNMD630) and 5) PVX-based viral vector disabled for both systemic and cell-to-cell movement PVX(ΔCP)-GFP (pNMD620). In some cases vacuum infiltration was performed.

We demonstrated the efficient Agrobacterium-mediated transfection for several Solanaceae species including Nicotiana benthamiana (all five vectors), tobacco *Nicotiana tabacum* (all five vectors), tomato *Lycopersicon esculentum* (PVX-based and transcriptional vectors), pepper *Capsicum annuum,* Inca berry *Physalis peruviana,* eggplant *Solanum melongena,* potato *Solanum tuberosum* (all with PVX-based vectors) (Fig. 13).

Agrobacterium-mediated transfection was denonstrated for the lettuce *Lactuca sativa* from Asteraceae family (transcriptional vector), beet *Beta vulgaris* from Chenopodiaceae family (all five vectors), zucchini *Cucurbita pepo* from Cucurbitaceae family (transcriptional vector), and cotton *Gossypium hirsutum* from Malvaceae family (all five vectors (Fig. 14).

Treatment of agrobacterial cells with acetosyringone (200 µM, 2 hours) significantly increased the transfection efficiency for several plant species including tomato, eggplant and zucchini (Fig. 15).

Based on infiltration data, spraying with diluted agrobacterial suspensions was tested for the number of plant species. The efficient delivery of diluted agrobacteria (10⁻³) by spraying with suspensions containing 0.1 % Silwet was demonstrated for several Nicotiana species *(Nicotiana benthamiana, Nicotiana debne, Nicotiana excelsior, Nicotiana exigua, Nicotiana maritima* and *Nicotiana simulans*) as it is shown using PVX with cell-to cell and systemic movement ability in Fig. 16.

Delivery of agrobacteria to other species including Solanaceae, Chenopodiaceae, Amarantaceae and Aizoaceae families was demonstrated by both dipping in agrobacterial suspension and spraying with and without abrasive using transcriptional vectors as well as TMV and PVX vectors with and without cell-to-cell movement ability (Fig. 17-21). The list of species successfully transfected includes spinach *Spinacea oleracea* from Amaranthaceae family (transcriptional and PVX-based vectors), beet *Beta vulgaris* varieties from Chenopodiaceae family (TMV-based and PVX-based viral vectors) (Fig. 17), tomato *Lycopersicon esculentum* (PVX-based vector) (Fig. 18) and Inca berry *Physalis peruviana* (PVX-based vector) (Fig. 19) from Solanaceae family, cotton *Gossypium hirsutum* from Malvaceae family (TMV-based vector) (Fig. 20). The expression of GFP in cotton tissue after agrobacterial transfection was confirmed using Western blot probed with GFP-specific antibodies (Fig. 21).

In all examples described herein, spraying was performed either with a pump spray flasks with nominal volume or 500 or 1000 ml (Carl Roth, # 0499.1 and # 0500.1) based on direct manual pumping or with a pressure sprayer with 1.25 L volume (Gardena, # 00864-20) exploiting the increased pressure for pumping. Plants were sprayed so as to wet completely leaves. Sprayers were shaken from time to time to ensure homogeneity of the suspensions to be sprayed, notably if the suspensions contained an abrasive.

### Example 4: Transfection of plants using Agrobacterum suspensions containing abrasive

The carborundum used in these experiments was a mixture of carborundum (silicon carbide) F800, F1000 and F1200 particles from Mineraliengrosshandel Hausen GmbH, Telfs, Austria. According to the provider, F800, F1000 and F1200 have surface median diameters of 6.5, 4.5 and 3 µm, respectively. 97 mass-% of the particles of F800, F1000 and F1200 have a surface diameter smaller than 14, 10 and 7 µm, respectively. 94 mass-% of the particles have a surface diameter larger than 2, 1, and 1 µm, respectively. F800, F1000 and F1200 were mixed in equal amounts by weight. 0.3% (w/v) of the mixed carborundum was added into the agrobacterial suspensions supplemented with 0.1% Silwet L-77 and used for the spraying of plants using the sprayers described in example 3.

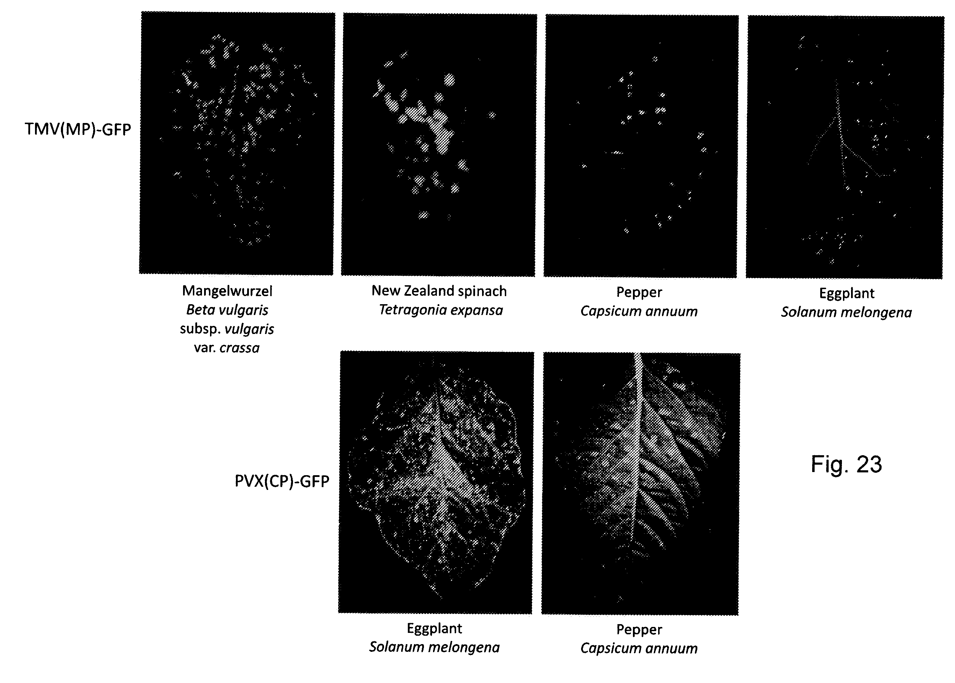

The results shown in Fig. 22 demonstrated that use of the abrasive significantly increases the transfection efficiency. Spraying of red beets with agrobacterial suspension containing 0.3 % of carborundum (silicon carbide SiC) provided a 15-fold increase of transfection efficiency (Fig. 22). List of species transfected using spraying with surfactant and abrasive includes also Mangelwurzel, another variety of *Beta vulgaris,* New Zealand spinach *Tetragonia expansa* from Aizoaceae family, pepper *Capsicum annuum* and eggplant *Solanum melongena* from Solanaceae (Fig. 23).

### Example 5: Treatment with agrobacteria can be repeated: multiple subsequent treatments

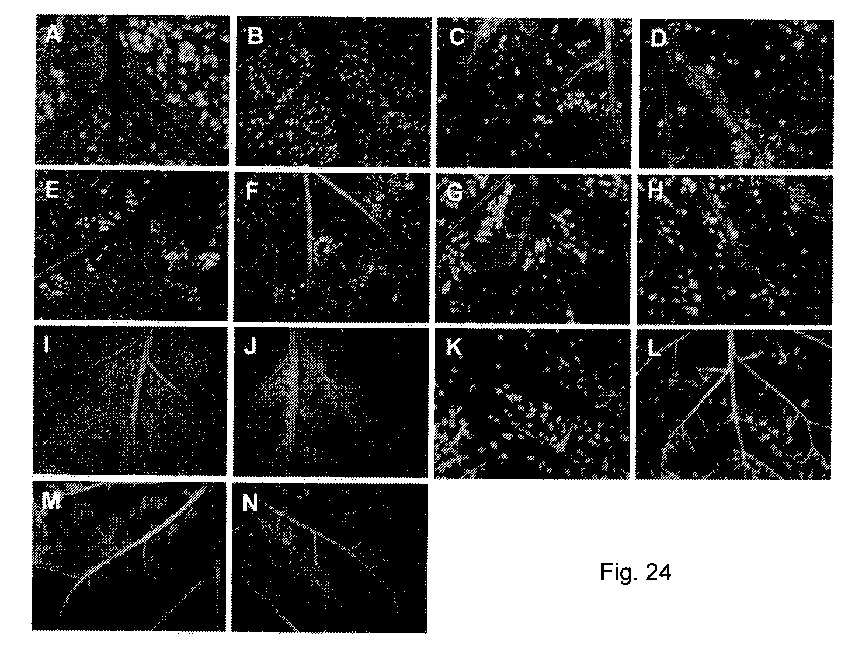

We performed multiple subsequent treatments of Nicotiana benthamiana plants with diluted agrobacteria. For this purpose leaves were dipped in diluted suspensions of Agrobacterium carrying next constructs: pNMD570 (TMV(fsMP)-GFP without cell-to-cell movement ability), pNMD560 (TMV(MP)-GFP with cell-to-cell movement ability), pNMD580 (TMV(MP)-DsRED with cell-to-cell movement ability), pNMD620 (PVX(ΔCP)-GFP without cell-to-cell movement ability, pNMD600 (PVX(CP)-GFP with cell-to-cell and systemic movement ability) and pNMD610 (PVX(CP)-dsRED with cell-to-cell and systemic movement ability). After the transfection, these vectors form fluorescing spots differing in colour and size (Fig. 24). We performed the dipping transfection of each tested leaf with 3 different cultures and with 7 days intervals between transfections. For each of eight tested vector combinations all transfections with agrobacterium were successful, no particular silencing effect was observed (Fig. 24).

### Example 6: Spraying with agrobacteria can deliver viral replicons capable of cell-to-cell movement

We demonstrated that spraying of *Nicotiana benthamiana* plants with 1:100 and 1:1000 dilutions of agrobacterial suspension provides efficient delivery of viral replicons capable of cell to cell-movement, which results in high expression of genes of interest, comparable with expression achieved using infiltration of agrobacteria. This was demonstrated for GFP (Fig. 25), human alpha-a interferon and klip27-mini-insulin (Fig. 26), and several cellulases including exocellulase E3 from *Thermobifida fusca,* exoglucanase 1 (CBH I) from *Trichoderma reesei,* ß-glucosidase BGL4 from *Humicola grisea* and exocellulase E3 from *Thermobifida fusca* (Fig. 27).

### Example 7: Agrobacteria can be used to deliver transcription factors as secondary messengers

We demonstrated the induction of anthocyanin biosynthesis in *Nicotiana tabacum* leaves infiltrated with agrobacterial suspension harbouring the PVX-based viral vector providing the expression of MYB transcription factor anthocyanin 1 (ANT1) from *Lycopersicon esculentum* (Fig. 28).

The attached sequence listing contains the following nucleotide sequences:
SEQ ID NO: 1: TNA region of T-DNA region of pNMD280.
SEQ ID NO: 2: TNA region of T-DNA region of pNMD033.
SEQ ID NO: 3: TNA region of T-DNA region of pNMD035.
SEQ ID NO: 4: TNA region of T-DNA region of pNMD661.
SEQ ID NO: 5: TNA region of T-DNA region of pNMD670.
SEQ ID NO: 6: TNA region of T-DNA region of pNMD694.

### References

1. Andrews, L. B. & Curtis, W. R. (2005). Comparison of transient protein expression in tobacco leaves and plant suspension culture. Biotechnol Prog 21, 946-52.
2. Arguelles-Arias, A., Ongena, M., Halimi, B., Lara, Y., Brans, A., Joris, B. & Fickers, P. (2009). Bacillus amyloliquefaciens GA1 as a source of potent antibiotics and other secondary metabolites for biocontrol of plant pathogens. Microb Cell Fact 8, 63.
3. Azhakanandam, K., Weissinger, S. M., Nicholson, J. S., Qu, R. & Weissinger, A. K. (2007). Amplicon-plus targeting technology (APTT) for rapid production of a highly unstable vaccine protein in tobacco plants. Plant Mol Biol 63, 393-404.
4. Barton, K. A., Binns, A. N., Matzke, A. J. & Chilton, M. D. (1983). Regeneration of intact tobacco plants containing full length copies of genetically engineered T-DNA, and transmission of T-DNA to R1 progeny. Cell 32, 1033-43.
5. Baum, J. A., Bogaert, T., Clinton, W., Heck, G. R., Feldmann, P., Ilagan, O., Johnson, S., Plaetinck, G., Munyikwa, T., Pleau, M., Vaughn, T. & Roberts, J. (2007). Control of coleopteran insect pests through RNA interference. Nat Biotechnol 25, 1322-6.6.
6. Bergey, D. R., Howe, G. A. & Ryan, C. A. (1996). Polypeptide signaling for plant defensive genes exhibits analogies to defense signaling in animals. Proc Natl Acad Sci U S A 93, 12053-8.
7. Broothaerts, W., Mitchell, H. J., Weir, B., Kaines, S., Smith, L. M., Yang, W., Mayer, J. E., Roa-Rodriguez, C. & Jefferson, R. A. (2005). Gene transfer to plants by diverse species of bacteria. Nature 433, 629-33.
8. Chuang, C. F. & Meyerowitz, E. M. (2000). Specific and heritable genetic interference by double-stranded RNA in Arabidopsis thaliana. Proc Natl Acad Sci U S A 97, 4985-90.
9. Chung, M. H., Chen, M. K. & Pan, S. M. (2000). Floral spray transformation can efficiently generate Arabidopsis transgenic plants. Transgenic Res 9, 471-6.
10. Clough, S. J. & Bent, A. F. (1998). Floral dip: a simplified method for Agrobacterium-mediated transformation of Arabidopsis thaliana. Plant J 16, 735-43.
11. Corbesier, L., Vincent, C., Jang, S., Fornara, F., Fan, Q., Searle, I., Giakountis, A., Farrona, S., Gissot, L., Turnbull, C. & Coupland, G. (2007). FT protein movement contributes to long-distance signaling in floral induction of Arabidopsis. Science 316, 1030-3.
12. D'Aoust, M. A., Lavoie, P. O., Belles-Isles, J., Bechtold, N., Martel, M. & Vezina, L. P. (2009). Transient expression of antibodies in plants using syringe agroinfiltration. Methods Mol Biol 483, 41-50.
13. D'Aoust, M. A., Lavoie, P. O., Couture, M. M., Trepanier, S., Guay, J. M., Dargis, M., Mongrand, S., Landry, N., Ward, B. J. & Vezina, L. P. (2008). Influenza virus-like particles produced by transient expression in Nicotiana benthamiana induce a protective immune response against a lethal viral challenge in mice. Plant Biotechnol J 6, 930-40.
14. De Buck, S., De Wilde, C., Van Montagu, M. & Depicker, A. (2000). Determination of the T-DNA transfer and the T-DNA integration frequencies upon cocultivation of Arabidopsis thaliana root explants. Mol Plant Microbe Interact 13, 658-65.
15. De Buck, S., Jacobs, A., Van Montagu, M. & Depicker, A. (1998). Agrobacterium tumefaciens transformation and cotransformation frequencies of Arabidopsis thaliana root explants and tobacco protoplasts. Mol Plant Microbe Interact 11, 449-57.
16. de Felippes, F. F. & Weigel, D. (2010). Transient assays for the analysis of miRNA processing and function. Methods Mol Biol 592, 255-64.
17. Deluc, L., Bogs, J., Walker, A. R., Ferrier, T., Decendit, A., Merillon, J. M., Robinson, S. P. & Barrieu, F. (2008). The transcription factor VvMYB5b contributes to the regulation of anthocyanin and proanthocyanidin biosynthesis in developing grape berries. Plant Physiol 147, 2041-53.
18. Escobar, M. A., Civerolo, E. L., Summerfelt, K. R. & Dandekar, A. M. (2001). RNAi-mediated oncogene silencing confers resistance to crown gall tumorigenesis. Proc Natl Acad Sci U S A 98, 13437-42.
19. Fraley, R. T., Rogers, S. G., Horsch, R. B., Sanders, P. R., Flick, J. S., Adams, S. P., Bittner, M. L., Brand, L. A., Fink, C. L., Fry, J. S., Galluppi, G. R., Goldberg, S. B., Hoffmann, N. L. & Woo, S. C. (1983). Expression of bacterial genes in plant cells. Proc Natl Acad Sci U S A 80, 4803-7.
20. Giritch, A., Marillonnet, S., Engler, C., van Eldik, G., Botterman, J., Klimyuk, V. & Gleba, Y. (2006). Rapid high-yield expression of full-size IgG antibodies in plants coinfected with noncompeting viral vectors. Proc Natl Acad Sci U S A 103, 14701-6.
21. Gleba, Y., Klimyuk, V. & Marillonnet, S. (2007). Viral vectors for the expression of proteins in plants. Curr Opin Biotechnol 18, 134-41.
22. Gleba, Y., Marillonnet, S. & Klimyuk, V. (2004). Engineering viral expression vectors for plants: the 'full virus' and the 'deconstructed virus' strategies. Curr Opin Plant Biol 7, 182-8.
23. Gleba, Y., Marillonnet, S. & Klimyuk, V. (2008). Plant virus vectors (gene expression systems). In Encyclopedia of Virology, Third Edition. M.H.V. van Regenmortel, Mahy, B.W..J., eds. (San Diego, CA: Elsevier Academic Press), vol. 4, pp. 229-237.
24. Green, B. J., Fujiki, M., Mett, V., Kaczmarczyk, J., Shamloul, M., Musiychuk, K., Underkoffler, S., Yusibov, V. & Mett, V. (2009). Transient protein expression in three Pisum sativum (green pea) varieties. Biotechnol J 4, 230-7.
25. Grimsley, N., Hohn, B., Hohn, T. & Walden, R. (1986). "Agroinfection," an alternative route for viral infection of plants by using the Ti plasmid. Proc Natl Acad Sci U S A 83, 3282-3286.
26. Huang, G., Allen, R., Davis, E. L., Baum, T. J. & Hussey, R. S. (2006). Engineering broad root-knot resistance in transgenic plants by RNAi silencing of a conserved and essential root-knot nematode parasitism gene. Proc Natl Acad Sci U S A 103, 14302-6.
27. Huang, Z., Santi, L., LePore, K., Kilbourne, J., Arntzen, C. J. & Mason, H. S. (2006). Rapid, high-level production of hepatitis B core antigen in plant leaf and its immunogenicity in mice. Vaccine 24, 2506-13.
28. Joh, L. D., McDonald, K. A. & VanderGheynst, J. S. (2006). Evaluating extraction and storage of a recombinant protein produced in agroinfiltrated lettuce. Biotechnol Prog 22, 723-30.
29. Joh, L. D., Wroblewski, T., Ewing, N. N. & VanderGheynst, J. S. (2005). High-level transient expression of recombinant protein in lettuce. Biotechnol Bioeng 91, 861-71.
30. Johnson, K. B. (2009). Optimization of Biocontrol of Plant Diseases. Annu Rev Phytopathol.
31. Jones, H. D., Doherty, A. & Sparks, C. A. (2009). Transient transformation of plants. Methods Mol Biol 513, 131-52.
32. Kerr, A. & Tate, M. E. (1984). Agrocins and the biological control of crown gall. Microbiol Sci 1, 1-4.
33. Kim, J. G., Park, B. K., Kim, S. U., Choi, D., Nahm, B. H., Moon, J. S., Reader, J. S., Farrand, S. K. & Hwang, 1. (2006). Bases of biocontrol: sequence predicts synthesis and mode of action of agrocin 84, the Trojan horse antibiotic that controls crown gall. Proc Natl Acad Sci U S A 103, 8846-51.
34. Lee, J. J., Woodward, A. W. & Chen, Z. J. (2007). Gene expression changes and early events in cotton fibre development. Ann Bot 100, 1391-401.
35. Lee, M. W. & Yang, Y. (2006). Transient expression assay by agroinfiltration of leaves. Methods Mol Biol 323, 225-9.
36. Li, J. F., Park, E., von Arnim, A. G. & Nebenfuhr, A. (2009). The FAST technique: a simplified Agrobacterium-based transformation method for transient gene expression analysis in seedlings of Arabidopsis and other plant species. Plant Methods 5, 6.
37. Li, X. Q., Liu, C. N., Ritchie, S. W., Peng, J. Y., Gelvin, S. B. & Hodges, T. K. (1992). Factors influencing Agrobacterium-mediated transient expression of gusA in rice. Plant Mol Biol 20, 1037-48.
38. Lico, C., Chen, Q. & Santi, L. (2008). Viral vectors for production of recombinant proteins in plants. J Cell Physiol 216, 366-77.
39. Lindbo, J. A. (2007). TRBO: a high-efficiency tobacco mosaic virus RNA-based overexpression vector. Plant Physiol 145, 1232-40.
40. Lindbo, J. A. (2007). High-efficiency protein expression in plants from agroinfection-compatible Tobacco mosaic virus expression vectors. BMC Biotechnol 7, 52.
41. Liu, C. N., Li, X. Q. & Gelvin, S. B. (1992). Multiple copies of virG enhance the transient transformation of celery, carrot and rice tissues by Agrobacterium tumefaciens. Plant Mol Biol 20, 1071-87.
42. Marillonnet, S., Giritch, A., Gils, M., Kandzia, R., Klimyuk, V. & Gleba, Y. (2004). In planta engineering of viral RNA replicons: efficient assembly by recombination of DNA modules delivered by Agrobacterium. Proc Natl Acad Sci U S A 101, 6852-7.
43. Marillonnet, S., Thoeringer, C., Kandzia, R., Klimyuk, V. & Gleba, Y. (2005). Systemic Agrobacterium tumefaciens-mediated transfection of viral replicons for efficient transient expression in plants. Nat Biotechnol 23, 718-23.
44. Martinez-Garcia, J. F., Virgos-Soler, A. & Prat, S. (2002). Control of photoperiod-regulated tuberization in potato by the Arabidopsis flowering-time gene CONSTANS. Proc Natl Acad Sci U S A 99, 15211-6.
45. Mett, V., Lyons, J., Musiychuk, K., Chichester, J. A., Brasil, T., Couch, R., Sherwood, R., Palmer, G. A., Streatfield, S. J. & Yusibov, V. (2007). A plant-produced plague vaccine candidate confers protection to monkeys. Vaccine 25, 3014-7.
46. Moore, L. W. (1988). Use of Agrobacterium radiobacter in agricultural ecosystems. Microbiol Sci 5, 92-5.
47. Nam, M. H., Park, M. S., Kim, H. G. & Yoo, S. J. (2009). Biological control of strawberry Fusarium wilt caused by Fusarium oxysporum f. sp. fragariae using Bacillus velezensis BS87 and RK1 formulation. J Microbiol Biotechnol 19, 520-4.
48. Pandolfini, T., Rotino, G. L., Camerini, S., Defez, R. & Spena, A. (2002). Optimisation of transgene action at the post-transcriptional level: high quality parthenocarpic fruits in industrial tomatoes. BMC Biotechnol 2, 1.
49. Plesha, M. A., Huang, T. K., Dandekar, A. M., Falk, B. W. & McDonald, K. A. (2007). High-level transient production of a heterologous protein in plants by optimizing induction of a chemically inducible viral amplicon expression system. Biotechnol Prog 23, 1277-85.
50. Plesha, M. A., Huang, T. K., Dandekar, A. M., Falk, B. W. & McDonald, K. A. (2009). Optimization of the bioprocessing conditions for scale-up of transient production of a heterologous protein in plants using a chemically inducible viral amplicon expression system. Biotechnol Prog 25, 722-34.
51. Pooggin, M., Shivaprasad, P. V., Veluthambi, K. & Hohn, T. (2003). RNAi targeting of DNA virus in plants. Nat Biotechnol 21, 131-2.
52. Reader, J. S., Ordoukhanian, P. T., Kim, J. G., de Crecy-Lagard, V., Hwang, I., Farrand, S. & Schimmel, P. (2005). Major biocontrol of plant tumors targets tRNA synthetase. Science 309, 1533.
53. Regnard, G. L., Halley-Stott, R. P., Tanzer, F. L., Hitzeroth, II & Rybicki, E. P. (2010). High level protein expression in plants through the use of a novel autonomously replicating geminivirus shuttle vector. Plant Biotechnol J 8, 38-46.
54. Ryu, C. M., Anand, A., Kang, L. & Mysore, K. S. (2004). Agrodrench: a novel and effective agroinoculation method for virus-induced gene silencing in roots and diverse Solanaceous species. Plant J 40, 322-31.
55. Santi, L., Giritch, A., Roy, C. J., Marillonnet, S., Klimyuk, V., Gleba, Y., Webb, R., Arntzen, C. J. & Mason, H. S. (2006). Protection conferred by recombinant Yersinia pestis antigens produced by a rapid and highly scalable plant expression system. Proc Natl Acad Sci U S A 103, 861-6.
56. Shang, Y., Schwinn, K. E., Bennett, M. J., Hunter, D. A., Waugh, T. L., Pathirana, N. N., Brummell, D. A., Jameson, P. E. & Davies, K. M. (2007). Methods for transient assay of gene function in floral tissues. Plant Methods 3, 1.
57. Shiboleth, Y. M., Arazi, T., Wang, Y. & Gal-On, A. (2001). A new approach for weed control in a cucurbit field employing an attenuated potyvirus-vector for herbicide resistance. J Biotechnol 92, 37-46.
58. Shoji, Y., Farrance, C. E., Bi, H., Shamloul, M., Green, B., Manceva, S., Rhee, A., Ugulava, N., Roy, G., Musiychuk, K., Chichester, J. A., Mett, V. & Yusibov, V. (2009). Immunogenicity of hemagglutinin from A/Bar-headed Goose/Qinghai/1A/05 and A/Anhui/1/05 strains of H5N1 influenza viruses produced in Nicotiana benthamiana plants. Vaccine 27, 3467-70.
59. Simmons, C. W., VanderGheynst, J. S. & Upadhyaya, S. K. (2009). A model of Agrobacterium tumefaciens vacuum infiltration into harvested leaf tissue and subsequent in planta transgene transient expression. Biotechnol Bioeng 102, 965-70.
60. Sudarshana, M. R., Plesha, M. A., Uratsu, S. L., Falk, B. W., Dandekar, A. M., Huang, T. K. & McDonald, K. A. (2006). A chemically inducible cucumber mosaic virus amplicon system for expression of heterologous proteins in plant tissues. Plant Biotechnol J 4, 551-9.
61. Turpen, T. H., Turpen, A. M., Weinzettl, N., Kumagai, M. H. & Dawson, W. O. (1993). Transfection of whole plants from wounds inoculated with Agrobacterium tumefaciens containing cDNA of tobacco mosaic virus. J Virol Methods 42, 227-39.
62. Vaquero, C., Sack, M., Chandler, J., Drossard, J., Schuster, F., Monecke, M., Schillberg, S. & Fischer, R. (1999). Transient expression of a tumor-specific single-chain fragment and a chimeric antibody in tobacco leaves. Proc Natl Acad Sci U S A 96, 11128-33.
63. Vezina, L. P., Faye, L., Lerouge, P., D'Aoust, M. A., Marquet-Blouin, E., Burel, C., Lavoie, P. O., Bardor, M. & Gomord, V. (2009). Transient co-expression for fast and high-yield production of antibodies with human-like N-glycans in plants. Plant Biotechnol J 7, 442-55.
64. Vicedo, B., Penalver, R., Asins, M. J. & Lopez, M. M. (1993). Biological Control of Agrobacterium tumefaciens, Colonization, and pAgK84 Transfer with Agrobacterium radiobacter K84 and the Tra Mutant Strain K1026. Appl Environ Microbiol 59, 309-315.
65. Voinnet, O., Rivas, S., Mestre, P. & Baulcombe, D. (2003). An enhanced transient expression system in plants based on suppression of gene silencing by the p19 protein of tomato bushy stunt virus. Plant J 33, 949-56.
66. Wroblewski, T., Tomczak, A. & Michelmore, R. (2005). Optimization of Agrobacterium-mediated transient assays of gene expression in lettuce, tomato and Arabidopsis. Plant Biotechnol J 3, 259-73.
67. Yang, L., Wang, H., Liu, J., Li, L., Fan, Y., Wang, X., Song, Y., Sun, S., Wang, L., Zhu, X. & Wang, X. (2008). A simple and effective system for foreign gene expression in plants via root absorption of agrobacterial suspension. J Biotechnol 134, 320-4.
68. Yang, Y., Li, R. & Qi, M. (2000). In vivo analysis of plant promoters and transcription factors by agroinfiltration of tobacco leaves. Plant J 22, 543-51.
69. Zambre, M., Terryn, N., De Clercq, J., De Buck, S., Dillen, W., Van Montagu, M., Van Der Straeten, D. & Angenon, G. (2003). Light strongly promotes gene transfer from Agrobacterium tumefaciens to plant cells. Planta 216, 580-6.
70. Zeevaart, J. A. (2008). Leaf-produced floral signals. Curr Opin Plant Biol 11, 541-7.
71. Zhang, C. & Ghabrial, S. A. (2006). Development of Bean pod mottle virus-based vectors for stable protein expression and sequence-specific virus-induced gene silencing in soybean. Virology 344, 401-11.
72. Zhao, M. M., An, D. R., Zhao, J., Huang, G. H., He, Z. H. & Chen, J. Y. (2006). Transiently expressed short hairpin RNA targeting 126 kDa protein of tobacco mosaic virus interferes with virus infection. Acta Biochim Biophys Sin (Shanghai) 38, 22-8.

## Claims

1. A process of transfecting a plant, comprising spraying aerial parts of said plant with an aqueous suspension containing cells of an *Agrobacterium* strain and at least one abrasive suspended in said suspension, said *Agrobacterium* strain comprising a DNA molecule comprising a nucleic acid construct containing a DNA sequence of interest to be transfected into cells of the plant.

2. A process of generating or altering a trait in a plant, comprising
(i) growing said plant up to a desired growth state;
(ii) expressing, in said plant, a protein or an RNA capable of generating or altering said trait, comprising spraying aerial parts of said plant with an aqueous suspension containing cells of an *Agrobacterium* strain and at least one abrasive suspended in said suspension,
said *Agrobacterium* strain comprising a DNA molecule comprising a nucleic acid construct containing a DNA sequence of interest, said DNA sequence of interest encoding said protein or said RNA.

3. A process of producing a protein of interest in a plant, comprising
(i) growing said plant up to a desired growth state;
(ii) expressing, in said plant, said protein of interest, comprising spraying aerial parts of said plant with an aqueous suspension containing cells of an *Agrobacterium* strain and at least one abrasive suspended in said suspension,
said *Agrobacterium* strain comprising a DNA molecule comprising a nucleic acid construct containing a DNA sequence of interest, said DNA sequence of interest encoding said protein of interest.

4. A process of protecting crop plants on a field from a pest, comprising
(i) growing said plants in soil of said field;
(ii) determining, in a desired growth state of said plants, infestation of at least one of said plants by a pest;
(iii) expressing, in said plants, a protein or an RNA that is detrimental to the pest determined in the previous step, comprising spraying aerial parts of said plants with an aqueous suspension containing cells of an *Agrobacterium* strain and at least one abrasive suspended in said suspension,
said *Agrobacterium* strain comprising a DNA molecule comprising a nucleic acid construct containing a DNA sequence of interest operably linked to a promoter, said DNA sequence of interest encoding said protein or said RNA.

5. The process according to any one of claims 1 to 4, wherein said aqueous suspension contains said cells of said *Agrobacterium* strain in a concentration of at most 2.2·10⁷, preferably of at most 1.1·10⁷, more preferably of at most 4.4·10⁶, more preferably of at most 1.1·10⁶ cfu/ml of said suspension.

6. The process according to any one of claims 1 to 5, wherein said abrasive is a particulate inorganic carrier for wettable powders, such as silica or carborundum.

7. The process according to claim 6, wherein said aqueous suspension contains said abrasive in an amount of between 0.02 and 2, preferably between 0.05 and 1 and more preferably between 0.1 and 0.5 % by weight of said suspension.

8. The process according to claim 6 or 7, wherein the median particle size of the abrasive added to the suspension is between 0.1 and 30, preferably between 0.1 and 10, more preferably between 0.5 and 10, and most preferably between 0.5 and 5 µm.

9. The process according to any one of claims 6 to 8, wherein the abrasive has a D90 value of at most 40 µm, preferably of at most 30 µm; and wherein the abrasive does not contain particles having a size above 45 µm, preferably not above 40 µm.

10. The process according to any one of claims 1 to 9, wherein said suspension further comprises an agricultural spray adjuvant, preferably a non-ionic surfactant or wetting agent.

11. The process according to claim 10, wherein the spray adjuvant is an organo-silicone wetting agent, such as Silwet L-77.

12. The process according to any one of claims 1 to 11, wherein said nucleic acid construct is flanked by a T-DNA border sequence on at least one side, which permits the transfer of said nucleic acid construct into cells of said plant.

13. The process according to any one of claims 1 to 12, wherein said nucleic acid construct encodes a replicating viral vector encoding said protein of interest, said replicating viral vector being incapable of system movement in said plant.

14. The process according to any one of claims 1 to 12, wherein said DNA sequence of interest is operably linked to a promoter active in plant cells.

15. Aqueous suspension containing cells of an *Agrobacterium* strain and at least one abrasive suspended in said suspension, wherein said aqueous suspension contains said cells of said *Agrobacterium* strain in a concentration of at most 4.4·10⁷, preferably of at most 1.1·10⁷, preferably of at most 4.4·10⁶, more preferably of at most 1.1·10⁶ cfu/ml of said suspension; said *Agrobacterium* strain comprising a heterologous DNA molecule comprising a nucleic acid construct containing a heterologous DNA sequence of interest that may be operably linked to a promoter; said suspension optionally further comprising a preferably non-ionic wetting agent such as an organosilicone surfactant.
